# EUROPEAN PATENT APPLICATION

(11) **EP 4 834 937 A1**
(43) Date of publication of application: **04.11.2026**
(21) Application number: 24911455.4
(22) Date of filing: 27.12.2024
(51) Int. Cl.: C07K 16/28, C07K 14/55, A61P 35/00, A61K 38/20, A61K 39/00

(54) **PD-1/IL-2R BISPECIFIC ANTIBODY FUSION PROTEIN, AND USE AND USAGE METHOD THEREOF**

(30) Priority: 28.12.2023 CN 202311840553; 24.05.2024 CN 202410652504
(71) Applicant: Fortvita Biologics (Singapore) Pte. Ltd., Singapore 189767 (SG)
(72) Inventor: ZHOU, Hui, Suzhou, Jiangsu 215123 (CN); DING, Luyin, Suzhou, Jiangsu 215123 (CN); CHEN, Yuling, Suzhou, Jiangsu 215123 (CN); HE, Kaijie, Suzhou, Jiangsu 215123 (CN); SUN, Xing, Suzhou, Jiangsu 215123 (CN); ZHENG, Shirui, Suzhou, Jiangsu 215123 (CN)
(74) Representative: Heath, Abigail
(86) International application number: PCT/CN2024/143132
(87) International publication number: WO 2025/140535

(57) **Abstract**

The present invention relates to a PD-1/IL-2R bispecific antibody fusion protein for treating tumors (such as solid tumors or hematologic tumors), or a pharmaceutical composition or pharmaceutical combination containing same, and a use and usage method thereof.

## Description

### TECHNICAL FIELD

The present invention relates to the field of medicine. Specifically, the present invention relates to a PD-1/IL-2R bispecific antibody fusion protein or a pharmaceutical composition or pharmaceutical combination comprising the same for treating tumors (for example, solid tumors or hematologic tumors) and their uses and methods of use.

### BACKGROUND

Malignant tumor is one of the major threats to human life and health. It is estimated that nearly 10 million people died of cancer worldwide in 2020. Effective drug therapy can help patients obtain longer survival time and improved quality of life. Currently available drug therapies include chemotherapeutic drugs, targeted drugs, immunotherapeutic drugs, and the like. In addition, genetic recombination technology has also caused vigorous development of genetically engineered protein drugs, among which drugs successfully developed and marketed include cytokines, therapeutic humanized monoclonal antibodies, and the like.

However, due to the lack of fundamental metabolic differences between tumor cells and normal cells, antitumor drugs cannot completely avoid damage to normal tissues. Moreover, although immunotherapy (IO) has obtained some benefits in some tumor treatments, clinical needs for tumor resistant to immunotherapy and cold tumor still remain to be urgently addressed. For a specific antitumor drug, it is necessary to carefully design and determine a dosage regimen, so as to exert the expected efficacy and have acceptable side effects. An inappropriate dosage regimen (including administration route, dose level, dosing frequency, and the like) will affect clinical efficacy; for example, an excessively high dose may even lead to unacceptable side effects, reducing patient compliance and decreasing quality of life. On the other hand, toxic side effects also limit the application of later-line therapy for tumors. For patients who have experienced side effects of antitumor drugs, it is generally more difficult to tolerate later-line therapy for tumors, especially in the presence of superimposed toxicity.

Therefore, optimization of a dosage regimen is particularly critical for antitumor drugs. On the one hand, antitumor drugs should be considered based on an upper safety limit; on the other hand, killing of tumor cells should be achieved as much as possible under conditions tolerable by normal cells, good clinical benefit should be obtained and longer survival should be provided, while having improved quality of life and improving patient compliance. In particular, for a specific antitumor drug, various influencing factors need to be comprehensively considered, and a dosage regimen needs to be carefully designed and determined. There remains an unmet need in the art for providing safe and effective antitumor drug therapy.

In addition, the biological behavior of most tumors is not dominated by a single signaling pathway, but multiple signaling pathways act together, which leads to limited therapeutic effects of most anticancer drugs. Furthermore, anticancer drugs are often accompanied by significant adverse reactions. Therefore, combined use of drugs having different mechanisms of action to enhance anticancer effects and/or reduce side effects is one of the hot spots in anticancer drug research and development. For example, pembrolizumab has been approved by the U.S. Food and Drug Administration (FDA) for use in combination with chemotherapeutic agents, for example, in combination with pemetrexed and a platinum-based chemotherapeutic agent or in combination with carboplatin and paclitaxel as first-line therapy of metastatic non-squamous non-small cell lung cancer, in combination with a platinum-based chemotherapeutic agent and fluorouracil as first-line therapy of metastatic head and neck squamous epithelial cell carcinoma or unresectable recurrent head and neck squamous epithelial cell carcinoma, and in combination with lenvatinib for treating advanced endometrial cancer. palbociclib has been approved by the FDA for use in combination with an aromatase inhibitor or in combination with fulvestrant for treating hormone receptor (HR)-positive, human epidermal growth factor receptor postmenopausal women estrogen receptor (ER)-positive, human epidermal growth factor receptor 2 (HER2)-negative advanced breast (HER)-negative advanced or metastatic breast cancer. bevacizumab has been approved by the National Medical Products Administration (NMPA) for use in combination with fluorouracil for treating metastatic colorectal cancer, and in combination with a platinum-based chemotherapeutic agent for treating unresectable advanced, metastatic, or recurrent non-squamous non-small cell lung cancer.

Although there are needs in the prior art for combination medication regimens and products targeting different signaling pathways, in view of factors such as the complexity of mechanisms of tumorigenesis and the unpredictability of interactions between different drugs, combined use of anticancer drugs having different mechanisms of action does not necessarily produce beneficial effects. Therefore, discovering combination medication regimens and products having effective anticancer effects is also a major challenge in the field of medicine.

Therefore, the present invention aims to provide a PD-1/IL-2R bispecific antibody fusion protein capable of effectively treating a tumor such as a solid tumor or a hematologic tumor, or a pharmaceutical composition or pharmaceutical combination comprising the same, and use or method thereof, such that more therapeutic means are provided for patients and clinical needs are met.

### SUMMARY

### BRIEF DESCRIPTION OF THE INVENTION

The present inventors have unexpectedly found that, by administering the PD-1/IL-2R bispecific antibody fusion protein of the present invention, particularly administering according to the dosage regimen of the present invention, encouraging therapeutic effects are obtained in patients with tumors (including solid tumors and hematologic tumors), and the administration can be well tolerated, provides longer survival and/or improved quality of life, and has acceptable and controllable safety.

The present inventors have also unexpectedly found that administering the PD-1/IL-2R bispecific antibody fusion protein according to the dosage regimen of the present invention can produce favorable efficacy in treating tumors resistant to immunotherapy (IO) and cold tumors, and has acceptable and controllable safety.

The present inventors have also unexpectedly found that a pharmaceutical combination comprising the PD-1/IL-2R bispecific antibody fusion protein of the present invention can effectively treat tumors, for example cancers, particularly advanced cancers. Cancers that can be treated with the pharmaceutical combination of the present invention include, for example, intestinal cancer (including rectal cancer, colon cancer, and colorectal cancer), melanoma, non-small cell lung cancer (including squamous and non-squamous non-small cell lung cancer), hepatocellular carcinoma, lymphoma, renal cell carcinoma, esophageal squamous cell carcinoma, gastric and gastroesophageal junction adenocarcinoma, pancreatic cancer, breast cancer, ovarian cancer, and the like.

Therefore, in a first aspect, provided is use of the PD-1/IL-2R bispecific antibody fusion protein of the present invention for treating a tumor such as a solid tumor or a hematologic tumor.

In a second aspect, provided is a method for treating a tumor such as a solid tumor or a hematologic tumor in an individual, the method comprising administering the PD-1/IL-2R bispecific antibody fusion protein of the present invention to an individual in need thereof.

In a third aspect, provided is the PD-1/IL-2R bispecific antibody fusion protein of the present invention for treating a tumor such as a solid tumor or a hematologic tumor.

In a fourth aspect, provided is use of the PD-1/IL-2R bispecific antibody fusion protein of the present invention in preparing a medicament for treating a tumor such as a solid tumor or a hematologic tumor.

In a fifth aspect, provided is a pharmaceutical composition, which comprises the PD-1/IL-2R bispecific antibody fusion protein of the present invention and one or more pharmaceutically acceptable excipients.

In a sixth aspect, provided is a kit, which comprises the PD-1/IL-2R bispecific antibody fusion protein of the present invention and instructions indicating that the fusion protein is used for treating a tumor such as a solid tumor or a hematologic tumor.

In another aspect, provided is a pharmaceutical combination for treating a tumor such as a solid tumor or a hematologic tumor, wherein the pharmaceutical combination comprises the PD-1/IL-2R bispecific antibody fusion protein of the present invention as a first active ingredient, and comprises a second active ingredient.

In another aspect, provided is use of the pharmaceutical combination of the present invention in preparing a medicament.

In another aspect, provided is a method for treating a tumor (including a solid tumor or a hematologic tumor) for example cancer by administering the pharmaceutical combination of the present invention.

In another aspect, provided is a single-dose administration unit, which comprises a single therapeutically effective amount of the PD-1/IL-2R bispecific antibody fusion protein of the present invention.

In other aspects, provided is also use of the pharmaceutical combination of the present invention for treating a tumor (including a solid tumor or a hematologic tumor) for example cancer. In another aspect, provided is also a kit comprising the pharmaceutical combination of the present invention and instructions for using the pharmaceutical combination for treating a tumor such as a solid tumor or a hematologic tumor. Various modifications of the aspects described in the present invention are also included in the present invention, provided that they do not depart from the spirit of the present invention.

### DETAILED DESCRIPTION OF THE INVENTION

Interleukin-2 (IL-2) is one of the earliest therapies approved for antitumor treatment; however, its safety is poor, and it may lead to various serious toxic reactions including vascular leak syndrome (VLS), pulmonary edema, hypotension, and cardiotoxicity. In addition, because IL-2 acts on both T_{regs} and effector T cells, how to guide it to exert the desired immunomodulatory effect also brings a great challenge to the antitumor application of IL-2 therapy. In recent years, breakthrough progress in immune checkpoint inhibitor (ICI) therapy represented by CTLA-4 and PD-1 has brought new development ideas for IL-2. Compared with conventional chemotherapy and radiotherapy, ICI therapy can provide patients with more durable clinical benefit; however, most patients exhibit primary or secondary resistance to ICI after treatment, which is speculated to be due, to a certain extent, to tumor antigen-specific T cell exhaustion, characterized by multi-checkpoint expression and impaired cytokine secretion and activity. As an important cytokine for activating tumor-specific CD8⁺ T cells, IL-2 is mechanistically complementary to ICI, and multiple study results have confirmed that IL-2 can reverse T cell exhaustion in preclinical models.

The PD-1/IL-2R bispecific antibody fusion protein of the present invention can simultaneously block the PD-1/PD-L1 pathway and activate the IL-2 pathway, and therefore can simultaneously achieve PD-1 blockade and IL-2 delivery, and has great potential for addressing the clinical needs of immunotherapy (IO) resistance and cold tumors. The fusion protein of the present invention exhibited good antitumor activity in multiple tumor-bearing pharmacological models, including a PD-1-resistant and metastatic model (B16F10).

Through carefully designed phase Ia, phase Ib, and phase II clinical trials, and comprehensively considering preclinical pharmacodynamic results as well as pharmacokinetics, pharmacodynamics, immunogenicity, toxicology, and the like in human subjects, the present inventors determined the use and method of the PD-1/IL-2R bispecific antibody fusion protein of the present invention for treating tumors for example solid tumors or hematologic tumors, and suitable optimized dosage regimens (including combination administration).

Specifically, the present invention provides the following embodiments:
**Embodiments1.** Use of a PD-1/IL-2R bispecific antibody fusion protein for treating a solid tumor or a hematologic tumor, wherein the PD-1/IL-2R bispecific antibody fusion protein comprises: (a) a first monomer comprising an IL-2 mutein fused with an Fc fragment, optionally via or not via a linker, wherein the IL-2 mutein has T3A+N88R+S130R mutations and IL15 B'C' loop region AGDASIH relative to wild-type IL-2 (for example an amino acid sequence as set forth in SEQ ID NO: 3); and (b) a second monomer comprising a heavy chain and a light chain of an antibody that specifically binds PD-1.
**Embodiments2.** The use according to embodiment 1, wherein the IL-2 mutein comprises or consists of the following amino acid sequence:
   an amino acid sequence as set forth in SEQ ID NO: 4; or
   an amino acid sequence having at least 90%, 91%, 92%, 93%, 94%, 95%, 96%, 97%, 98%, or 99% identity to the amino acid sequence as set forth in SEQ ID NO: 4 and having T3A+N88R+S130R mutations and IL15B'C' loop region AGDASIH relative to wild-type IL-2 (for example, an amino acid sequence as set forth in SEQ ID NO: 3).
**Embodiments3.** The use according to embodiment 1, wherein the linker comprises (GGGGS)n, wherein n is an integer of at least 1, for example 1, 2, 3, or 4, for example SEQ ID NO: 5.
**Embodiments4.** The use according to any one of embodiments 1-3, wherein the heavy chain of the antibody that specifically binds PD-1 comprises an Fc fragment, and the Fc fragment as well as the Fc fragment fused to the IL-2 mutein is an Fc fragment of IgG1, IgG2, IgG3, or IgG4, for example human IgG1 Fc.
**Embodiments5.** The use according to embodiment 4, wherein the Fc fragment has mutations that bind to an Fcγ receptor, for example L234A/L235A mutations or L234A/L235E/G237A.
**Embodiments6.** The use according to embodiment 4 or 5, wherein the Fc fragment of the first monomer comprises a Knob mutation, and the antibody heavy-chain Fc fragment of the second monomer contains a Hole mutation, or the Fc fragment of the first monomer comprises a Hole mutation, and the antibody heavy-chain Fc fragment of the second monomer comprises a Knob mutation; for example, the hole mutations are Y349C, T366S, L368A, and Y407V mutations, and/or the Knob mutations are T366W and S354C mutations.
**Embodiments7.** The use according to embodiment 6, wherein
   the Fc fragment of the first monomer comprises or consists of the following amino acid sequence: an amino acid sequence as set forth in SEQ ID NO: 6, or an amino acid sequence having at least 90%, 91%, 92%, 93%, 94%, 95%, 96%, 97%, 98%, or 99% identity to the amino acid sequence as set forth in SEQ ID NO: 6;
      or
   the antibody heavy-chain Fc fragment of the second monomer comprises or consists of the following amino acid sequence: an amino acid sequence as set forth in SEQ ID NO: 12, or an amino acid sequence having at least 90%, 91%, 92%, 93%, 94%, 95%, 96%, 97%, 98%, or 99% identity to the amino acid sequence as set forth in SEQ ID NO: 12.
**Embodiments8.** The use according to any one of embodiments 1-6, wherein the first monomer comprises:
   an amino acid sequence as set forth in SEQ ID NO: 7; or
   an amino acid sequence having at least 90%, 91%, 92%, 93%, 94%, 95%, 96%, 97%, 98% or 99% identity to the amino acid sequence as set forth in SEQ ID NO: 7 and T3A+N88R+S130R mutations and IL15B'C' loop region AGDASIH relative to wild-type IL-2 (for example, an amino acid sequence as set forth in SEQ ID NO: 3) in the IL-2 mutein, and/or S354C/T366W and optionally L234A/L235A mutations in the Fc fragment.
**Embodiments9.** The use according to any one of embodiments 1-7, wherein the heavy chain of the antibody that specifically binds PD-1 comprises HCDR1, HCDR2, and HCDR3, and the light chain of the antibody that specifically binds PD-1 comprises LCDR1, LCDR2, and LCDR3, wherein the amino acid sequences of HCDR1, HCDR2, and HCDR3 are respectively as follows: SEQ ID NOs: 9, 10, and 11, and the amino acid sequences of LCDR1, LCDR2, and LCDR3 are respectively as follows: SEQ ID NOs: 16, 17, and 18.
**Embodiments10.** The use according to embodiment 9, wherein the heavy chain of the antibody that specifically binds PD-1 comprises a heavy-chain variable region VH, and the light chain of the antibody that specifically binds PD-1 comprises a light-chain variable region VL, wherein the VH comprises or consists of an amino acid sequence as set forth in SEQ ID NO: 8 or an amino acid sequence having at least 90%, 91%, 92%, 93%, 94%, 95%, 96%, 97%, 98%, or 99% identity thereto, and the VL comprises or consists of an amino acid sequence as set forth in SEQ ID NO: 15 or an amino acid sequence having at least 90%, 91%, 92%, 93%, 94%, 95%, 96%, 97%, 98%, or 99% identity thereto.
**Embodiments11.** The use according to embodiment 9 or 10, wherein the heavy chain comprises or consists of the following amino acid sequence: an amino acid sequence as set forth in SEQ ID NO: 14, or an amino acid sequence comprising a Hole mutation and having at least 85%, 90%, 91%, 92%, 93%, 94%, 95%, 96%, 97%, 98%, or 99% identity to the amino acid sequence as set forth in SEQ ID NO: 14; the light chain comprises or consists of the following amino acid sequence: an amino acid sequence as set forth in SEQ ID NO: 20, or an amino acid sequence having at least 85%, 90%, 91%, 92%, 93%, 94%, 95%, 96%, 97%, 98%, or 99% identity to the amino acid sequence as set forth in SEQ ID NO: 20.
**Embodiments12.** The use according to embodiment 1, wherein
   the first monomer comprises or consists of the amino acid sequence as set forth in SEQ ID NO: 7;
   the heavy chain of the second monomer comprises or consists of the amino acid sequence as set forth in SEQ ID NO: 14;
   the light chain of the second monomer comprises or consists of the amino acid sequence as set forth in SEQ ID NO: 20.
**Embodiments13.** The use according to any one of the preceding embodiments, wherein the fusion protein is administered by injection, preferably intravenous injection, according to the following dosage regimen: a dose level of 0.2 µg/kg to 3 mg/kg body weight, for example 0.2 µg/kg, 2 µg/kg, 10 µg/kg, 30 µg/kg, 0.1 mg/kg, 0.3 mg/kg, 0.6 mg/kg, 1 mg/kg, 1.5 mg/kg, 2 mg/kg, 2.5 mg/kg, 3 mg/kg body weight, or a range composed of any two thereof.
**Embodiments14.** The use according to any one of the preceding embodiments, wherein the fusion protein is administered by injection, preferably intravenous injection, according to the following dosage regimen: a dose of 0.2 µg/kg to 3 mg/kg body weight, for example 0.2 µg/kg, 2 µg/kg, 10 µg/kg, 30 µg/kg, 0.1 mg/kg, 0.3 mg/kg, 0.6 mg/kg, 1 mg/kg, 1.5 mg/kg, 2 mg/kg, 2.5 mg/kg, 3 mg/kg body weight, or a range composed of any two thereof, for example a dose of 30 µg/kg-3 mg/kg, 0.1-3 mg/kg, 0.1-2 mg/kg, 0.6-3 mg/kg, 0.6-2 mg/kg, 1-3 mg/kg, or 1-2 mg/kg body weight administered once weekly (QW), once every two weeks (Q2W), or once every three weeks (Q3W); wherein the fusion protein is administered for one or more treatment cycles, and the treatment cycle is three weeks for once weekly or once every three weeks administration and four weeks for once every two weeks administration.
**Embodiments15.** The use according to any one of the preceding embodiments, wherein the fusion protein is administered by injection, preferably intravenous injection, according to the following dosage regimen:
   1-3 mg/kg or 1-2 mg/kg body weight administered once weekly (QW), once every two weeks (Q2W), or once every three weeks (Q3W), wherein the fusion protein is administered for one or more treatment cycles, and the treatment cycle is three weeks for once weekly or once every three weeks administration, and four weeks for once every two weeks administration;
   preferably, 0.3, 0.6, 1, or 2 mg/kg body weight administered once every two weeks (Q2W), wherein the fusion protein is administered for one or more treatment cycles, and the treatment cycle is four weeks; or
   0.6, 1, 1.5, 2, or 3 mg/kg body weight administered once every three weeks (Q3W), wherein the fusion protein is administered for one or more treatment cycles, and the treatment cycle is three weeks.
**Embodiments16.** The use according to any one of the preceding embodiments, wherein when a single dose is ≥0.6 mg/kg body weight, a dose of the fusion protein of ≤200 µg/kg body weight is administered once by injection, preferably intravenous injection, 7±1 days before a first single dose as a priming dose.
**Embodiments17.** The use according to embodiment 16, wherein the fusion protein is administered by injection, preferably intravenous injection, according to the following dosage regimen: a priming dose of 0.2-200 µg/kg body weight administered on day 1, and from day 8±1, a normal dose of 0.6-3 mg/kg body weight administered once weekly (QW), once every two weeks (Q2W), or once every three weeks (Q3W).
**Embodiments18.** The use according to embodiments 16-17, wherein the priming dose is 2-200 µg/kg body weight, preferably 30-200 µg/kg body weight, more preferably 30-150 µg/kg body weight, most preferably 30-100 µg/kg body weight.
**Embodiments19.** The use according to embodiment 17 or 18, wherein the normal dose is 0.6-3 mg/kg, or 0.6-2 mg/kg, or 1-3 mg/kg, or 1-2 mg/kg body weight administered once weekly (QW), once every two weeks (Q2W), or once every three weeks (Q3W), and wherein the normal dose of the fusion protein is administered for one or more treatment cycles, and the treatment cycle is three weeks for once weekly or once every three weeks administration, and four weeks for once every two weeks administration.
**Embodiments20.** The use according to any one of embodiments 17-19, wherein the fusion protein is administered by injection, preferably intravenous injection, according to the following dosage regimen: a priming dose of 30-100 µg/kg body weight administered on day 1, and from day 8±1, a normal dose of 0.6-3 mg/kg, for example 0.6-2 mg/kg, for example 1-3 mg/kg, for example 1-2 mg/kg body weight administered once weekly (QW), once every two weeks (Q2W), or once every three weeks (Q3W), wherein the normal dose of the fusion protein is administered for one or more treatment cycles, and the treatment cycle is three weeks for once weekly or once every three weeks administration, and four weeks for once every two weeks administration.
**Embodiments21.** The use according to any one of embodiments 17-20, wherein the fusion protein is administered by injection, preferably intravenous injection, according to the following dosage regimen:
   a priming dose of 100 µg/kg body weight administered on day 1, and from day 8±1, a normal dose of 0.6, 1, or 2 mg/kg body weight administered once every two weeks (Q2W), wherein the normal dose of the fusion protein is administered for one or more treatment cycles, and the treatment cycle is four weeks; or
   a priming dose of 100 µg/kg body weight administered on day 1, and from day 8±1, a normal dose of 0.6, 1, 1.5, 2, or 3 mg/kg body weight administered once every three weeks (Q3W), wherein the normal dose of the fusion protein is administered for one or more treatment cycles, and the treatment cycle is three weeks.
**Embodiments22.** The use according to any one of embodiments 14-21, wherein when there is more than one treatment cycle, the treatment cycles may be consecutive or separated.
**Embodiments23.** The use according to any one of the preceding embodiments, wherein the solid tumor or hematologic tumor is selected from intestinal cancer (comprising rectal cancer, colon cancer, colorectal cancer), melanoma, lung cancer (for example non-small cell lung cancer, comprising squamous and non-squamous non-small cell lung cancer), breast cancer (for example triple-negative breast cancer), pancreatic cancer, cholangiocarcinoma, ovarian cancer, cervical cancer, uterine cancer, head and neck cancer (for example head and neck squamous cell carcinoma), liver cancer (for example hepatocellular carcinoma), renal cancer (for example renal cell carcinoma), stomach cancer, lymphoma, leukemia, or multiple myeloma;
   preferably, the solid tumor or hematologic tumor is selected from colorectal cancer, melanoma, non-small cell lung cancer (comprising squamous and non-squamous non-small cell lung cancer), breast cancer, pancreatic cancer, cholangiocarcinoma, ovarian cancer, cervical cancer, head and neck cancer (for example head and neck squamous cell carcinoma), hepatocellular carcinoma, renal cell carcinoma, and lymphoma;
   more preferably, the solid tumor or hematologic tumor is selected from colorectal cancer, melanoma, and non-small cell lung cancer (comprising squamous and non-squamous non-small cell lung cancer).
**Embodiments24.** The use according to any one of the preceding embodiments, wherein the solid tumor or hematologic tumor is standard therapy-failed or standard therapy-intolerant, advanced, recurrent, metastatic, or unresectable.
**Embodiments25.** The use according to any one of the preceding embodiments, wherein the solid tumor or hematologic tumor is selected from:
   melanoma, for example cutaneous melanoma, acral melanoma, mucosal melanoma, or melanoma of unknown primary origin, particularly those that are failed or intolerant to standard therapy, advanced, recurrent, metastatic, or unresectable; for example, unresectable locally advanced or metastatic melanoma that has progressed or recurred after prior immune checkpoint inhibitor treatment, or unresectable locally advanced or metastatic melanoma untreated by systemic immune checkpoint inhibitor therapy;
   colorectal cancer, preferably colorectal cancer that is failed or intolerant to standard therapy, advanced, recurrent, metastatic, or unresectable, particularly colorectal cancer with proficient mismatch repair (pMMR) and/or microsatellite stable (MSS) status; and
   non-small cell lung cancer, comprising squamous and non-squamous non-small cell lung cancer, particularly non-small cell lung cancer without known driver gene mutation, failed or intolerant to standard therapy, advanced, recurrent, metastatic, or unresectable.
**Embodiments26.** A method of treating a solid tumor or a hematologic tumor in a subject, wherein the method comprises administering to the subject in need thereof an effective amount of the PD-1/IL-2R bispecific antibody fusion protein as defined in any one of embodiments 1-12.
**Embodiments27.** The method according to embodiment 26, wherein the PD-1/IL-2R bispecific antibody fusion protein is administered by injection, preferably intravenous injection, according to the dosage regimen as defined in any one of embodiments 13-22.
**Embodiments28.** The method according to embodiment 26, wherein the fusion protein is administered by injection, preferably intravenous injection, according to the following dosage regimen:
   a priming dose of 100 µg/kg body weight administered on day 1, and from day 8±1, a normal dose of 0.6, 1, or 2 mg/kg body weight administered once every two weeks (Q2W), wherein the normal dose of the fusion protein is administered for one or more treatment cycles, and the treatment cycle is four weeks; or
   a priming dose of 100 µg/kg body weight administered on day 1, and from day 8±1, a normal dose of 0.6, 1, 1.5, 2, or 3 mg/kg body weight administered once every three weeks (Q3W), wherein the normal dose of the fusion protein is administered for one or more treatment cycles, and the treatment cycle is three weeks.
**Embodiments29.** The method according to any one of embodiments 26-28, wherein the solid tumor or the hematologic tumor is as defined in any one of embodiments 23-25.
**Embodiments30.** A pharmaceutical composition for treating a solid tumor or a hematologic tumor, comprising the PD-1/IL-2R bispecific antibody fusion protein as defined in any one of embodiments 1-12, wherein the pharmaceutical composition is administered by injection, preferably intravenous injection, according to the dosage regimen as defined in any one of embodiments 13-22. Preferably, the pharmaceutical composition is for treating the solid tumor or the hematologic tumor as defined in any one of embodiments 23-25.

The present invention further provides the following embodiments:
**Embodiments31.** A pharmaceutical combination for treating a solid tumor or a hematologic tumor, wherein the pharmaceutical combination comprises:
   (i) a first active ingredient, wherein the first active ingredient is a PD-1/IL-2R bispecific antibody fusion protein, and the PD-1/IL-2R bispecific antibody fusion protein comprises: (a) a first monomer comprising an IL-2 mutein fused with an Fc fragment, optionally via or not via a linker, wherein the IL-2 mutein has T3A+N88R+S130R mutations and IL15 B'C' loop region AGDASIH relative to wild-type IL-2 (for example an amino acid sequence as set forth in SEQ ID NO: 3); and (b) a second monomer comprising a heavy chain and a light chain of an antibody that specifically binds PD-1; and
   (ii) a second active ingredient, wherein the second active ingredient is bevacizumab.
**Embodiments32.** The pharmaceutical combination according to embodiment 31, wherein the IL-2 mutein comprises or consists of the following amino acid sequence:
   an amino acid sequence as set forth in SEQ ID NO: 4; or
   an amino acid sequence having at least 90%, 91%, 92%, 93%, 94%, 95%, 96%, 97%, 98%, or 99% identity to the amino acid sequence as set forth in SEQ ID NO: 4 and having T3A+N88R+S130R mutations and IL15B'C' loop region AGDASIH relative to wild-type IL-2 (for example, an amino acid sequence as set forth in SEQ ID NO: 3).
**Embodiments33.** The pharmaceutical combination according to embodiment 31, wherein the linker comprises (GGGGS)n, wherein n is an integer of at least 1, for example 1, 2, 3, or 4, for example SEQ ID NO: 5.
**Embodiments34.** The pharmaceutical combination according to any one of embodiments 31-33, wherein the heavy chain of the antibody that specifically binds PD-1 comprises an Fc fragment, and the Fc fragment as well as the Fc fragment fused to the IL-2 mutein is an Fc fragment of IgG1, IgG2, IgG3, or IgG4, for example human IgG1 Fc.
**Embodiments35.** The pharmaceutical combination according to embodiment 34, wherein the Fc fragment has mutations that bind to an Fcγ receptor, for example L234A/L235A mutations or L234A/L235E/G237A.
**Embodiments36.** The pharmaceutical combination according to embodiment 34 or 35, wherein the Fc fragment of the first monomer comprises a Knob mutation, and the antibody heavy-chain Fc fragment of the second monomer contains a Hole mutation, or the Fc fragment of the first monomer comprises a Hole mutation, and the antibody heavy-chain Fc fragment of the second monomer comprises a Knob mutation; for example, the hole mutations are Y349C, T366S, L368A, and Y407V mutations, and/or the Knob mutations are T366W and S354C mutations.
**Embodiments37.** The pharmaceutical combination according to embodiment 36, wherein
   the Fc fragment of the first monomer comprises or consists of the following amino acid sequence: an amino acid sequence as set forth in SEQ ID NO: 6, or an amino acid sequence having at least 90%, 91%, 92%, 93%, 94%, 95%, 96%, 97%, 98%, or 99% identity to the amino acid sequence as set forth in SEQ ID NO: 6;
      or
   the antibody heavy-chain Fc fragment of the second monomer comprises or consists of the following amino acid sequence: an amino acid sequence as set forth in SEQ ID NO: 12, or an amino acid sequence having at least 90%, 91%, 92%, 93%, 94%, 95%, 96%, 97%, 98%, or 99% identity to the amino acid sequence as set forth in SEQ ID NO: 12.
**Embodiments38.** The pharmaceutical combination according to any one of embodiments 31-36, wherein the first monomer comprises:
   an amino acid sequence as set forth in SEQ ID NO: 7; or
   an amino acid sequence having at least 90%, 91%, 92%, 93%, 94%, 95%, 96%, 97%, 98% or 99% identity to the amino acid sequence as set forth in SEQ ID NO: 7 and T3A+N88R+S130R mutations and IL15B'C' loop region AGDASIH relative to wild-type IL-2 (for example, an amino acid sequence as set forth in SEQ ID NO: 3) in the IL-2 mutein, and/or S354C/T366W and optionally L234A/L235A mutations in the Fc fragment.
**Embodiments39.** The pharmaceutical combination according to any one of embodiments 31-37, wherein the heavy chain of the antibody that specifically binds PD-1 comprises HCDR1, HCDR2, and HCDR3, and the light chain of the antibody that specifically binds PD-1 comprises LCDR1, LCDR2, and LCDR3, wherein the amino acid sequences of HCDR1, HCDR2, and HCDR3 are respectively: SEQ ID NO: 9, 10, and 11, and the amino acid sequences of LCDR1, LCDR2, and LCDR3 are respectively: SEQ ID NO: 16, 17, and 18.
**Embodiments40.** The pharmaceutical combination according to embodiment 39, wherein the heavy chain of the antibody that specifically binds PD-1 comprises a heavy-chain variable region VH, and the light chain of the antibody that specifically binds PD-1 comprises a light-chain variable region VL, wherein the VH comprises or consists of an amino acid sequence as set forth in SEQ ID NO: 8 or an amino acid sequence having at least 90%, 91%, 92%, 93%, 94%, 95%, 96%, 97%, 98%, or 99% identity thereto, and the VL comprises or consists of an amino acid sequence as set forth in SEQ ID NO: 15 or an amino acid sequence having at least 90%, 91%, 92%, 93%, 94%, 95%, 96%, 97%, 98%, or 99% identity thereto.
**Embodiments41.** The pharmaceutical combination according to embodiment 39 or 40, wherein the heavy chain comprises or consists of the following amino acid sequence: an amino acid sequence as set forth in SEQ ID NO: 14, or an amino acid sequence comprising a Hole mutation and having at least 85%, 90%, 91%, 92%, 93%, 94%, 95%, 96%, 97%, 98%, or 99% identity to the amino acid sequence as set forth in SEQ ID NO: 14; the light chain comprises or consists of the following amino acid sequence: an amino acid sequence as set forth in SEQ ID NO: 20, or an amino acid sequence having at least 85%, 90%, 91%, 92%, 93%, 94%, 95%, 96%, 97%, 98%, or 99% identity to the amino acid sequence as set forth in SEQ ID NO: 20.
**Embodiments42.** The pharmaceutical combination according to embodiment 31, wherein
   the first monomer comprises or consists of the amino acid sequence as set forth in SEQ ID NO: 7;
   the heavy chain of the second monomer comprises or consists of the amino acid sequence as set forth in SEQ ID NO: 14;
   the light chain of the second monomer comprises or consists of the amino acid sequence as set forth in SEQ ID NO: 20.
**Embodiments43.** The pharmaceutical combination according to any one of embodiments 31 to 42, wherein the first active ingredient is for injection administration, for example intravenous infusion; the second active ingredient is for injection administration, for example intravenous infusion.
**Embodiments44.** The pharmaceutical combination according to any one of embodiments 31-42, wherein the first active ingredient is administered by injection, preferably intravenous injection, according to the dosage regimen as defined in any one of embodiments 13-22; and the second active ingredient is for intravenous infusion administration at a dose of 5 mg/kg body weight to 15 mg/kg body weight administered once every two weeks with every four weeks constituting one treatment cycle, or once every three weeks with every three weeks constituting one treatment cycle, for one or more treatment cycles.
**Embodiments45.** The pharmaceutical combination according to embodiment 44,
   the first active ingredient is administered for injection, preferably intravenous injection as follows: a dose of 0.6-3 mg/kg, for example 0.6-2 mg/kg, for example 1-3 mg/kg, or for example 1-2 mg/kg body weight administered once weekly (QW), once every two weeks (Q2W), or once every three weeks (Q3W), for one or more treatment cycles with each treatment cycle being three weeks; or a priming dose of 30-100 µg/kg body weight administered on day 1, and from day 8±1, a normal dose of 0.6-3 mg/kg, for example 0.6-2 mg/kg, for example 1-3 mg/kg, or for example 1-2 mg/kg body weight administered once weekly (QW), once every two weeks (Q2W), or once every three weeks (Q3W), wherein the normal dose is administered for one or more treatment cycles, and each treatment cycle is three weeks; and
   the second active ingredient is for intravenous infusion administration at a dose of 5-15 mg/kg body weight (for example 5, 7.5, 10, 12.5, or 15 mg/kg body weight or a range composed of any two thereof) administered once every two weeks with each four weeks constituting a treatment cycle, or once every three weeks with each three weeks constituting a treatment cycle, for one or more treatment cycles.
**Embodiments46.** The pharmaceutical combination according to any one of embodiments 31-42, wherein
   the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
   the second active ingredient is for intravenous infusion administration at a dose of 5 mg/kg to 15 mg/kg body weight administered once every two weeks with each four weeks constituting a treatment cycle, or once every three weeks with each three weeks constituting a treatment cycle.
**Embodiments47.** The pharmaceutical combination according to any one of embodiments 31-46, wherein the solid tumor or the hematologic tumor is selected from intestinal cancer (comprising rectal cancer, colon cancer, colorectal cancer), melanoma, non-small cell lung cancer (comprising squamous and non-squamous non-small cell lung cancer), hepatocellular carcinoma, renal cell carcinoma, lymphoma, or other advanced solid tumors.
**Embodiments48.** The pharmaceutical combination according to embodiment 47, wherein the solid tumor is colorectal cancer, preferably advanced colorectal cancer, more preferably advanced colorectal cancer failed or intolerant to standard therapy;
   the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
   the second active ingredient is for intravenous infusion administration at a dose of 5 mg/kg to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg to 10 mg/kg body weight (for example 5 mg/kg or 7.5 mg/kg) administered once every two weeks with each four weeks constituting a treatment cycle, or once every three weeks with each three weeks constituting a treatment cycle; especially 7.5 mg/kg administered once every three weeks (Q3W).
**Embodiments49.** The pharmaceutical combination according to embodiment 47, wherein the solid tumor is melanoma, preferably advanced melanoma, more preferably advanced melanoma untreated with immunotherapy or advanced melanoma with prior immunotherapy failure;
   the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
   the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg body weight, 7.5 mg/kg, 10 mg/kg, 12.5 mg/kg body weight, or 15 mg/kg body weight administered once every two weeks with each four weeks constituting a treatment cycle, or once every three weeks with each three weeks constituting a treatment cycle.
**Embodiments50.** The pharmaceutical combination according to embodiment 47, wherein the solid tumor is non-small cell lung cancer, for example squamous or non-squamous non-small cell lung cancer (preferably advanced non-squamous non-small cell lung cancer, more preferably advanced non-squamous non-small cell lung cancer failed or intolerant to standard therapy), hepatocellular carcinoma (preferably advanced hepatocellular carcinoma, more preferably advanced hepatocellular carcinoma untreated with systemic therapy), or renal cell carcinoma (preferably advanced renal cell carcinoma, more preferably advanced renal cell carcinoma failed or intolerant to standard therapy);
   the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
   the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 10 mg/kg body weight to 15 mg/kg body weight (for example 10 mg/kg body weight, 12.5 mg/kg body weight, or 15 mg/kg body weight) administered once every two weeks with each four weeks constituting a treatment cycle, or once every three weeks with each three weeks constituting a treatment cycle.
**Embodiments51.** The pharmaceutical combination according to embodiment 47, wherein the solid tumor is lymphoma and other solid tumors, preferably lymphoma or other advanced solid tumors failed or intolerant to standard therapy;
   the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
   the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg body weight, 7.5 mg/kg, 10 mg/kg, 12.5 mg/kg body weight, or 15 mg/kg body weight administered once every two weeks with each four weeks constituting a treatment cycle, or once every three weeks with each three weeks constituting a treatment cycle.
**Embodiments52.** A pharmaceutical combination for treating melanoma, wherein the pharmaceutical combination comprises:
   (i) a first active ingredient, wherein the first active ingredient is the PD-1/IL-2R bispecific antibody fusion protein as defined in any one of embodiments 1-12 or embodiments 31-42; and
   (ii) a second active ingredient, wherein the second active ingredient is dacarbazine.
**Embodiments53.** The pharmaceutical combination according to embodiment 52, wherein the first active ingredient is for injection administration, for example intravenous infusion, and is administered by injection, preferably intravenous injection, according to the dosage regimen defined in any one of embodiments 13-22; the second active ingredient is administered by injection, for example intravenous infusion, intravenous bolus, intraarterial infusion, or intraarterial bolus.
**Embodiments54.** The pharmaceutical combination according to embodiment 53, wherein
   the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
   the second active ingredient dacarbazine is administered by intravenous or intraarterial infusion, and when administered by intravenous or intraarterial infusion, the dacarbazine is administered at a dose of 2.5-6 mg/kg body weight or 200-400 mg/m² body surface area once daily for 5-10 consecutive days, followed by a drug-free interval, with each 3-6 weeks constituting a treatment cycle; or a dose of 650-1450 mg/m² body surface area once every 4-6 weeks, correspondingly with each 4-6 weeks constituting a treatment cycle; when administered by intravenous or intraarterial bolus, the dacarbazine is administered at a dose of 200-400 mg/m² once daily for 5 consecutive days, followed by a drug-free interval, with each 3-4 weeks constituting a treatment cycle.
**Embodiments55.** The pharmaceutical combination according to any one of embodiments 52-54, wherein the melanoma is advanced melanoma untreated with systemic therapy; the second active ingredient dacarbazine is for intravenous infusion administration at a dose of 850 mg/m² body surface area administered once every four weeks with each four weeks constituting a treatment cycle; or for intravenous infusion administration at a dose of 650-1050 mg/m² body surface area (for example, 850 mg/m² body surface area) administered once every three weeks with each three weeks constituting a treatment cycle; or for intravenous bolus administration at a dose of 200-300 mg/m² body surface area (for example, 250 mg/m² body surface area) administered once daily for 5 consecutive days, followed by a drug-free interval, with each four weeks constituting a treatment cycle; or for intravenous bolus administration at a dose of 200-300 mg/m² body surface area (for example, 250 mg/m² body surface area) administered once daily for 5 consecutive days, followed by a drug-free interval, with each three weeks constituting a treatment cycle.
**Embodiments56.** A pharmaceutical combination for treating non-small cell lung cancer, wherein the pharmaceutical combination comprises:
   (i) a first active ingredient, wherein the first active ingredient is the PD-1/IL-2R bispecific antibody fusion protein as defined in any one of embodiments 1-12 or embodiments 31-42; and
   (ii) a second active ingredient, wherein the second active ingredient is a compound of formula (II) or a pharmaceutically acceptable salt thereof, 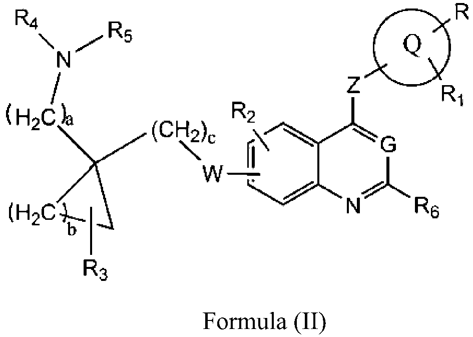 wherein
      W is O; Z is O; G is CR; a is 0; c is 1; b is 1; R is H or C₁₋₆ alkyl; 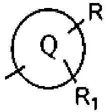 is 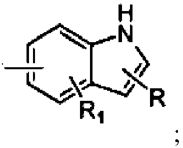
      R₁, R₂, R₃ are each independently selected from H, halogen, and C₁₋₆ alkoxy;
      R₄ and R₅ are each independently selected from H or C₁₋₆ alkyl;
      R₆ is H.
**Embodiments57.** The pharmaceutical combination according to embodiment 56, wherein the second active ingredient is anlotinib or a pharmaceutically acceptable salt thereof, for example anlotinib dihydrochloride.
**Embodiments58.** The pharmaceutical combination according to embodiment 55 or 57, wherein the first active ingredient is for injection administration, for example intravenous infusion, and is administered by injection, preferably intravenous injection, according to the dosage regimen defined in any one of embodiments 13-22; the second active ingredient is for oral administration.
**Embodiments59.** The pharmaceutical combination according to embodiment 58, wherein
   the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
   the second active ingredient is for oral administration at a dose of 8 mg to 12 mg administered once daily for 2 consecutive weeks, followed by a drug-free interval of 1 week, with each three weeks constituting a treatment cycle.
**Embodiments60.** The pharmaceutical combination according to any one of embodiments 56-59, wherein the non-small cell lung cancer is advanced non-small cell lung cancer, preferably advanced non-small cell lung cancer failed or intolerant to standard therapy; the second active ingredient (for example anlotinib dihydrochloride) is for oral administration at a dose of 12 mg (when the second active ingredient is a pharmaceutically acceptable salt of anlotinib for example dihydrochloride of anlotinib, calculated as anlotinib) administered once daily for 2 consecutive weeks, followed by a drug-free interval of 1 week, with each three weeks constituting a treatment cycle.
**Embodiments61.** The pharmaceutical combination according to any one of embodiments 31-60, wherein the pharmaceutical combination comprises the first active ingredient and the second active ingredient as the only active ingredients.
**Embodiments62.** The pharmaceutical combination according to any one of embodiments 31-61, wherein the pharmaceutical combination is in the form of a kit or pack, and the kit or pack comprises: a pharmaceutical composition comprising the first active ingredient; a pharmaceutical composition comprising the second active ingredient; and an instruction for use, wherein the instruction describes administration methods of the two pharmaceutical compositions.
**Embodiments63.** Use of the pharmaceutical combination according to any one of embodiments 31-61 in the preparation of a medicament.
**Embodiments64.** A method for treating cancer, wherein the method comprises administering the pharmaceutical combination according to any one of embodiments 31-61, wherein the first active ingredient and the second active ingredient are administered simultaneously in a single pharmaceutical composition, or administered simultaneously or separately in separate pharmaceutical compositions.

In various aspects or embodiments of the present disclosure, the PD-1/IL-2R bispecific antibody fusion protein of the present invention comprises: (a) a first monomer, which comprises an IL-2 mutein fused to an Fc fragment, optionally via or not via a linker; and (b) a second monomer, which comprises one heavy chain and one light chain of an antibody that specifically binds PD-1.

In some embodiments, the IL-2 mutein, relative to wild-type IL-2 (preferably human IL-2, more preferably IL-2 comprising the sequence of SEQ ID NO: 3), comprises: (i) N88D; N88R; N88R + S130R; F42A+N88R + S127E; F42A+N88R + S127E; or K35E+N88R + S127E; and (ii) a B'C' loop region replaced with an IL15B'C' loop region AGDASIH (SEQ ID NO: 25) or AQSKNFH (SEQ ID NO: 26); and optionally (iii) T3A.

In some embodiments, the IL-2 mutein has T3A+N88R+S130R mutations and an IL15B'C' loop region replacement relative to wild-type IL-2 (for example, the amino acid sequence as set forth in SEQ ID NO: 3) (i.e., the B'C' loop region sequence is replaced with AGDASIH (SEQ ID NO: 25)).

In some embodiments, the IL-2 mutein comprises or consists of the following amino acid sequence: the amino acid sequence as set forth in SEQ ID NO: 4, or an amino acid sequence having at least 90%, 91%, 92%, 93%, 94%, 95%, 96%, 97%, 98%, or 99% identity to the amino acid sequence as set forth in SEQ ID NO: 4. In some embodiments, the IL-2 mutein comprises an amino acid sequence having at least 90%, 91%, 92%, 93%, 94%, 95%, 96%, 97%, 98%, or 99% identity to the amino acid sequence as set forth in SEQ ID NO: 4 and comprising T3A+N88R+S130R mutations and an IL15B'C' loop region AGDASIH relative to wild-type IL-2 (for example, the amino acid sequence as set forth in SEQ ID NO: 3).

In some embodiments, the PD-1/IL-2R bispecific antibody fusion protein comprises or consists of the following: (a) a first monomer, which comprises or consists of the IL-2 mutein of the present invention fused to an Fc fragment, optionally fused via or not via a linker; and (b) a second monomer, which comprises or consists of an antibody that specifically binds PD-1 or a fragment thereof, preferably, the fragment comprises or consists of one heavy chain and one light chain of the anti-PD-1 antibody.

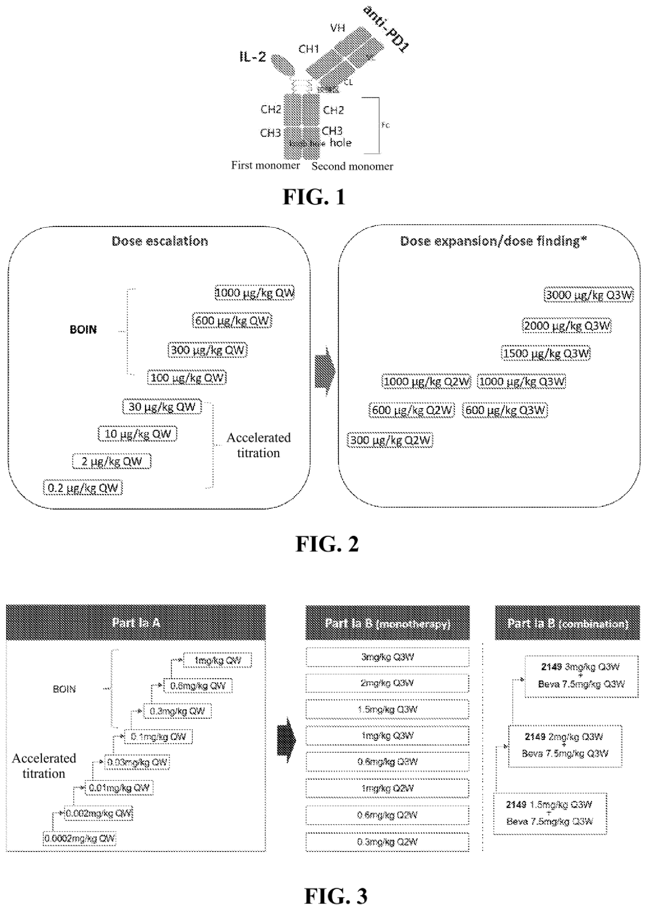

In some embodiments, the molecular form of the PD-1/IL-2R bispecific antibody fusion protein is as shown in FIG. 1.

In some embodiments, the heavy chain of the anti-PD-1 antibody comprises an Fc fragment.

In some embodiments, the linker may be selected from the following linker sequences: (GS)n, (GSGGS)n, (GGGGS)n, and (GGGS)n, wherein n is an integer of at least 1, for example 1, 2, 3, or 4. Preferably, the linker comprises (G₄S)₂, i.e., GGGGSGGGGS (SEQ ID NO: 5).

In some embodiments, the Fc fragment is an Fc fragment of IgG1, IgG2, IgG3, or IgG4. In some embodiments, the Fc fragment is human IgG Fc, for example human IgG1 Fc, human IgG2 Fc, or human IgG4 Fc. In some embodiments, the Fc fragment comprises the amino acid sequence SEQ ID NO: 24 or an amino acid sequence having at least 90%, 91%, 92%, 93%, 94%, 95%, 96%, 97%, 98%, or 99% identity thereto, or consists thereof.

In some embodiments, the Fc fragment has mutations reducing binding to Fcγ receptor, for example L234A/L235A mutations or L234A/L235E/G237A. In some embodiments, the Fc fragment comprises the amino acid sequence SEQ ID NO: 23 or an amino acid sequence having at least 90%, 91%, 92%, 93%, 94%, 95%, 96%, 97%, 98%, or 99% identity thereto, or consists thereof. In some embodiments, the Fc fragment comprises an amino acid sequence having at least 90%, 91%, 92%, 93%, 94%, 95%, 96%, 97%, 98%, or 99% identity to the amino acid sequence SEQ ID NO: 23 and having L234A/L235A mutations.

In some embodiments, the Fc fragment may comprise mutations facilitating dimerization of the first monomer and the second monomer. Preferably, based on Knob-in-Hole technology, corresponding Knob mutations and Hole mutations are introduced into the first monomer and the second monomer. In some embodiments, the Fc fragment of the first monomer comprises Knob mutations and the antibody heavy-chain Fc fragment of the second monomer comprises Hole mutations, or the Fc fragment of the first monomer comprises Hole mutations and the antibody heavy-chain Fc fragment of the second monomer comprises Knob mutations. In some embodiments, the Knob mutations include T366W. In some embodiments, the hole mutations include T366S, L368A, and Y407V. In some embodiments, the Knob mutations and Hole mutations in the first monomer and the second monomer further include cysteine mutations (for example, one monomer comprises Y349C and the other monomer comprises S354C), thereby forming a non-natural disulfide bond.

In some embodiments, in the PD-1/IL-2R bispecific antibody fusion protein,
a) one Fc-region polypeptide comprises mutation T366W, and the other Fc-region polypeptide comprises mutations T366S, L368A, and Y407V; or
b) one Fc-region polypeptide comprises mutations T366W and Y349C, and the other Fc-region polypeptide comprises mutations T366S, L368A, Y407V, and S354C; or
c) one Fc-region polypeptide comprises mutations T366W and S354C, and the other Fc-region polypeptide comprises mutations T366S, L368A, Y407V, and Y349C.

In some embodiments, one Fc region comprises or consists of the following sequence:
(i) the amino acid sequence as set forth in SEQ ID NO: 6, or
(ii) an amino acid sequence having at least 90%, 91%, 92%, 93%, 94%, 95%, 96%, 97%, 98%, or 99% identity to the amino acid sequence as set forth in SEQ ID NO: 6; or
(ii) an amino acid sequence having at least 90%, 91%, 92%, 93%, 94%, 95%, 96%, 97%, 98%, or 99% identity to the amino acid sequence as set forth in SEQ ID NO: 6 and comprising S354C/T366W;
optionally, the Fc region further comprises L234A/L235A mutations.

In some embodiments, the other Fc region comprises or consists of the following sequence:
(i) the amino acid sequence as set forth in SEQ ID NO: 12, or
(ii) an amino acid sequence having at least 90%, 91%, 92%, 93%, 94%, 95%, 96%, 97%, 98%, or 99% identity to the amino acid sequence as set forth in SEQ ID NO: 12; or
(ii) an amino acid sequence having at least 90%, 91%, 92%, 93%, 94%, 95%, 96%, 97%, 98%, or 99% identity to the amino acid sequence as set forth in SEQ ID NO: 12 and comprising Y349C/T366S/L368A/Y407V;
optionally, the Fc region further comprises L234A/L235A mutations.

In some embodiments, wherein the IL-2 mutein fused to an Fc fragment comprises the amino acid sequence as set forth in SEQ ID NO: 7 or an amino acid sequence having at least 90%, 91%, 92%, 93%, 94%, 95%, 96%, 97%, 98%, or 99% identity thereto, or consists thereof. In some embodiments, the IL-2 mutein fused to an Fc fragment comprises an amino acid sequence having at least 90%, 91%, 92%, 93%, 94%, 95%, 96%, 97%, 98%, or 99% identity to the amino acid sequence as set forth in SEQ ID NO: 7, and comprises T3A+N88R+S130R mutations and an IL15B'C' loop region AGDASIH in the IL-2 mutein relative to wild-type IL-2 (for example, the amino acid sequence as set forth in SEQ ID NO: 3), and/or comprises S354C/T366W in the Fc fragment, and optionally L234A/L235A mutations.

In some embodiments, the second monomer comprises an antibody that specifically binds PD-1 or an antigen-binding fragment thereof, for example an antibody fragment selected from the group consisting of Fab, Fab', Fab'-SH, Fv, single-chain antibody (for example scFv), (Fab')2, single-domain antibody for example VHH, dAb (domain antibody), or linear antibody or half antibody, so long as it can specifically bind PD-1.

In some embodiments, the second monomer comprises or consists of one heavy chain and one light chain of an antibody that specifically binds PD-1. In some embodiments, the heavy chain comprises a heavy-chain variable region VH of an antibody that specifically binds PD-1, and the light chain comprises a light-chain variable region VL of an antibody that specifically binds PD-1.

In some embodiments, the heavy-chain variable region VH comprises three complementarity determining regions HCDR1, HCDR2, and HCDR3 contained in the VH set forth in SEQ ID NO: 8. In some embodiments, the light-chain variable region VL comprises three complementarity determining regions LCDR1, LCDR2, and LCDR3 contained in the VL set forth in SEQ ID NO: 15. In some embodiments, the heavy-chain variable region comprises HCDR1, HCDR2, and HCDR3 of an antibody that specifically binds PD-1, which are respectively set forth in the following amino acid sequences: SEQ ID NO: 9, 10, and 11. In some embodiments, the light-chain variable region comprises LCDR1, LCDR2, and LCDR3 respectively set forth in the following amino acid sequences: SEQ ID NO: 16, 17, and 18.

In some embodiments, the VH comprises or consists of the amino acid sequence as set forth in SEQ ID NO: 8 or an amino acid sequence having at least 90%, 91%, 92%, 93%, 94%, 95%, 96%, 97%, 98%, or 99% identity thereto. In some embodiments, the VL comprises the amino acid sequence as set forth in SEQ ID NO: 15 or an amino acid sequence having at least 90%, 91%, 92%, 93%, 94%, 95%, 96%, 97%, 98%, or 99% identity thereto, or consists of the amino acid sequence.

In some embodiments, the second monomer comprises a heavy-chain variable region VH and a light-chain variable region VL of an antibody that specifically binds PD-1, which comprise three complementarity determining regions HCDR1, HCDR2, and HCDR3 contained in the VH set forth in SEQ ID NO: 8, and three complementarity determining regions LCDR1, LCDR2, and LCDR3 contained in the VL set forth in SEQ ID NO: 15.

In some embodiments, the second monomer comprises HCDR1, HCDR2, and HCDR3 of an antibody that specifically binds PD-1 respectively set forth in the following amino acid sequences: SEQ ID NO: 9, 10, and 11, and LCDR1, LCDR2, and LCDR3 respectively set forth in the following amino acid sequences: SEQ ID NO: 16, 17, and 18.

In some embodiments, the second monomer comprises the following VH and VL of an antibody that specifically binds PD-1: a VH comprising the amino acid sequence as set forth in SEQ ID NO: 8 or an amino acid sequence having at least 90%, 91%, 92%, 93%, 94%, 95%, 96%, 97%, 98%, or 99% identity thereto, or consisting of the amino acid sequence, and a VL comprising the amino acid sequence as set forth in SEQ ID NO: 15 or an amino acid sequence having at least 90%, 91%, 92%, 93%, 94%, 95%, 96%, 97%, 98%, or 99% identity thereto, or consisting of the amino acid sequence.

In some embodiments, the heavy chain comprises a heavy chain constant region. In some embodiments, the light chain comprises a light chain constant region. In some embodiments, the second monomer comprises a heavy chain constant region and/or a light chain constant region. In some embodiments, the heavy chain constant region HC is a heavy chain constant region of (human) IgG1, IgG2, IgG3, or IgG4, preferably a heavy chain constant region of human IgG1, for example a heavy chain constant region of IgG1 with L234A and L235A (LALA) mutations. In some embodiments, Knob into hole mutations are introduced into the heavy chain constant region, for example mutations S354C and T366W are introduced to obtain an antibody heavy chain constant region comprising Knob mutations, and/or mutations Y349C, T366S, L368A, and Y407V are introduced to obtain an antibody heavy chain constant region comprising Hole mutations. In some embodiments, the light chain constant region is a lambda or Kappa light chain constant region.

In some embodiments, the heavy chain constant region comprises an amino acid sequence having at least 85%, 90%, 91%, 92%, 93%, 94%, 95%, 96%, 97%, 98%, or 99% identity to an amino acid sequence selected from SEQ ID NO: 21, or consists of the amino acid sequence; or comprises or consists of an amino acid sequence selected from SEQ ID NO: 21. In some embodiments, the heavy chain constant region comprising Hole mutations comprises an amino acid sequence having at least 85%, 90%, 91%, 92%, 93%, 94%, 95%, 96%, 97%, 98%, or 99% identity to an amino acid sequence selected from SEQ ID NO: 13, or consists of the amino acid sequence; or comprises or consists of an amino acid sequence selected from SEQ ID NO: 13. In some embodiments, the light chain constant region comprises an amino acid sequence having at least 85%, 90%, 91%, 92%, 93%, 94%, 95%, 96%, 97%, 98%, or 99% identity to an amino acid sequence selected from SEQ ID NO: 19, or consists of the amino acid sequence; or comprises or consists of an amino acid sequence selected from SEQ ID NO19.

In some embodiments, the second monomer comprises the heavy chain and light chain of an antibody that specifically binds PD-1. In some embodiments, the heavy chain comprises or consists of a heavy-chain variable region and a heavy chain constant region (for example, a heavy chain constant region comprising Hole mutations) of an antibody that specifically binds PD-1. In some embodiments, the light chain comprises or consists of a light-chain variable region and a light chain constant region of an antibody that specifically binds PD-1.

In some embodiments, the second monomer comprises the heavy chain and light chain of an antibody that specifically binds PD-1, wherein the heavy chain comprises the amino acid sequence as set forth in SEQ ID NO: 14 or an amino acid sequence comprising a Hole mutation and having at least 85%, 90%, 91%, 92%, 93%, 94%, 95%, 96%, 97%, 98%, or 99% identity thereto, or consists of the amino acid sequence (optionally further comprising L234A/L235A mutations); the light chain comprises the amino acid sequence as set forth in SEQ ID NO: 20 or an amino acid sequence having at least 85%, 90%, 91%, 92%, 93%, 94%, 95%, 96%, 97%, 98%, or 99% identity thereto, or consists of the amino acid sequence.

In some embodiments of the present invention, the antibody against PD-1 or antigen-binding fragment thereof comprised in the second monomer is the anti-PD-1 antibody or antigen-binding fragment thereof disclosed in WO2017024465A1. In some embodiments, the anti-PD-1 antibody or antigen-binding fragment thereof comprises one or more CDRs (preferably 3 CDRs, i.e., HCDR1, HCDR2H, and HCDR3; or LCDR1, LCDR2, and LCDR3, more preferably 6 CDRs, i.e., HCDR1, HCDR2, HCDR3, LCDR1, LCDR2, and LCDR3) of the anti-PD-1 antibody or antigen-binding fragment thereof disclosed in WO2017024465A1, or comprises VH and/or VL of the anti-PD-1 antibody or antigen-binding fragment thereof disclosed in WO2017024465A1, or comprises the heavy chain and/or light chain of the antibody.

In some embodiments, the PD-1/IL-2R bispecific antibody fusion protein of the present invention comprises:
(a) a first monomer, which comprises or consists of the IL-2 mutein of the present invention fused to an Fc fragment via a linker; and
(b) a second monomer, which comprises or consists of one heavy chain and one light chain of an anti-PD-1 antibody;

wherein the first monomer comprises or consists of the amino acid sequence as set forth in SEQ ID NO: 7;
the heavy chain of the second monomer comprises or consists of the amino acid sequence as set forth in SEQ ID NO: 14;
the light chain of the second monomer comprises or consists of the amino acid sequence as set forth in SEQ ID NO: 20.

In some embodiments, the PD-1/IL-2R bispecific antibody fusion protein of the present invention is the immunoconjugate disclosed in WO2023/045977A1, particularly in the claims, the entire content of which patent application (including term definitions) is incorporated herein by reference in its entirety. The PD-1/IL-2R bispecific antibody fusion protein of the present invention is preferably immunoconjugate **3010**, **2061**, **2063**, **2132**, **2149**, **2213**, **2214**, or **2219** disclosed in WO2023/045977A1, and particularly preferably immunoconjugate **2149** disclosed in WO2023/045977A1 (also referred to herein as "PD-1/IL-2R bispecific antibody fusion protein **2149**" or "**2149**").

Fusion protein **2149** is a recombinant IgG1-Fc fusion protein bispecific molecule, targets PD-1 and IL2 receptor, consists of 1030 amino acids, and consists of the following
(a) a first monomer, which consists of the IL-2 mutein of the present invention fused to an Fc fragment via a linker; and
(b) a second monomer, which consists of one heavy chain and one light chain of an anti-PD-1 antibody;
wherein
the first monomer consists of the amino acid sequence as set forth in SEQ ID NO: 7;
the heavy chain of the second monomer consists of the amino acid sequence as set forth in SEQ ID NO: 14;
the light chain of the second monomer consists of the amino acid sequence as set forth in SEQ ID NO: 20;
wherein the first monomer and the heavy chain and light chain of the anti-PD-1 antibody are connected by 9 pairs of intrachain disulfide bonds and 4 pairs of interchain disulfide bonds, and a schematic structural diagram thereof is shown in FIG. 1.

In fusion protein **2149**, the anti-PD-1 heavy chain is of γ1 type and consists of 450 amino acids, the anti-PD-1 light chain is of κ type and consists of 214 amino acids, and the IL2 chain is an Fc fusion protein and consists of 366 amino acids. Fusion protein **2149** contains two N-glycosylation sites, respectively located at asparagine at position 300 of the anti-PD-1 heavy chain and asparagine at position 216 of the IL2 chain. The molecular formula of fusion protein **2149** is C₅₀₀₆H₇₇₉₆N₁₃₃₄O₁₅₅₄S₃₈ (disulfide bonds are in an oxidized state), the theoretical molecular weight is 115.4 kDa (G0F/G0F glycoform), and the theoretical extinction coefficient is 1.31 (mg/ml)⁻¹·cm⁻¹.

As used herein, the term "PD-1/IL-2R bispecific antibody fusion protein of the present invention" and "fusion protein of the present invention" are used interchangeably and mean those as defined herein.

As used herein, when referring to "fusion protein", unless otherwise specified, it also encompasses a pharmaceutical composition or pharmaceutical formulation comprising the fusion protein.

Therefore, when referring to molecule **2149**, unless otherwise specified, it encompasses both molecule **2149** itself and a pharmaceutical composition or pharmaceutical formulation comprising the same.

In some embodiments, the fusion protein of the present invention (for example **2149**) may be administered by any suitable route, preferably by a parenteral route, for example injection administration. For example, the fusion protein of the present invention (for example **2149**) may be administered by intravenous infusion (for example intravenous infusion or intravenous bolus administration), intramuscular injection, intratumoral injection, and the like. Preferably, the fusion protein of the present invention (for example **2149**) is administered by intravenous injection.

In some embodiments, the fusion protein of the present invention (for example **2149**) is administered by injection, preferably intravenous injection, according to the following dosage regimen: a dose level of 0.2 µg/kg body weight to 3 mg/kg body weight, for example 0.2 µg/kg, 2 µg/kg, 10 µg/kg, 30 µg/kg, 0.1 mg/kg, 0.3 mg/kg, 0.6 mg/kg, 1 mg/kg, 1.5 mg/kg, 2 mg/kg, 2.5 mg/kg, 3 mg/kg body weight, or a range composed of any two thereof. For example, the fusion protein of the present invention (for example **2149**) may be administered by injection, preferably intravenous injection, according to the following dosage regimen: dose levels of 0.2 µg/kg to 3 mg/kg, 0.2 µg/kg to 2.5 mg/kg, 0.2 µg/kg to 2 mg/kg, 0.2 µg/kg to 1.5 mg/kg, 0.2 µg/kg to 1 mg/kg, 2 µg/kg to 3 mg/kg, 2 µg/kg to 2.5 mg/kg, 2 µg/kg to 2 mg/kg, 2 µg/kg to 1.5 mg/kg, 2 µg/kg to 1 mg/kg, 10 µg/kg to 3 mg/kg, 10 µg/kg to 2.5 mg/kg, 10 µg/kg to 2 mg/kg, 10 µg/kg to 1.5 mg/kg, 10 µg/kg to 1 mg/kg, 30 µg/kg to 3 mg/kg, 30 µg/kg to 2.5 mg/kg, 30 µg/kg to 2 mg/kg, 30 µg/kg to 1.5 mg/kg, 30 µg/kg to 1 mg/kg, 0.1 mg/kg to 3 mg/kg, 0.1 mg/kg to 2.5 mg/kg, 0.1 mg/kg to 2 mg/kg, 0.1 mg/kg to 1.5 mg/kg, 0.1 mg/kg to 1 mg/kg, 0.3 mg/kg to 3 mg/kg, 0.3 mg/kg to 2.5 mg/kg, 0.3 mg/kg to 2 mg/kg, 0.3 mg/kg to 1.5 mg/kg, 0.3 mg/kg to 1 mg/kg, 0.6 mg/kg to 3 mg/kg, 0.6 mg/kg to 2.5 mg/kg, 0.6 mg/kg to 2 mg/kg, 0.6 mg/kg to 1.5 mg/kg, 0.6 mg/kg to 1 mg/kg, 1 mg/kg to 3 mg/kg, 1 mg/kg to 2.5 mg/kg, 1 mg/kg to 2 mg/kg, 1 mg/kg to 1.5 mg/kg body weight.

In further embodiments, the fusion protein of the present invention (for example **2149**) is administered by injection, preferably intravenous injection, according to the following dosage regimen: a dose of 0.2 µg/kg to 3 mg/kg body weight, for example 0.2 µg/kg, 2 µg/kg, 10 µg/kg, 30 µg/kg, 0.1 mg/kg, 0.3 mg/kg, 0.6 mg/kg, 1 mg/kg, 1.5 mg/kg, 2 mg/kg, 2.5 mg/kg, 3 mg/kg body weight, or a range composed of any two thereof administered once weekly (QW), once every two weeks (Q2W), or once every three weeks (Q3W); wherein the fusion protein is administered for one or more treatment cycles, and the treatment cycle is three weeks for once weekly or once every three weeks administration and four weeks for once every two weeks administration. For example, the fusion protein of the present invention (for example **2149**) may be administered by injection, preferably intravenous injection, according to the following dosage regimen: dose levels of 0.2 µg/kg to 3 mg/kg, 0.2 µg/kg to 2.5 mg/kg, 0.2 µg/kg to 2 mg/kg, 0.2 µg/kg to 1.5 mg/kg, 0.2 µg/kg to 1 mg/kg, 2 µg/kg to 3 mg/kg, 2 µg/kg to 2.5 mg/kg, 2 µg/kg to 2 mg/kg, 2 µg/kg to 1.5 mg/kg, 2 µg/kg to 1 mg/kg, 10 µg/kg to 3 mg/kg, 10 µg/kg to 2.5 mg/kg, 10 µg/kg to 2 mg/kg, 10 µg/kg to 1.5 mg/kg, 10 µg/kg to 1 mg/kg, 30 µg/kg to 3 mg/kg, 30 µg/kg to 2.5 mg/kg, 30 µg/kg to 2 mg/kg, 30 µg/kg to 1.5 mg/kg, 30 µg/kg to 1 mg/kg, 0.1 mg/kg to 3 mg/kg, 0.1 mg/kg to 2.5 mg/kg, 0.1 mg/kg to 2 mg/kg, 0.1 mg/kg to 1.5 mg/kg, 0.1 mg/kg to 1 mg/kg, 0.3 mg/kg to 3 mg/kg, 0.3 mg/kg to 2.5 mg/kg, 0.3 mg/kg to 2 mg/kg, 0.3 mg/kg to 1.5 mg/kg, 0.3 mg/kg to 1 mg/kg, 0.6 mg/kg to 3 mg/kg, 0.6 mg/kg to 2.5 mg/kg, 0.6 mg/kg to 2 mg/kg, 0.6 mg/kg to 1.5 mg/kg, 0.6 mg/kg to 1 mg/kg, 1 mg/kg to 3 mg/kg, 1 mg/kg to 2.5 mg/kg, 1 mg/kg to 2 mg/kg, 1 mg/kg to 1.5 mg/kg body weight, wherein the fusion protein is administered for one or more treatment cycles, and the treatment cycle is three weeks for once weekly or once every three weeks administration and four weeks for once every two weeks administration.

In still further embodiments, the fusion protein of the present invention (for example **2149**) is administered by injection, preferably intravenous injection, according to the following dosage regimen: a dose of 30 µg/kg to 3 mg/kg body weight, for example 30 µg/kg-3 mg/kg, 0.1-3 mg/kg, or 0.1-2 mg/kg, or 0.6-3 mg/kg, or 0.6-2 mg/kg, or 1-3 mg/kg, or 1-2 mg/kg body weight administered once weekly (QW), once every two weeks (Q2W), or once every three weeks (Q3W); wherein the fusion protein is administered for one or more treatment cycles, and the treatment cycle is three weeks for once weekly or once every three weeks administration and four weeks for once every two weeks administration.

Preferably, the fusion protein of the present invention (for example **2149**) is administered by injection, preferably intravenous injection, according to the following dosage regimen: 0.6-3 mg/kg body weight administered once weekly (QW), once every two weeks (Q2W), or once every three weeks (Q2W), wherein the fusion protein is administered for one or more treatment cycles, and the treatment cycle is three weeks for once weekly or once every three weeks administration and four weeks for once every two weeks administration. For example, the fusion protein of the present invention (for example **2149**) is administered by injection, preferably intravenous injection, according to the following dosage regimen: 0.6-3 mg/kg body weight administered once weekly (QW), wherein the fusion protein is administered for one or more treatment cycles, and the treatment cycle is three weeks. Alternatively, the fusion protein of the present invention (for example **2149**) is administered by injection, preferably intravenous injection, according to the following dosage regimen: 0.6-3 mg/kg body weight administered once every two weeks (Q2W), wherein the fusion protein is administered for one or more treatment cycles, and the treatment cycle is four weeks. Alternatively, the fusion protein of the present invention (for example **2149**) is administered by injection, preferably intravenous injection, according to the following dosage regimen: 0.6-3 mg/kg body weight administered once every three weeks (Q3W), wherein the fusion protein is administered for one or more treatment cycles, and the treatment cycle is three weeks.

Further preferably, the fusion protein of the present invention (for example **2149**) is administered by injection, preferably intravenous injection, according to the following dosage regimen: 1-3 mg/kg or 1-2 mg/kg body weight administered once weekly (QW), once every two weeks (Q2W), or once every three weeks (Q3W), wherein the fusion protein is administered for one or more treatment cycles, and the treatment cycle is three weeks for once weekly or once every three weeks administration, and four weeks for once every two weeks administration. For example, the fusion protein of the present invention (for example **2149**) is administered by injection, preferably intravenous injection, according to the following dosage regimen: 1-2 mg/kg body weight administered once weekly (QW), wherein the fusion protein is administered for one or more treatment cycles, and the treatment cycle is three weeks. Alternatively, the fusion protein of the present invention (for example **2149**) is administered by injection, preferably intravenous injection, according to the following dosage regimen: 1-2 mg/kg body weight administered once every two weeks (Q2W), wherein the fusion protein is administered for one or more treatment cycles, and the treatment cycle is four weeks. Alternatively, the fusion protein of the present invention (for example **2149**) is administered by injection, preferably intravenous injection, according to the following dosage regimen: 1-2 mg/kg body weight administered once every three weeks (Q3W), wherein the fusion protein is administered for one or more treatment cycles, and the treatment cycle is three weeks.

Still further preferably, the fusion protein of the present invention (for example **2149**) is administered by injection, preferably intravenous injection, according to the following dosage regimen: 0.3, 0.6, 1, or 2 mg/kg body weight administered once weekly (Q2W), wherein the fusion protein is administered for one or more treatment cycles, and the treatment cycle is four weeks.

Still further preferably, the fusion protein of the present invention (for example **2149**) is administered by injection, preferably intravenous injection, according to the following dosage regimen: 0.6, 1, 1.5, 2, or 3 mg/kg body weight administered once every three weeks (Q3W), for one or more treatment cycles, wherein the treatment cycle is three weeks.

Still further preferably, the fusion protein of the present invention (for example **2149**) is administered by injection, preferably intravenous injection, according to the following dosage regimen: 3 mg/kg body weight administered once every three weeks (Q3W), wherein the fusion protein is administered for one or more treatment cycles, and the treatment cycle is three weeks.

When a single administration dose of the fusion protein of the present invention (for example **2149**) is ≥0.6 mg/kg body weight (i.e., a normal dose or therapeutic dose), a low dose of the fusion protein is administered once by injection, preferably intravenous injection, 7±1 days before the first single administration dose as a priming dose, and the priming dose is 2-50% of the single administration dose (normal dose or therapeutic dose). Preferably, the priming dose is about 2%, 3.33%, 5%, 6.67%, 10%, 15%, 16.67%, 20%, 25%, 30%, 33.3%, 35%, 40%, 45%, or 50% of the single administration dose (normal dose or therapeutic dose), or a range composed of any two thereof. In particular, the priming dose is 0.2 µg/kg-1 mg/kg, 0.2 µg/kg-0.6 mg/kg, 0.2 µg/kg-0.3 mg/kg, 0.2 µg/kg-0.2 mg/kg, or 0.2 µg/kg-0.1 mg/kg body weight. The priming dose is usually administered 6, 7, or 8 days before the first normal dose or therapeutic dose, preferably 7 days before the first normal dose or therapeutic dose.

In some embodiments, when a single administration dose of the fusion protein of the present invention (for example **2149**) is ≥0.6 mg/kg body weight, a dose of ≤200 µg/kg body weight of the fusion protein is administered once by injection, preferably intravenous injection, 7±1 days before the first single administration dose as a priming dose. Preferably, the priming dose is administered 7 days before the first single administration dose.

In further embodiments, the fusion protein of the present invention (for example **2149**) is administered by injection, preferably intravenous injection, according to the following dosage regimen: a priming dose of 0.2-200 µg/kg body weight administered on day 1, and from day 8±1, a normal dose of 0.6-3 mg/kg body weight administered once weekly (QW), once every two weeks (Q2W), or once every three weeks (Q3W). Preferably, the normal dose is administered starting from day 8.

Preferably, the priming dose is 2-200 µg/kg body weight, preferably 30-200 µg/kg body weight, more preferably 30-150 µg/kg body weight, and most preferably 30-100 µg/kg body weight. For example, the priming dose is 100 µg/kg body weight.

Further preferably, the normal dose is a dose of 0.6-3 mg/kg body weight, for example 0.6 mg/kg, 1 mg/kg, 1.5 mg/kg, 2 mg/kg, 2.5 mg/kg, 3 mg/kg body weight, or a range composed of any two thereof administered once weekly (QW), once every two weeks (Q2W), or once every three weeks (Q3W). For example, the normal dose may be the following dose: 0.6-3 mg/kg, 0.6-2.5 mg/kg, 0.6-2 mg/kg, 0.6-1.5 mg/kg, 0.6-1 mg/kg, 1-3 mg/kg, 1-2.5 mg/kg, 1-2 mg/kg, or 1-1.5 mg/kg body weight. Preferably, the normal dose is a dose of 0.6-3 mg/kg, or 0.6-2.5 mg/kg, or 0.6-2 mg/kg, or 1-3 mg/kg, or 1-2.5 mg/kg, or 1-2 mg/kg body weight administered once weekly (QW), once every two weeks (Q2W), or once every three weeks (Q3W). The normal dose is administered for one or more treatment cycles, and the treatment cycle is three weeks for once weekly or once every three weeks administration and four weeks for once every two weeks administration.

In some embodiments, the fusion protein of the present invention (for example **2149**) is administered by injection, preferably intravenous injection, according to the following dosage regimen: a priming dose of 30-100 µg/kg body weight administered on day 1, and from day 8±1, a normal dose of 0.6-3 mg/kg, for example 0.6-2 mg/kg, for example 1-3 mg/kg, for example 1-2 mg/kg body weight administered once weekly (QW), once every two weeks (Q2W), or once every three weeks (Q3W), wherein the normal dose of the fusion protein is administered for one or more treatment cycles, and the treatment cycle is three weeks for once weekly or once every three weeks administration, and four weeks for once every two weeks administration. Preferably, the fusion protein of the present invention (for example **2149**) is administered by injection, preferably intravenous injection, according to the following dosage regimen: a priming dose of 30-100 µg/kg body weight administered on day 1, and from day 8±1, a normal dose of 0.6-3 mg/kg, preferably 0.6-2 mg/kg, more preferably 1-2 mg/kg body weight administered once weekly (QW), wherein the normal dose of the fusion protein is administered for one or more treatment cycles, and the treatment cycle is three weeks. Further preferably, the fusion protein of the present invention (for example **2149**) is administered by injection, preferably intravenous injection, according to the following dosage regimen: a priming dose of 30-100 µg/kg body weight administered on day 1, and from day 8±1, a normal dose of 0.6-3 mg/kg, preferably 0.6-2 mg/kg, more preferably 1-2 mg/kg body weight administered once every two weeks (Q2W), wherein the normal dose of the fusion protein is administered for one or more treatment cycles, and the treatment cycle is four weeks. Still further preferably, the fusion protein of the present invention (for example **2149**) is administered by injection, preferably intravenous injection, according to the following dosage regimen: a priming dose of 30-100 µg/kg body weight administered on day 1, and from day 8±1, a normal dose of 0.6-3 mg/kg, preferably 0.6-2 mg/kg, more preferably 1-2 mg/kg body weight administered once every three weeks (QW), wherein the normal dose of the fusion protein is administered for one or more treatment cycles, and the treatment cycle is three weeks. Preferably, the normal dose is administered starting from day 8.

In some embodiments, the fusion protein of the present invention (for example **2149**) is administered by injection, preferably intravenous injection, according to the following dosage regimen: a priming dose of 100 µg/kg body weight administered on day 1, and from day 8±1, a normal dose of 0.6, 1, or 2 mg/kg body weight administered once every two weeks (Q2W) , wherein the normal dose of the fusion protein is administered for one or more treatment cycles, and the treatment cycle is four weeks. Preferably, the normal dose is administered starting from day 8.

In some embodiments, the fusion protein of the present invention (for example **2149**) is administered by injection, preferably intravenous injection, according to the following dosage regimen: a priming dose of 100 µg/kg body weight administered on day 1, and from day 8±1, a normal dose of 0.6, 1, 1.5, 2, or 3 mg/kg body weight administered once every three weeks (Q3W), wherein the normal dose of the fusion protein is administered for one or more treatment cycles, and the treatment cycle is three weeks. Preferably, the normal dose is administered starting from day 8.

In some embodiments, the fusion protein of the present invention (for example **2149**) is administered by injection, preferably intravenous injection, according to the following dosage regimen: a priming dose of 100 µg/kg body weight administered on day 1, and from day 8±1, a normal dose of 1.5 mg/kg body weight administered once every three weeks (Q3W), wherein the normal dose of the fusion protein is administered for one or more treatment cycles, and the treatment cycle is three weeks. Preferably, the normal dose is administered starting from day 8.

In some embodiments, the fusion protein of the present invention (for example **2149**) is administered by injection, preferably intravenous injection, according to the following dosage regimen: a priming dose of 100 µg/kg body weight administered on day 1, and from day 8±1, a normal dose of 3 mg/kg body weight administered once every three weeks (Q3W), wherein the normal dose of the fusion protein is administered for one or more treatment cycles, and the treatment cycle is three weeks. Preferably, the normal dose is administered starting from day 8.

It can be understood that the normal dose is usually administered on day 8 after administration of the priming dose, but may be administered earlier or later for various reasons, for example patient inconvenience and the like.

It can also be understood that when more than one treatment cycle is present, the treatment cycles may each be carried out continuously or at intervals (for example, pausing the drug for a period of time for various reasons).

The fusion protein of the present invention (for example **2149**) may be formulated into a form suitable for administration, particularly a form suitable for injection administration (injection formulation), for example a solution, emulsion, suspension, concentrate, lyophilized powder (reconstituted before use with a solvent for injection for example water for injection, physiological saline, glucose injection, and the like), and the like, for example formulated by methods known in the art. Preferably, the fusion protein of the present invention (for example **2149**) is formulated into a form for administration according to the dosage regimen described herein. In some embodiments, the fusion protein of the present invention is in the form of a sterile injection solution, preferably containing 0.5-150 mg/ml of the fusion protein, preferably 0.5-100 mg/mL, for example 0.5-50 mg/ml or 1-20 mg/ml, for example about 0.5, 1, 2, 3, 4, 5, 6, 7, 8, 9, 10, 11, 12, 13, 14, 15, 16, 17, 18, 19, or 20 mg/ml. It can be understood that, if necessary, the injection solution may be diluted with a suitable solvent for injection to a desired concentration so as to be suitable for the dosage regimen of the present invention. In some embodiments, the fusion protein of the present invention is in the form of a sterile lyophilized powder, which may be reconstituted with a suitable solvent for injection before use to a desired concentration so as to be suitable for the dosage regimen of the present invention.

In some embodiments, the fusion protein of the present invention (for example **2149**) may be used for treating a solid tumor or hematologic tumor, wherein the solid tumor or hematologic tumor is selected from intestinal cancer (including rectal cancer, colon cancer, and colorectal cancer), melanoma, lung cancer (for example non-small cell lung cancer, including squamous and non-squamous non-small cell lung cancer), breast cancer (for example triple-negative breast cancer), pancreatic cancer, cholangiocarcinoma, ovarian cancer, cervical cancer, uterine cancer, head and neck cancer (for example head and neck squamous cell carcinoma), liver cancer (for example hepatocellular carcinoma), kidney cancer (for example renal cell carcinoma), gastric cancer, lymphoma, leukemia, or multiple myeloma.

Preferably, the fusion protein of the present invention (for example **2149**) may be used for treating a solid tumor or hematologic tumor, wherein the solid tumor or hematologic tumor is selected from colorectal cancer, melanoma, non-small cell lung cancer (including squamous and non-squamous non-small cell lung cancer), breast cancer, pancreatic cancer, cholangiocarcinoma, ovarian cancer, cervical cancer, head and neck cancer (for example head and neck squamous cell carcinoma), and lymphoma. more preferably, the solid tumor or hematologic tumor is selected from colorectal cancer, melanoma, and non-small cell lung cancer (comprising squamous and non-squamous non-small cell lung cancer).

In some embodiments, the melanoma is selected from cutaneous melanoma, acral melanoma, mucosal melanoma, and melanoma of unknown primary.

In some embodiments, the non-small cell lung cancer is selected from squamous and non-squamous non-small cell lung cancer, for example lung squamous cell carcinoma or lung adenocarcinoma.

In other embodiments, the hematologic tumor is selected from lymphoma, leukemia, and multiple myeloma, particularly lymphoma.

In some embodiments, the solid tumor or hematologic tumor is advanced, recurrent, metastatic, or unresectable, failed or intolerant to standard therapy.

In some embodiments, the fusion protein of the present invention (for example **2149**) may be used for treating melanoma, for example cutaneous melanoma, acral melanoma, mucosal melanoma, or melanoma of unknown primary, particularly those that are advanced, recurrent, metastatic, or unresectable, failed or intolerant to standard therapy; for example unresectable locally advanced or metastatic melanoma that has progressed or recurred after previous treatment with an immune checkpoint inhibitor, or unresectable locally advanced or metastatic melanoma that has not been treated with a systemic immune checkpoint inhibitor. Preferably, the fusion protein of the present invention (for example **2149**) is used for treating melanoma by injection, preferably intravenous injection, according to the following regimen: a priming dose of 30-100 µg/kg, preferably 100 µg/kg administered on day 1, and from day 8±1, a normal dose of 1-3 mg/kg body weight administered once weekly (QW), once every two weeks (Q2W), or once every three weeks (Q3W), wherein the normal dose is administered for one or more treatment cycles, and the treatment cycle is three weeks for once weekly or once every three weeks administration and four weeks for once every two weeks administration. Further preferably, the fusion protein of the present invention (for example **2149**) is used for treating melanoma by injection, preferably intravenous injection, according to the following regimen: a priming dose of 30-100 µg/kg, preferably 100 µg/kg, administered on day 1, and from day 8±1, a normal dose of 1-3 mg/kg, preferably 1 mg/kg body weight administered once every two weeks (Q2W), wherein the normal dose is administered for one or more treatment cycles, and the treatment cycle is four weeks. Further preferably, the fusion protein of the present invention (for example **2149**) is used for treating melanoma by injection, preferably intravenous injection, according to the following regimen: a priming dose of 30-100 µg/kg, preferably 100 µg/kg, administered on day 1, and from day 8±1, a normal dose of 1-3 mg/kg, preferably 3 mg/kg body weight administered once every three weeks (Q3W), wherein the normal dose is administered for one or more treatment cycles, and the treatment cycle is three weeks.

In some embodiments, the fusion protein of the present invention (for example **2149**) may be used for treating colorectal cancer, preferably colorectal cancer that is failed or intolerant to standard therapy, advanced, recurrent, metastatic, or unresectable, particularly colorectal cancer with proficient mismatch repair (pMMR) and/or microsatellite stable (MSS) status. Preferably, the fusion protein of the present invention (for example **2149**) is used for treating colorectal cancer by injection, preferably intravenous injection, according to the following regimen: a priming dose of 30-100 µg/kg, preferably 100 µg/kg, administered on day 1, and from day 8±1, a normal dose of 0.1-3 mg/kg body weight administered once weekly (QW), once every two weeks (Q2W), or once every three weeks (Q3W), wherein the normal dose is administered for one or more treatment cycles, and the treatment cycle is three weeks for once weekly or once every three weeks administration and four weeks for once every two weeks administration. Further preferably, the fusion protein of the present invention (for example **2149**) is used for treating colorectal cancer by injection, preferably intravenous injection, according to the following regimen: a priming dose of 30-100 µg/kg, preferably 100 µg/kg, administered on day 1, and from day 8±1, a normal dose of 1-3 mg/kg, preferably 1 mg/kg body weight administered once every two weeks (Q2W), wherein the normal dose is administered for one or more treatment cycles, and the treatment cycle is four weeks. Further preferably, the fusion protein of the present invention (for example **2149**) is used for treating colorectal cancer by injection, preferably intravenous injection, according to the following regimen: a priming dose of 30-100 µg/kg, preferably 100 µg/kg, administered on day 1, and from day 8±1, a normal dose of 1-3 mg/kg, preferably 3 mg/kg body weight administered once every three weeks (Q3W), wherein the normal dose is administered for one or more treatment cycles, and the treatment cycle is three weeks.

In some embodiments, the fusion protein of the present invention (for example **2149**) may be used for treating non-small cell lung cancer, including squamous and non-squamous non-small cell lung cancer, particularly advanced, recurrent, metastatic, or unresectable non-small cell lung cancer without known driver gene mutations, failed or intolerant to standard therapy. Preferably, the fusion protein of the present invention (for example **2149**) is used for treating non-small cell lung cancer by injection, preferably intravenous injection, according to the following regimen: a priming dose of 30-100 µg/kg, preferably 100 µg/kg, administered on day 1, and from day 8±1, a normal dose of 0.6-3 mg/kg body weight administered once weekly (QW), once every two weeks (Q2W), or once every three weeks (Q3W), wherein the normal dose is administered for one or more treatment cycles, and the treatment cycle is three weeks for once weekly or once every three weeks administration and four weeks for once every two weeks administration. Further preferably, the fusion protein of the present invention (for example **2149**) is used for treating non-small cell lung cancer by injection, preferably intravenous injection, according to the following regimen: a priming dose of 30-100 µg/kg, preferably 100 µg/kg, administered on day 1, and from day 8±1, a normal dose of 1-3 mg/kg, preferably 1 mg/kg body weight administered once every two weeks (Q2W), wherein the normal dose is administered for one or more treatment cycles, and the treatment cycle is four weeks. Further preferably, the fusion protein of the present invention (for example **2149**) is used for treating non-small cell lung cancer by injection, preferably intravenous injection, according to the following regimen: a priming dose of 30-100 µg/kg, preferably 100 µg/kg, administered on day 1, and from day 8±1, a normal dose of 1-3 mg/kg, preferably 3 mg/kg body weight administered once every three weeks (Q3W), wherein the normal dose is administered for one or more treatment cycles, and the treatment cycle is three weeks.

In some embodiments, the fusion protein of the present invention (for example **2149**) may be used for treating lymphoma, particularly advanced, recurrent, or metastatic lymphoma, lymphoma failed or intolerant to standard therapy, or unresectable lymphoma. Preferably, the fusion protein of the present invention (for example **2149**) is used for treating lymphoma by injection, preferably intravenous injection, according to the following regimen: a priming dose of 30-100 µg/kg, preferably 100 µg/kg, administered on day 1, and from day 8±1, a normal dose of 1-3 mg/kg body weight administered once weekly (QW), once every two weeks (Q2W), or once every three weeks (Q3W), wherein the normal dose is administered for one or more treatment cycles, and the treatment cycle is three weeks for once weekly or once every three weeks administration and four weeks for once every two weeks administration. Further preferably, the fusion protein of the present invention (for example **2149**) is used for treating melanoma by injection, preferably intravenous injection, according to the following regimen: a priming dose of 30-100 µg/kg, preferably 100 µg/kg, administered on day 1, and from day 8±1, a normal dose of 1-3 mg/kg, preferably 1 mg/kg body weight administered once every two weeks (Q2W), wherein the normal dose is administered for one or more treatment cycles, and the treatment cycle is four weeks. Further preferably, the fusion protein of the present invention (for example **2149**) is used for treating melanoma by injection, preferably intravenous injection, according to the following regimen: a priming dose of 30-100 µg/kg, preferably 100 µg/kg, administered on day 1, and from day 8±1, a normal dose of 1-3 mg/kg, preferably 3 mg/kg body weight administered once every three weeks (Q3W), wherein the normal dose is administered for one or more treatment cycles, and the treatment cycle is three weeks.

In a second aspect, provided is a method for treating a solid tumor or hematologic tumor in an individual, the method comprising administering to an individual in need thereof the PD-1/IL-2R bispecific antibody fusion protein of the present invention, wherein the PD-1/IL-2R bispecific antibody fusion protein is as defined herein. In some embodiments, the PD-1/IL-2R bispecific antibody fusion protein is administered according to a dosage regimen as described herein. In some embodiments, the solid tumor or hematologic tumor is as defined herein.

In a third aspect, provided is the PD-1/IL-2R bispecific antibody fusion protein of the present invention for use in treating a solid tumor or hematologic tumor, wherein the PD-1/IL-2R bispecific antibody fusion protein is as defined herein. In some embodiments, the PD-1/IL-2R bispecific antibody fusion protein is administered according to a dosage regimen as described herein. In some embodiments, the solid tumor or hematologic tumor is as defined herein.

In a fourth aspect, provided is use of the PD-1/IL-2R bispecific antibody fusion protein of the present invention in the manufacture of a medicament for treating a solid tumor or hematologic tumor, wherein the PD-1/IL-2R bispecific antibody fusion protein is as defined herein. In some embodiments, the medicament is in a form suitable for injection administration (injection formulation), for example a solution, emulsion, suspension, concentrate, lyophilized powder (reconstituted before use with a solvent for injection for example water for injection, physiological saline, glucose injection, and the like), and the like. In some embodiments, the PD-1/IL-2R bispecific antibody fusion protein is administered according to a dosage regimen as described herein. In some embodiments, the medicament is in a formulation form capable of administering or delivering the PD-1/IL-2R bispecific antibody fusion protein by injection, preferably intravenous injection, according to the dosage regimen of the present invention. In some embodiments, the medicament is administered according to a dosage regimen as described herein. In some embodiments, the solid tumor or hematologic tumor is as defined herein.

In a fifth aspect, provided is a pharmaceutical composition, which comprises the PD-1/IL-2R bispecific antibody fusion protein of the present invention and one or more pharmaceutically acceptable excipients, wherein the PD-1/IL-2R bispecific antibody fusion protein is as defined herein. In some embodiments, the pharmaceutical composition is in a form suitable for injection administration (injection formulation), for example a solution, emulsion, suspension, concentrate, lyophilized powder (reconstituted before use with a solvent for injection for example water for injection, physiological saline, glucose injection, and the like), and the like. In some embodiments, the PD-1/IL-2R bispecific antibody fusion protein is administered according to a dosage regimen as described herein. In some embodiments, the pharmaceutical composition is in a formulation form capable of administering or delivering the PD-1/IL-2R bispecific antibody fusion protein by injection, preferably intravenous injection, according to the dosage regimen of the present application. In some embodiments, the pharmaceutical composition is administered according to a dosage regimen as described herein. In some embodiments, the solid tumor or hematologic tumor is as defined herein.

In some embodiments, provided is a single-dose administration unit, which comprises a single therapeutically effective amount of the fusion protein of the present invention (for example **2149**). In some embodiments, the single-dose administration unit is for injection administration, preferably for administration by intravenous injection. In some embodiments, the single-dose administration unit is in a form suitable for injection administration (injection formulation), for example a solution, emulsion, suspension, concentrate, lyophilized powder (reconstituted before use with a solvent for injection for example water for injection, physiological saline, glucose injection, and the like), and the like. Preferably, the single-dose administration unit is in the form of an injection solution or lyophilized powder injection.

In some embodiments, the single-dose administration unit of the present invention comprises 10 µg to 300 mg of the fusion protein, for example 10 µg, 200 µg, 800 µg, 1 mg, 1.5 mg, 3 mg, 10 mg, 12 mg, 25 mg, 50 mg, 100 mg, 150 mg, 200 mg, 225 mg, 250 mg, 275 mg, 300 mg, or a range composed of any two thereof. For example, the dose administration unit comprises 1-300 mg, 1-250 mg, 1-200 mg, 1-150 mg, 1-100 mg, 1-50 mg, 1-25 mg, 1-12 mg, 3-300 mg, 3-250 mg, 3-200 mg, 3-150 mg, 3-100 mg, 3-50 mg, 3-25 mg, 3-12 mg, 10-300 mg, 10-250 mg, 10-200 mg, 10-150 mg, 10-100 mg, 10-50 mg, 10-25 mg, 10-12 mg, 50-300 mg, 50-250 mg, 50-200 mg, 50-150 mg, 50-100 mg, 100-300 mg, 100-250 mg, 100-200 mg, or 100-150 mg of the fusion protein.

In some embodiments, the single-dose administration unit of the present invention provides a patient with 0.2 µg/kg to 3 mg/kg body weight of the fusion protein of the present invention (for example **2149**). Preferably, the single-dose administration unit of the present invention is capable of providing a patient with the fusion protein of the present invention (for example **2149**) at any dose level described herein, for example any dose level as defined in any one of embodiments 13-22. For example, the single-dose administration unit of the present invention is capable of providing a patient with the fusion protein of the present invention (for example **2149**) at a dose level of 0.2 µg/kg, 2 µg/kg, 10 µg/kg, 30 µg/kg, 0.1 mg/kg, 0.3 mg/kg, 0.6 mg/kg, 1 mg/kg, 1.5 mg/kg, 2 mg/kg, 2.5 mg/kg, 3 mg/kg body weight, or a range composed of any two thereof.

In a sixth aspect, provided is a kit, which comprises the PD-1/IL-2R bispecific antibody fusion protein of the present invention and instructions indicating use of the fusion protein for treating a solid tumor or hematologic tumor, wherein the PD-1/IL-2R bispecific antibody fusion protein is as defined herein. In some embodiments, the kit is configured in a kit form for administering or delivering the PD-1/IL-2R bispecific antibody fusion protein by injection, preferably intravenous injection, according to the dosage regimen of the present application. In some embodiments, the solid tumor or hematologic tumor is as defined herein.

In another aspect, provided is a kit, which comprises one or more single-dose administration units of the present invention and instructions indicating administration of the one or more single-dose administration units according to a dosage regimen as defined herein, for example the dosage regimen as defined in any one of embodiments 13-22. Preferably, the kit is for treating a solid tumor or hematologic tumor.

It can be understood that the fusion protein of the present invention may also be used in combination with one or more other active agents or therapies for the uses described herein, and the other active agents or therapies may have the same or different pharmacological efficacy as the fusion protein of the present invention. The fusion protein of the present invention may be administered simultaneously with, before, or after the other active agents or therapies, and the fusion protein of the present invention and the other active agents may be administered in the same or different dosage forms and by the same or different routes of administration. The dosage regimen of the other active agents or therapies may be determined by the attending physician. The various aspects, embodiments, features, and the like described herein for the PD-1/IL-2R bispecific antibody fusion protein of the present invention also apply to the first active ingredient in the pharmaceutical combination of the present invention.

In a seventh aspect, the present invention provides a pharmaceutical combination, the pharmaceutical combination comprises:
(i) a first active ingredient, wherein the first active ingredient is a PD-1/IL-2R bispecific antibody fusion protein, and the PD-1/IL-2R bispecific antibody fusion protein comprises: (a) a first monomer comprising an IL-2 mutein fused with an Fc fragment, optionally via or not via a linker; and (b) a second monomer comprising a heavy chain and a light chain of an antibody that specifically binds PD-1; and
(ii) a second active ingredient, wherein the second active ingredient is bevacizumab.

In some embodiments, the first active ingredient is the PD-1/IL-2R bispecific antibody fusion protein as defined herein, for example as defined in the preceding embodiments 1-12 or 31-42.

The administration dose of the PD-1/IL-2R bispecific antibody fusion protein (for example immunoconjugate 2149) may vary according to the route of administration, the disease being treated, the age, body weight, and general health status of the patient, concomitant medication, and the like. Those skilled in the relevant art can readily determine the therapeutically effective amount thereof in practice according to the specific circumstances of the individual in need of treatment. For example, when administered by intravenous infusion, the PD-1/IL-2R bispecific antibody fusion protein (for example immunoconjugate 2149) may be administered at a dose of 0.2 µg/kg body weight to 1 mg/kg body weight, for example at a dose of 0.2, 0.5, 1, 2, 4, 6, 8, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, or 950 µg/kg body weight or 1 mg/kg body weight, administered once weekly (QW), with every three weeks constituting one treatment cycle; or at a dose of 1 mg/kg body weight to 3 mg/kg body weight, for example 1 mg/kg body weight, 1.5 mg/kg body weight, 2 mg/kg body weight, 2.5 mg/kg body weight, or 3 mg/kg body weight, administered once weekly, with every three weeks constituting one treatment cycle; or at a dose of 1 mg/kg body weight to 3 mg/kg body weight, for example 1 mg/kg body weight, 1.5 mg/kg body weight, 2 mg/kg body weight, 2.5 mg/kg body weight, or 3 mg/kg body weight, administered once every two weeks (Q2W), with every four weeks constituting one treatment cycle; or at a dose of 1 mg/kg body weight to 3 mg/kg body weight, for example 1 mg/kg body weight, 1.5 mg/kg body weight, 2 mg/kg body weight, 2.5 mg/kg body weight, or 3 mg/kg body weight, administered once every three weeks (Q3W), with every three weeks constituting one treatment cycle. In one embodiment, the dosage regimen of the PD-1/IL-2R bispecific antibody fusion protein (for example immunoconjugate 2149) is intravenous infusion at a priming dose of 0.2 µg/kg body weight to 1 mg/kg body weight (for example 20, 40, 60, 80, 100, 150, 200, 250, 300, 350, 400, 300, 350, 400, 450, 500, 550, 600, 650, 700, 750, 800, 850, 900, or 950 µg/kg body weight or 1 mg/kg body weight), preferably 100 µg/kg body weight to 1 mg/kg body weight, administered on day 1, and a maintenance dose of 2 mg/kg body weight administered on day 8±1, and the maintenance dose is administered once every two weeks; or intravenous infusion at a priming dose of 0.2 µg/kg body weight to 1 mg/kg body weight (for example 20, 40, 60, 80, 100, 150, 200, 250, 300, 350, 400, 300, 350, 400, 450, 500, 550, 600, 650, 700, 750, 800, 850, 900, or 950 µg/kg body weight or 1 mg/kg body weight), preferably 100 µg/kg body weight to 1 mg/kg body weight, administered on day 1, and a maintenance dose of 2 mg/kg body weight or 3 mg/kg administered on day 8±1, and the maintenance dose is administered once every three weeks.

The second active ingredient bevacizumab is for intravenous infusion administration at a dose of 5 mg/kg body weight to 15 mg/kg body weight (for example 5, 5.5, 6, 6.5, 7, 7.5, 8, 8.5, 9, 9.5, 10, 10.5, 11, 11.5, 12, 12.5, 13, 13.5, 14, 14.5, or 15 mg/kg body weight) administered once every two weeks, with every four weeks constituting one treatment cycle, or once every three weeks, with every three weeks constituting one treatment cycle; preferably, the second active ingredient bevacizumab is for intravenous infusion administration at a dose of 5 mg/kg body weight, 7.5 mg/kg body weight, 10 mg/kg body weight, or 15 mg/kg body weight administered once every two weeks, with every four weeks constituting one treatment cycle, or once every three weeks, with every three weeks constituting one treatment cycle.

Bevacizumab is approved by the National Medical Products Administration (NMPA) for treating recurrent glioblastoma, and is approved by the NMPA in combination with fluorouracil for treating metastatic colorectal cancer, in combination with platinum-based chemotherapeutic agents for treating unresectable advanced, metastatic, or recurrent non-squamous non-small cell lung cancer, and the like. In addition, bevacizumab can also enhance dendritic cell maturation and promote lymphocyte migration into tumors, suggesting that bevacizumab may enhance IL-2-mediated antitumor immune responses.

In one embodiment, the bevacizumab is bevacizumab sold under the trade name Dayoutong or Avastin.

The pharmaceutical combination of the seventh aspect is used for treating a solid tumor, wherein the solid tumor is selected from colorectal cancer, melanoma, non-squamous non-small cell lung cancer, hepatocellular carcinoma, lymphoma, and other solid tumors. Preferably, the solid tumor is advanced.

In one embodiment, the solid tumor is colorectal cancer, particularly advanced colorectal cancer, more particularly advanced colorectal cancer with standard treatment failure;
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg body weight to 15 mg/kg body weight administered once every two weeks, with every four weeks constituting one treatment cycle, or once every three weeks, with every three weeks constituting one treatment cycle; preferably, the second active ingredient is for intravenous infusion administration at a dose of 5 mg/kg body weight to 10 mg/kg body weight (for example 5 mg/kg body weight or 7.5 mg/kg body weight) administered once every two weeks, with every four weeks constituting one treatment cycle, or once every three weeks, with every three weeks constituting one treatment cycle.

In one embodiment, the solid tumor is melanoma, preferably advanced melanoma, more preferably advanced melanoma that has not received immunotherapy or advanced melanoma with prior immunotherapy failure;
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg body weight, 7.5 mg/kg, 10 mg/kg, 12.5 mg/kg body weight, or 15 mg/kg body weight administered once every two weeks with each four weeks constituting a treatment cycle, or once every three weeks with each three weeks constituting a treatment cycle.

In one embodiment, the solid tumor is non-squamous non-small cell lung cancer (preferably advanced non-squamous non-small cell lung cancer, more preferably advanced non-squamous non-small cell lung cancer failed or intolerant to standard therapy), hepatocellular carcinoma (preferably advanced hepatocellular carcinoma, more preferably advanced hepatocellular carcinoma that has not received systemic treatment), or renal cell carcinoma (preferably advanced renal cell carcinoma, more preferably advanced renal cell carcinoma failed or intolerant to standard therapy);
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 10 mg/kg body weight to 15 mg/kg body weight (for example 10 mg/kg body weight, 12.5 mg/kg body weight, or 15 mg/kg body weight) administered once every two weeks with each four weeks constituting a treatment cycle, or once every three weeks with each three weeks constituting a treatment cycle.

In one embodiment, the solid tumor is lymphoma and other solid tumors, preferably lymphoma failed or intolerant to standard therapy or other advanced solid tumors;
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg body weight, 7.5 mg/kg, 10 mg/kg, 12.5 mg/kg body weight, or 15 mg/kg body weight administered once every two weeks with each four weeks constituting a treatment cycle, or once every three weeks with each three weeks constituting a treatment cycle.

The seventh aspect further includes a method for treating a solid tumor with the pharmaceutical combination described above, the method comprising simultaneously or separately administering the first active ingredient and the second active ingredient in the same or different pharmaceutical compositions at the administration dose and dosage regimen described above.

The seventh aspect further includes use of the pharmaceutical combination described above for treating a solid tumor.

The seventh aspect further includes use of the pharmaceutical combination described above in the manufacture of a pharmaceutical product for treating a solid tumor.

In an eighth aspect, the present invention provides a pharmaceutical combination, the pharmaceutical combination comprising a first active ingredient and a second active ingredient, wherein the first active ingredient is the PD-1/IL-2R bispecific antibody fusion protein described above, and the second active ingredient is dacarbazine. Specific embodiments, preferred embodiments, and administration routes and administration doses of the PD-1/IL-2R bispecific antibody fusion protein are as described herein, for example as defined in the preceding embodiments 1-64.

The chemical structure of dacarbazine is

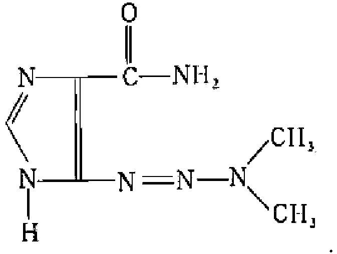

Dacarbazine, as an injection, is approved for treating malignant melanoma, and can be used for intravenous infusion, intravenous bolus administration, intraarterial infusion, or intraarterial bolus administration.

When administered by intravenous or intraarterial infusion, the dacarbazine is administered at a dose of 2.5-6 mg/kg body weight or 200-400 mg/m² body surface area once daily for 5-10 consecutive days, followed by a drug-free interval, with each 3-6 weeks constituting a treatment cycle; or a dose of 650-1450 mg/m² body surface area once every 4-6 weeks, correspondingly with each 4-6 weeks constituting a treatment cycle; when administered by intravenous or intraarterial bolus, the dacarbazine is administered at a dose of 200-400 mg/m² once daily for 5 consecutive days, followed by a drug-free interval, with each 3-4 weeks constituting a treatment cycle.

The pharmaceutical combination of the eighth aspect is used for treating melanoma, particularly advanced melanoma, more particularly advanced melanoma that has not received systemic treatment.

In one embodiment, the first active ingredient (for example molecule 2149) is for intravenous infusion administration at a dose of 0.2 µg/kg body weight to 1 mg/kg body weight administered once weekly, with every three weeks constituting one treatment cycle; or at a dose of 1 mg/kg body weight to 3 mg/kg body weight administered once weekly, with every three weeks constituting one treatment cycle; or at a dose of 1 mg/kg body weight to 3 mg/kg body weight once every two weeks, with every four weeks constituting one treatment cycle; or at a dose of 1 mg/kg body weight to 3 mg/kg body weight once every three weeks, with every three weeks constituting one treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, the maintenance dose is administered once every two weeks; or a priming dose of 0.2 µg/kg body weight to 1 mg/kg body weight (for example a priming dose of 100 µg/kg body weight or 1 mg/kg body weight) administered on day 1, and a maintenance dose of 2 mg/kg body weight or 3 mg/kg administered on day 8±1, the maintenance dose is administered once every three weeks;
the second active ingredient dacarbazine is administered by intravenous or intraarterial infusion, and when administered by intravenous or intraarterial infusion, the dacarbazine is administered at a dose of 2.5-6 mg/kg body weight or 200-400 mg/m² body surface area once daily for 5-10 consecutive days, followed by a drug-free interval, with each 3-6 weeks constituting a treatment cycle; or a dose of 650-1450 mg/m² body surface area once every 4-6 weeks, correspondingly with each 4-6 weeks constituting a treatment cycle; when administered by intravenous or intraarterial bolus, the dacarbazine is administered at a dose of 200-400 mg/m² once daily for 5 consecutive days, followed by a drug-free interval, with each 3-4 weeks constituting a treatment cycle.

In one preferred embodiment, the second active ingredient dacarbazine is for intravenous infusion administration at a dose of 850 mg/m² body surface area administered once every four weeks with each four weeks constituting a treatment cycle; or for intravenous infusion administration at a dose of 650-1050 mg/m² body surface area (for example, 850 mg/m² body surface area) administered once every three weeks with each three weeks constituting a treatment cycle; or for intravenous bolus administration at a dose of 200-300 mg/m² body surface area (for example, 250 mg/m² body surface area) administered once daily for 5 consecutive days, followed by a drug-free interval, with each four weeks constituting a treatment cycle; or for intravenous bolus administration at a dose of 200-300 mg/m² body surface area (for example, 250 mg/m² body surface area) administered once daily for 5 consecutive days, followed by a drug-free interval, with each three weeks constituting a treatment cycle.

The eighth aspect further includes a method of treating melanoma, particularly advanced melanoma, and more particularly advanced melanoma untreated with systemic therapy, using the pharmaceutical combination described above, wherein the method comprises administering the first active ingredient and the second active ingredient in the same or different pharmaceutical compositions simultaneously or separately according to the above-described dosage and dosage regimen.

The eighth aspect further includes the use of the pharmaceutical combination described above for treating melanoma, particularly advanced melanoma, and more particularly advanced melanoma untreated with systemic therapy.

The eighth aspect further includes the use of the pharmaceutical combination described above for the manufacture of a medicament for treating melanoma, particularly advanced melanoma, and more particularly advanced melanoma untreated with systemic therapy.

In the ninth aspect, the present invention provides a pharmaceutical combination comprising a first active ingredient and a second active ingredient, wherein the first active ingredient is the PD-1/IL-2R bispecific antibody fusion protein described above, and the second active ingredient is a compound or a pharmaceutically acceptable salt thereof disclosed in WO2008/112407 and its family patents or patent applications (for example Chinese patents ZL200880007358.X, ZL201310454117.2, ZL 201610147579.3). The entire content of the patent or patent applications (including definitions of terms) is incorporated herein by reference. Specific embodiments, preferred embodiments, and administration routes and administration doses of the PD-1/IL-2R bispecific antibody fusion protein are as described herein, for example as defined in the preceding embodiments 1-64.

In one embodiment, the second active ingredient is a compound of formula (II) or a pharmaceutically acceptable salt thereof, 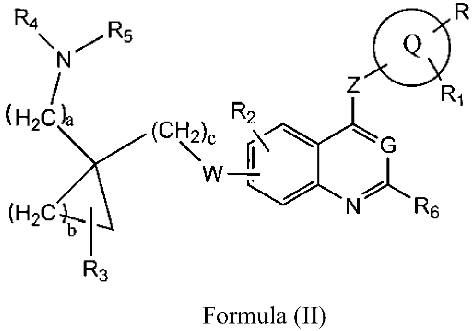 wherein
W is O; Z is O; G is CR; a is 0; c is 1; b is 1; R is H or C₁₋₆ alkyl; 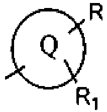 is 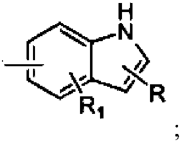
R₁, R₂, R₃ are each independently selected from H, halogen, and C₁₋₆ alkoxy;
R₄ and R₅ are each independently selected from H or C₁₋₆ alkyl;
R₆ is H.

In one preferred embodiment, the second active ingredient is anlotinib 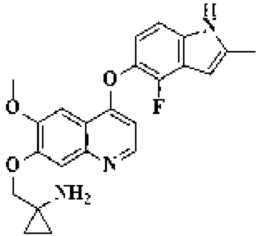 or a pharmaceutically acceptable salt thereof, particularly anlotinib dihydrochloride

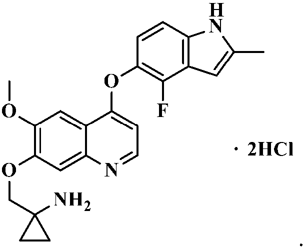

Anlotinib is a novel multi-targeted tyrosine kinase inhibitor that effectively inhibits kinases, for example VEGFR, PDGFR, FGFR, and c-Kit, exhibiting antitumor angiogenesis and tumor growth inhibition effects. Anlotinib dihydrochloride as an oral formulation has been approved by the NMPA for the treatment of patients with locally advanced or metastatic non-small cell lung cancer who have progressed or recurred after receiving at least 2 systemic chemotherapy regimens.

The compound of formula (II) or a pharmaceutically acceptable salt thereof (for example anlotinib dihydrochloride) can be formulated into suitable dosage forms for example tablets, capsules, powders, solutions, emulsions, suspensions, or lyophilized powders reconstituted shortly before use with injectable solvents (for example glucose injection, sodium chloride injection, injectable water, etc.). The compound of formula (II) or its pharmaceutically acceptable salt can be administered by any suitable route, preferably orally.

The compound of formula (II) or its pharmaceutically acceptable salt (for example, anlotinib dihydrochloride) may vary depending on the route of administration, the disease being treated, the patient's age, weight, overall health condition, and concomitant medications. Those skilled in the relevant art can readily determine the therapeutically effective amount thereof in practice according to the specific circumstances of the individual in need of treatment. For example, the compound of formula (II) or its pharmaceutically acceptable salt (for example anlotinib dihydrochloride) is administered orally at a dose of 8 mg to 12 mg, for example 8 mg, 9 mg, 10 mg, 11 mg, or 12 mg, once daily for 2 consecutive weeks followed by a drug-free interval of 1 week, with each three weeks constituting a treatment cycle.

In one embodiment, the first active ingredient is administered by injection, for example intravenous infusion; the second active ingredient is administered orally.

The pharmaceutical combination of the ninth aspect is used for treating non-small cell lung cancer, especially advanced non-small cell lung cancer, more particularly advanced non-small cell lung cancer failed or intolerant to standard therapy.

In one embodiment, the first active ingredient (for example molecule 2149) is for intravenous infusion administration at a dose of 0.2 µg/kg body weight to 1 mg/kg body weight administered once weekly, with every three weeks constituting one treatment cycle; or at a dose of 1 mg/kg body weight to 3 mg/kg body weight administered once weekly, with every three weeks constituting one treatment cycle; or at a dose of 1 mg/kg body weight to 3 mg/kg body weight once every two weeks, with every four weeks constituting one treatment cycle; or at a dose of 1 mg/kg body weight to 3 mg/kg body weight once every three weeks, with every three weeks constituting one treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, the maintenance dose is administered once every two weeks; or a priming dose of 0.2 µg/kg body weight to 1 mg/kg body weight (for example a priming dose of 100 µg/kg body weight or 1 mg/kg body weight) administered on day 1, and a maintenance dose of 2 mg/kg body weight or 3 mg/kg administered on day 8±1, the maintenance dose is administered once every three weeks;
the second active ingredient is for oral administration at a dose of 8 mg to 12 mg administered once daily for 2 consecutive weeks, followed by a drug-free interval of 1 week, with each three weeks constituting a treatment cycle.

In a preferred embodiment, the second active ingredient (for example anlotinib dihydrochloride) is administered orally at a dose of 12 mg (when the second active ingredient is a pharmaceutically acceptable salt of anlotinib, for example the dihydrochloride, calculated as anlotinib), administered once daily for 2 consecutive weeks followed by a drug-free interval of 1 week, with each three weeks constituting a treatment cycle.

The ninth aspect also includes a method of treating non-small cell lung cancer, especially advanced non-small cell lung cancer, more particularly advanced non-small cell lung cancer failed or intolerant to standard therapy, using the pharmaceutical combination described above, wherein the method comprises administering the first active ingredient and the second active ingredient simultaneously or separately in the same or different pharmaceutical compositions according to the above-mentioned dosage and dosage regimen.

The ninth aspect further includes the use of the pharmaceutical combination described above for the treatment of non-small cell lung cancer, especially advanced non-small cell lung cancer, more particularly advanced non-small cell lung cancer failed or intolerant to standard therapy.

The ninth aspect also includes the use of the pharmaceutical combination described above in the manufacture of a medicament for treating non-small cell lung cancer, especially advanced non-small cell lung cancer, more particularly advanced non-small cell lung cancer failed or intolerant to standard therapy.

The pharmaceutical combination according to the seventh to ninth aspects described above may additionally contain other suitable active ingredients besides the first active ingredient and the second active ingredient, provided that the other suitable active ingredients do not impair the beneficial effects of the first active ingredient and the second active ingredient. Preferably, the pharmaceutical combination according to the seventh to ninth aspects described above contains the first active ingredient and the second active ingredient as the only active ingredients.

In some embodiments, the first active ingredient may be in the form of a single-dose administration unit as defined herein. In some embodiments, the first active ingredient can provide the patient with any dose level described herein, for example, any dose level defined in embodiments 43-59.

In some embodiments, the first active ingredient may be in the form of a kit comprising one or more single-dose administration units, wherein the one or more single-dose administration units are administered according to the dosage regimen defined herein, for example, any dosage regimen defined in embodiments 43-59.

In another aspect, the present invention relates to a kit or kit package comprising the pharmaceutical combination according to the seventh to ninth aspects described above. In one embodiment, the kit or pack comprises: a pharmaceutical composition comprising a first active ingredient; a pharmaceutical composition comprising a second active ingredient; and instructions for use, wherein the instructions for use describe a method of administering said pharmaceutical compositions. In one preferred embodiment, the kit or pack comprises: a pharmaceutical composition comprising the first active ingredient as the sole active ingredient; a pharmaceutical composition comprising the second active ingredient as the sole active ingredient; and instructions for use, wherein the instructions for use describe methods of administering the two pharmaceutical compositions. The pharmaceutical composition optionally contains pharmaceutically acceptable excipients.

The pharmaceutical combination of the present invention has an effective therapeutic effect on the cancer it treats, even showing additive, enhanced, or synergistic therapeutic effects, and has acceptable safety.

The above aspects and embodiments and each of their features, as well as the definitions below, can be arbitrarily combined to form embodiments not explicitly described in the specification but consistent with the spirit of the present invention, and these embodiments are also included within the scope of the present invention.

### Definitions

As used herein, wild-type "interleukin-2" or "IL-2" refers to the parental IL-2 protein used as the template for introducing mutations or mutation combinations of the present invention, preferably the naturally occurring IL-2 protein, for example, naturally occurring IL-2 proteins derived from human, mouse, rat, or non-human primates, including both unprocessed forms (for example with signal peptide) and processed forms (for example with signal peptide removed). A full-length natural human IL-2 sequence containing the signal peptide is shown as SEQ ID NO: 1, and the sequence of its mature protein is shown as SEQ ID NO: 2. Furthermore, such expression also includes naturally occurring IL-2 allelic variants and splice variants, isoforms, homologs, and species homologs. Such expression also includes variants of natural IL-2, for example, variants that have at least 95%-99% or higher identity to natural IL-2 or have no more than 1-10 or 1-5 amino acid mutations (for example conservative substitutions), and preferably have essentially the same IL-2Rα binding affinity and/or IL2Rβγ binding affinity as the natural IL-2 protein. Accordingly, in some embodiments, wild-type IL-2 compared to natural IL-2 protein may contain amino acid mutations that do not affect its binding to IL-2 receptor, for example, the natural human IL-2 protein with a C125S mutation introduced at position 125 (uniprot: P60568) belongs to the wild-type IL-2 of the present invention. An example of a wild-type human IL-2 protein containing the C125S mutation is shown as SEQ ID NO: 3. In some embodiments, the wild-type IL-2 sequence may have at least 85%, 95%, or even at least 96%, 97%, 98%, or 99% or higher amino acid sequence identity to the amino acid sequences of SEQ ID NO: 1, 2, or 3.

As used herein, amino acid mutations may include amino acid substitutions, deletions, insertions, and additions. Any combination of substitutions, deletions, insertions, and additions can be made to obtain a final mutated protein construct having desired properties (for example reduced IL-2Rα binding affinity and/or improved developability and/or weakened IL-2Rβγ binding). Amino acid deletions and insertions include deletions and insertions at the amino and/or carboxyl termini of the polypeptide sequence, as well as deletions and insertions within the polypeptide sequence. For example, an alanine residue can be deleted at position 1 of full-length human IL-2, or one or more amino acids can be deleted in the B'C' loop region to shorten the length of this loop region. In some embodiments, preferred amino acid mutations are amino acid substitutions, for example combinations of single amino acid substitutions or replacements of amino acid sequence segments. For example, the B'C' loop region sequence of wild-type IL-2 can be wholly or partially replaced with a different sequence (for example the B'C' loop of IL-15), preferably to obtain a shortened B'C' loop region sequence.

Throughout this document, when referring to amino acid positions in IL-2 protein or IL-2 sequence segments, they are determined by referencing the amino acid sequence SEQ ID NO: 3 of wild-type human IL-2 protein (also referred to as IL-2^{WT}). Corresponding amino acid positions on other IL-2 proteins or peptides (including full-length sequences or truncated fragments) can be identified by amino acid sequence alignment with SEQ ID NO: 3. Therefore, in the present invention, unless otherwise specified, the amino acid positions of IL-2 proteins or peptides are according to the numbering based on SEQ ID NO: 3. For example, when referring to "F42," it indicates the phenylalanine residue F at position 42 in SEQ ID NO: 3, or the corresponding amino acid residue at the aligned position on other IL-2 peptide sequences. Meanwhile, for ease of understanding and comparison, when the mutations in this invention involve truncation or deletion at certain specific loci of segments (for example the B'C' loop region sequence, that is, residues 73-83 totaling 11 amino acids in SEQ ID NO: 3), with the given specific mutation region and its mutation method, the numbering of amino acid residues outside this region remains unchanged. For example, after truncating the B'C' loop region sequence (residues 73-83 in SEQ ID NO: 3, 11 amino acids) to 7 amino acids, numbers 80-83 are no longer assigned, and the amino acid position numbering immediately following the B'C' loop region remains 84. Sequence alignment performed for determining amino acid positions can use the Basic Local Alignment Search Tool available at https://blast.ncbi.nlm.nih.gov/Blast.cgi, employing default parameters.

In this document, IL-2 mutein single amino acid substitutions are described as follows: [original amino acid residue/position/substituted amino acid residue]. For example, lysine at position 35 substituted by glutamic acid can be represented as K35E. When multiple alternative amino acid substitutions are possible at a given position (for example position K35 can be substituted by D or E), the substitution can be represented as: K35D/E. Accordingly, multiple single amino acid substitutions can be connected by a plus "+" or minus "-" sign to indicate combination mutations at multiple given positions. For example, combination mutations at positions F42A, N88R, and S127E can be represented as: F42A+N88R+S127E or F42A-N88R-S127E.

In this document, "percent sequence identity" can be determined by comparing two best alignment sequences within a comparison window. Preferably, sequence identity is determined over the full length of the reference sequence (for example SEQ ID NO: 3). The sequence alignment methods used for comparison are well known in the art. Algorithms suitable for determining percent sequence identity include, for example, BLAST and BLAST 2.0 algorithms (see Altschul et al., Nuc. Acids Res. 25: 3389-402, 1977 and Altschul et al. J. Mol. Biol. 215: 403-10, 1990). Software for performing BLAST analysis can be obtained from the United States National Center for Biotechnology Information. For the purposes of this application, the percentage of identity is determined using the Basic Local Alignment Search Tool available at https://blast.ncbi.nlm.nih.gov/Blast.cgi with default parameters.

In this document, antibody Fc fragment refers to the C-terminal region of the immunoglobulin heavy chain constant domain containing at least a portion thereof, and may include native sequence Fc fragments and variant Fc fragments. Native sequence Fc fragments encompass a variety of immunoglobulin Fc sequences naturally occurring, for example various Ig subclasses and their allotypic Fc regions (Gestur Vidarsson et al., IgG subclasses and allotypes: from structure to effector functions, 20 October 2014, doi: 10.3389/fimmu.2014.00520). In some embodiments, the human IgG heavy chain Fc fragment extends from Cys226 or from Pro230 of the heavy chain to the carboxyl terminus. In another embodiment, the C-terminal lysine (Lys447) of the Fc fragment may be present or absent. In other embodiments, the Fc fragment is a variant Fc fragment containing mutations, for example containing the L234A-L235A mutations. Unless otherwise indicated herein, the numbering of amino acid residues in the Fc fragment is according to the EU numbering system, also known as the EU index, as described in Kabat, E.A. et al., Sequences of Proteins of Immunological Interest, 5th edition, Public Health Service, National Institutes of Health, Bethesda, MD (1991), NIH Publication 91-3242. In some embodiments, the antibody Fc fragment may include an IgG1 hinge sequence or partial IgG1 hinge sequence at the N-terminus, for example, sequences ranging from E216 to T225 or from D221 to T225 according to EU numbering. Mutations may be present in the hinge sequence.

IL-2 protein belongs to the short-chain type I cytokine family members with a four α-helix bundle (A, B, C, D) structure. In this document, the terms "B'C' Loop," "B'C' loop region," or "B'C' loop sequence" are used interchangeably and refer to the connecting sequence between the B helix and C helix of the IL-2 protein. Through crystal structure analysis of IL-2 (for example PDB:2ERJ), an IL-2 protein B'C' loop sequence can be identified. For purposes of the present invention and according to the numbering of SEQ ID NO: 3, the B'C' loop sequence refers to the sequence connecting the residues at positions 72 and 84 of the IL-2 polypeptide. In the wild-type IL-2 proteins of SEQ ID NOs: 1, 2, and 3, this connecting sequence includes 11 amino acids from A73 to R83.

"Antigen-binding fragment" refers to a molecule different from the full antibody that contains a portion of the full antibody and binds the antigen bound by the full antibody. Examples of antibody fragments include but are not limited to Fv, Fab, Fab', Fab'-SH, F(ab')₂; dAb (domain antibody); linear antibodies; single-chain antibodies (for example scFv); single-domain antibodies for example VHH; bivalent antibodies or fragments thereof; or camelid antibodies.

The term "linker" as used herein refers to any molecule that enables direct connection of different parts of a fusion protein. Examples of linkers that covalently connect different parts of the fusion protein include peptide linkers and non-protein polymers, including but not limited to polyethylene glycol (PEG), polypropylene glycol, polyoxyalkylene, or copolymers of polyethylene glycol and polypropylene glycol. As used herein, the term "peptide linker" refers to a sequence of amino acids wherein the sequence connects the amino acid sequence of the first part of the fusion protein to the second part of the fusion protein. For example, the peptide linker can connect an IL-2 portion of the fusion protein to the Fc domain or a fragment thereof. For example, the peptide linker can also connect an antibody to IL-2, for example connecting the C-terminus of the antibody heavy chain to IL-2. Preferably, the peptide linker has such a length that is sufficient to connect two entities in a manner that they maintain their conformations relative to each other, so as not to interfere with the desired activity. The peptide linker may primarily include or may not primarily include the following amino acid residues: Gly, Ser, Ala, or Thr. Useful linkers include glycine-serine polymers, for example (GS)n, (GSGGS)n, (GGGGS)n, (GGGS)n, and (GGGGS)nG, where n is an integer of at least 1 (and preferably 2, 3, 4, 5, 6, 7, 8, 9, 10). Useful linkers also include glycine-alanine polymers, alanine-serine polymers, and other flexible linkers. Preferably, the linker of the present invention is (GGGGS)n, where n = 1, 2, 3, 4, or 5, preferably 2. Preferably, the linker of the present invention is SEQ ID NO: 5.

As used herein, the term "fusion" refers to a fusion formed by linking two or more originally separate proteins/genes/compounds. If the entities forming the fusion are proteins (including antibodies or fragments thereof), they are referred to as fusion proteins. For example, IL-2 linked to an Fc dimer can constitute an IL-2 fusion protein. Fusions of IL-2 with an antibody or fragment thereof may also be referred to as fusion proteins. The connection between the two entity molecules forming the fusion can be made with or without a linker.

As used herein, the term "PD-1/IL-2R bispecific fusion protein" refers to a polypeptide molecule comprising at least one IL-2 molecule and at least one PD-1 antibody or antibody fragment. As described herein, an IL-2 molecule can be connected to an antibody through various interactions and multiple configurations. For example, a fusion protein of IL-2 and Fc with an antibody fragment containing heavy chain and light chain can form a fusion protein by dimerization. Preferably, the fusion protein of the present invention has the structure shown in FIG. 1, or the structure shown in FIG. 1 with the IL-2 portion and the PD-1 antibody portion interchanged.

The terms "first" and "second" used herein for the Fc domain or monomers are employed for distinction when there is more than one module of each type. Unless explicitly stated otherwise, the use of these terms is not intended to impart any specific order or orientation to the fusion protein.

The term "priming dose" as used herein refers to a dose administered to an individual prior to administration of the normal dose of an antitumor drug (for example the fusion protein of the present invention) to improve the individual's tolerance to subsequent administration of the normal dose and/or to result in fewer or less severe adverse events upon subsequent administration of the normal dose. Typically, the priming dose is a lower dose, for example 2-50% of the normal dose, for example about 2%, 3.33%, 5%, 6.67%, 10%, 15%, 16.67%, 20%, 25%, 30%, 33.3%, 35%, 40%, 45%, or 50%, or a range composed of any two thereof. In some embodiments, when the single administration normal dose is ≥0.6 mg/kg, particularly ≥1 mg/kg body weight, a 1 lower dose (for example 100 µg/kg) is administered first as the priming dose.

The terms "normal dose" and "treatment dose" as used herein are interchangeable, indicating an amount sufficient to alleviate, improve, or inhibit one or more symptoms of a solid tumor or hematologic tumor, or to delay or inhibit progression of the solid tumor or hematologic tumor (for example progressive disease-free, extended survival). The normal dose may be administered once or multiple times.

The term "treatment cycle" as used herein refers to a specific period expressed in days or weeks, repeated according to a conventional timetable. The treatment cycle can be repeated once or multiple times (for example 2, 3, 4, 5, 6, 7, 8, 9, 10, 11, 12, 13, 14 times or more). Within one treatment cycle, administrations can be given once or multiple times. For multiple repeated treatment cycles, the intervals between each treatment cycle may be continuous or may have a gap (for example inability to administer on time due to various reasons including non-disease causes).

The expression "8±1" day used herein means day 7, 8, or 9, preferably day 8.

In the present text, the dose unit "µg/kg" refers to the microgram amount of the fusion protein of the present invention per kilogram of individual body weight; the dose unit "mg/kg" refers to the milligram amount per kilogram of individual body weight.

Herein, "QD" means once daily administration, "QW" means once weekly administration, "Q2W" means once every two weeks administration, and "Q3W" means once every three weeks administration.

The term "treatment" used herein refers to alleviating or reducing the severity of at least one symptom or indication, temporarily or permanently eliminating the cause of symptoms, delaying or inhibiting tumor growth, reducing tumor cell burden or tumor load, promoting tumor regression, causing tumor shrinkage, necrosis and/or disappearance, preventing tumor recurrence, preventing or inhibiting metastasis, inhibiting metastatic tumor growth, obviating the need for surgery, extending individual survival, and/or improving patient quality of life.

The term "objective response rate" or "ORR" used herein is one of the important indicators evaluating antitumor drug efficacy, referring to the proportion of patients whose tumor volume shrinks by 30% or more and is maintained for more than 4 weeks, which is the sum of patients with complete response (CR) and partial response (PR).

The term "disease control rate" or "DCR" used herein refers to the percentage of evaluable tumor patients who achieve complete response (CR), partial response (PR), and stable disease (SD) after receiving a certain treatment.

The terms "complete response (CR)", "partial response (PR)", "stable disease (SD)", and "progressive disease (PD)" are commonly used in the field to evaluate tumor treatment efficacy and have their usual meanings as understood by those skilled in the art. Typically, complete response (CR) refers to the complete disappearance of the tumor, and such disappearance persists for at least one month. partial response (PR) refers to a reduction in tumor volume, but not complete disappearance. Usually, when tumor volume decreases by 50% or more, it is considered a partial response. Stable disease (SD) means there is no significant change in tumor size, indicating the disease has neither worsened nor significantly improved. progressive disease (PD) means there is an increase in tumor size or the appearance of new tumors, which usually indicates poor tumor control in the patient.

The term "overall survival" or "OS" as used herein refers to the time from the first administration of the studied drug to the death from any cause.

The term "progression free survival" or "PFS" as used herein refers to the time from the first administration of the studied drug to progressive disease or death from any cause.

The terms "cancer", "carcinoma", "tumor", and "neoplasm" are used interchangeably herein to refer to tissue masses formed by abnormal, uncontrolled cell proliferation that can occur at any site in the body and are characterized by extension and infiltration into surrounding tissues.

The term "solid tumor" as used herein refers to an abnormal tissue mass that typically does not contain cystic or liquid areas. Solid tumor can be benign (non-cancerous) or malignant (cancerous), particularly malignant solid tumors.

The term "other solid tumors" as used herein refers to solid tumors other than colorectal cancer, melanoma, non-squamous non-small cell lung cancer, hepatocellular carcinoma, and lymphoma, including but not limited to renal carcinoma, esophageal cancer (for example, esophageal squamous cell carcinoma), gastric cancer (for example, gastric and gastroesophageal junction adenocarcinoma), pancreatic cancer, breast cancer (for example, triple-negative breast cancer), cholangiocarcinoma, ovarian cancer, cervical cancer, endometrial cancer, head and neck cancer (for example, head and neck squamous cell carcinoma), and the like.

The term "standard treatment failure" as used herein refers to cancer that has been treated with at least one first-line therapy or even all available therapeutic agents associated with the term, without providing significant therapeutic benefit to the patient. For example, the term "advanced melanoma with prior immunotherapy failure" as used herein refers to advanced melanoma patients who did not derive significant therapeutic benefit from immunotherapy. Interleukin 2 (IL-2) is a cytokine primarily secreted by antigen-activated CD4+ T cells, with a molecular weight of approximately 15.5 kDa. IL-2 is one of the earliest approved immunotherapies for antitumor treatment. The FDA approved high-dose IL-2 for treatment of metastatic renal cell carcinoma and metastatic melanoma in 1992 and 1998, respectively. If an advanced melanoma patient has been treated with IL-2 but no significant therapeutic benefit was observed, this is considered "advanced melanoma with prior immunotherapy failure".

The term "standard therapy intolerant" as used herein refers to a cancer that related to the term exhibits an inappropriate benefit/risk ratio due to excessive side effects caused by treatment with at least one first-line therapy drug or even all available therapeutic drugs.

The term "pharmaceutically acceptable" as used herein refers to substances, compositions, formulations, etc. that do not cause excessive toxicity, irritation, allergic reactions, or other undesirable properties to mammals, especially humans, and have a reasonable benefit/risk ratio when administered to animals or humans.

The term "pharmaceutically acceptable salt" as used herein includes but is not limited to acid addition salts or base addition salts, for example acid addition salts formed with inorganic acids , for example hydrochloride, hydrobromide, carbonate, bicarbonate, phosphate, sulfate, sulfite, nitrate and the like; and acid addition salts formed with organic acids, for example formate, acetate, malate, maleate, fumarate, tartrate, succinate, citrate, lactate, methanesulfonate, p-toluenesulfonate, 2-hydroxyethanesulfonate, benzoate, salicylate, stearate, and salts formed with chain alkane dicarboxylic acids of the formula HOOC-(CH₂)ₙ-COOH (where n is 0-4). The term "pharmaceutically acceptable salt" also includes base addition salts formed by compounds bearing acidic groups with pharmaceutically acceptable cations for example sodium, potassium, calcium, aluminum, lithium, and ammonium.

The term "pharmaceutical composition" as used herein refers to a single pharmaceutical formulation containing active ingredients and optionally one or more pharmaceutically acceptable excipients. The pharmaceutical formulation can be used for various administration routes, for example oral administration, parenteral administration, topical administration, and the like. The term "parenteral" as used herein includes subcutaneous, intradermal, intravenous, intraarterial, intramuscular, intra-articular, intrasynovial, intrastemal, intraspinal, intracranial injection or infusion and the like. The pharmaceutical formulation can be any suitable dosage form, for example tablets, hard capsules, soft capsules, powders, suppositories, solutions, emulsions, suspensions, gels, creams, ointments, sprays, and the like. Suitable pharmaceutically acceptable excipients for formulating various preparations are known in the art (see, for example, Romingsheng and Gaotianhui, Eds., "The Complete Guide to Pharmaceutical Excipients", 2nd Edition, January 2006, Sichuan Science and Technology Press). For example, excipients used for preparing tablets or hard capsules include diluents (for example corn starch, lactose, dextrates, sucrose, mannitol, sorbitol, cellulose), lubricants (for example magnesium stearate), disintegrants (for example croscarmellose sodium and sodium carboxymethyl starch), binders (for example starch paste and povidone), colorants (for example titanium dioxide, β-carotene and sunset yellow), and the like. Excipients for preparing injectable solutions include solvents (for example water for injection, glucose solution, physiological saline and 1,3-butanediol), surfactants (for example Tween 80), and the like. pharmaceutical compositions can contain 1-99% w/w, preferably 1-50% w/w, more preferably 1-20% w/w of active ingredients.

The term "pharmaceutical combination" as used herein can be a fixed combination, i.e., a single pharmaceutical composition containing the first active ingredient and the second active ingredient, or a non-fixed combination (for example a kit or a pack) containing: a pharmaceutical composition comprising the first active ingredient, a pharmaceutical composition comprising the second active ingredient, and instructions describing the administration method of the pharmaceutical combination. Active ingredients in the pharmaceutical combination of the present invention can be administered in a single pharmaceutical composition or simultaneously administered in separate pharmaceutical compositions without specific time restrictions or separately administered at the same or different time intervals to provide beneficial therapeutic effects in the patient, preferably so that the two active ingredients exhibit additive, enhanced or synergistic therapeutic effects and/or fewer side effects in the patient.

In addition to the first active ingredient and the second active ingredient, the pharmaceutical combination of the present invention can further comprise additional active ingredients, provided that the aforementioned beneficial effects of the first and second active ingredients are not affected. Similarly, the additional active ingredients can be in the same pharmaceutical composition with the first active ingredient and/or second active ingredient or in separate pharmaceutical compositions. The active ingredients in the pharmaceutical combination of the present invention can have the same or different dosage forms and can be administered by the same or different administration routes.

The dosage regimen and dose of active ingredients in the pharmaceutical combination of the present invention are selected based on various factors including the type, species, age, weight, sex, and overall health condition of the patient; the disease and its severity to be treated; and the route of administration. A physician, clinician, or veterinarian skilled in the art can readily determine the effective amount required.

As used herein, the term "about" when referring to a specific value means that the value can vary by no more than 5%, preferably no more than 1%, of the stated value. For example, the expression "about 100" as used herein includes 95 and 105 as well as all values between them (for example 95.1, 95.2, 96, etc.).

All numerical ranges disclosed herein should be understood as disclosing each value and subrange within the range, including the endpoints, regardless of whether such values or subranges are specifically disclosed. For instance, mention of any numerical range should be deemed to disclose each value within that range. The invention encompasses all values falling within these ranges, all smaller ranges, and upper or lower limits of the numerical ranges.

It is understood that the terms used herein are solely for describing specific embodiments and are not intended to limit the invention. The scope of the invention is only limited by the appended claims.

Unless otherwise stated, all technical and scientific terms used herein have the same meanings as commonly understood by those skilled in the art to which the invention pertains. Technical and scientific terms not specifically defined herein have meanings commonly understood by those skilled in the art pertaining to this invention.

### BRIEF DESCRIPTION OF THE DRAWINGS

FIG. 1 shows a schematic diagram of the structure of the PD-1/IL-2R bispecific antibody fusion protein **2149**.
FIG. 2 shows the dosage regimen of molecule **2149** in a phase Ia clinical study.
FIG. 3 shows the dosage regimen of molecule **2149** and/or bevacizumab in a phase Ia clinical study.

### Examples

The following examples are provided to illustrate and explain the present invention. These examples are illustrative only and do not in any way limit the scope of the invention or the appended claims. Similarly, the invention is not limited to any particular preferred embodiments described herein. Those skilled in the art can make modifications to the present invention without departing from the spirit and scope thereof.

### Example 1. Phase Ia/Ib clinical study of PD-1/IL-2R bispecific antibody fusion protein 2149 in subjects with advanced solid tumors or lymphoma

PD-1/IL-2R bispecific fusion protein **2149** was studied in multiple different cancer models, including metastatic cancer models and PD-1 resistant models. Results from these preclinical studies showed that PD-1/IL-2R bispecific fusion protein **2149** exhibited antitumor activity and survival prolonging efficacy in different cancer models. In crab-eating macaque experiments, the molecule **2149** also demonstrated good efficacy at tolerable dose levels. These results indicate that molecule 2149 treatment has potential therapeutic benefits for patients with recurrent or refractory tumors who have limited treatment options and no known curative therapies.

During this trial, risk assessments will be continuously conducted and safety data will be regularly reviewed. If any evidence suggests an unreasonable risk to continue the trial, this clinical trial will be terminated.

The phase Ia clinical trial aims to determine the Maximum Tolerated Dose (MTD) of the drug by observing dose-limiting toxicity (DLT) according to the dose escalation scheme in the trial protocol.

To avoid discomfort or additional risk caused by venipuncture and multiple blood collections, this trial allows the use of intravenous indwelling catheters. Furthermore, the blood sample collection schedule has been designed to minimize the number and total volume of blood samples while meeting the pharmacokinetics study requirements of the investigational drug, thus reducing associated risks and improving subject compliance.

### 1. Study objectives and endpoints

| **Study objectives** | **Study endpoints** |
|---|---|
| Primary objectives | Primary endpoints |
| • To evaluate the safety and tolerability of molecule **2149** in subjects with advanced solid tumors or lymphoma; | • Incidence of dose-limiting toxicity (DLT) events; |
| | • Incidence and severity of all adverse events (AE), treatment emergent adverse events (TEAE), immune-related adverse events (irAE), and serious adverse events (SAE) (per CTCAE v5.0), treatment emergent adverse events leading to treatment discontinuation and death, and their relation to the investigational drug; |
| • To evaluate the dosage regimen of molecule **2149** to determine the recommended phase 2 dose (RP2D). | |
| | • Changes in vital signs, physical examinations, and laboratory test values before and after study treatment. |

| Secondary objectives | Secondary endpoints |
|---|---|
| • To evaluate the pharmacokinetics (PK) characteristics of molecule **2149** in subjects with advanced solid tumors or lymphoma; | PK evaluation: |
| | • pharmacokinetics parameters including but not limited to peak concentration (Cₘₐₓ), area under the drug concentration-time curve (AUC), half- |
| • To evaluate the immunogenicity of molecule **2149** in subjects with advanced solid tumors or lymphoma; | life (t_{1/2}), clearance (CL), and volume of distribution (V). |
| | Immunogenicity evaluation: |
| | • To evaluate the immunogenicity of molecule |
| • To evaluate the antitumor activity of molecule **2149** in subjects with advanced solid tumors or lymphoma. | **2149**. |
| | Efficacy evaluation: |
| | • solid tumor: to evaluate subjects' objective response rate (ORR), time to response (TTR), duration of response (DoR), disease control rate (DCR), progression free survival (PFS), and 6-month and 1-year PFS rates according to RECIST v1.1; |
| | • lymphoma: to evaluate subjects' ORR, TTR, DoR, DCR, PFS, and 6-month and 1-year PFS rates according to Lugano 2014 criteria; |
| | • overall survival (OS) and survival rates (6 months and 1 year) of subjects. |

| Exploratory objectives | Exploratory endpoints |
|---|---|
| • To explore the correlation between PD-L1 expression levels in tissue samples and immune cell infiltration in the tumor immune microenvironment (for example CD8+ T cells) with efficacy. | Biomarker evaluation in tumor tissue: |
| | • To explore the relationship between PD-L1 expression levels in tumor tissue at subject baseline and efficacy. |
| | • To explore the relationship between the tumor microenvironment (for example CD8+ T cells) in tumor tissue at subject baseline and efficacy. |
| • To explore the pharmacodynamics (PD) characteristics of molecule **2149** in subjects with advanced solid tumor or lymphoma. | |
| | • To explore the changes in PD-L1 expression levels and tumor microenvironment (for example CD8+ T cells) in tumor tissue post-treatment compared to baseline. |
| | Biomarker (PD indicators) evaluation in whole blood: |
| | • To explore changes over time in receptor occupancy. |
| | • To explore changes over time in immune cell subsets in peripheral blood (for example CD8+ T cells, Treg, etc.). |

### 2. Study design

### 2.1. Study protocol

This study is an open-label, multicenter Phase Ia/Ib study. It aims to assess the safety, tolerability, and preliminary efficacy of molecule **2149** in subjects with advanced, recurrent, metastatic solid tumor or lymphoma, to determine its maximum tolerated dose (MTD) or maximum administered dose (MAD), and to subsequently establish its recommended phase II dose (RP2D).

### ➢ Phase Ia Part A:

The dose escalation part of Phase Ia Part A will adopt a staged accelerated titration method and Bayesian Optimal Interval (BOIN) design, with a total of 8 dose groups planned and a maximum sample size of 48 subjects.

The administration route of molecule **2149** is intravenous infusion, with a dosing frequency of once weekly (QW), and the DLT observation period is 21 days after the first dose. After completing the DLT observation period, subjects will continue to receive the investigational drug at the same dose level until progressive disease, intolerable toxicity, subject withdrawal, other reasons requiring termination of study treatment, or treatment duration reaches 24 months (whichever occurs first).

### Accelerated titration phase:

The accelerated titration dose escalation phase includes 4 dose groups (0.2 µg/kg, 2 µg/kg, 10 µg/kg, and 30 µg/kg). The starting dose and dosing frequency of molecule **2149** are 0.2 µg/kg, QW. In the accelerated titration phase, 1 subject is enrolled per dose group. If no CTCAE ≥ grade 2 toxicity related to molecule **2149** is observed during the DLT observation period, subsequent dose groups will continue the accelerated titration design, enrolling 1 subject for DLT observation; if CTCAE ≥ grade 2 toxicity related to molecule **2149** is observed during the DLT observation period (excluding fever, nausea/vomiting, anemia, fatigue, and any grade 2 symptoms or abnormal laboratory values present at baseline), 2 additional subjects will be enrolled at the current dose, and dose escalation will proceed using the BOIN design from this dose group onward. (For example, if 1 subject is enrolled in the 30 µg/kg dose group during the accelerated titration phase and CTCAE ≥ grade 2 toxicity related to molecule **2149** is observed during the DLT observation period, dose escalation will continue under the BOIN design beginning from the 30 µg/kg dose group.)

### BOIN dose escalation phase:

The BOIN dose escalation phase includes 4 dose groups (100 µg/kg, 300 µg/kg, 600 µg/kg, and 1 mg/kg). The target toxicity rate for the maximum tolerated dose in the dose escalation portion of this study is 0.3. Each dose group initially allows enrollment of 3-6 subjects. After completing the DLT observation, if further enrollment is required according to the dose escalation/de-escalation or elimination rules, this dose group will continue to enroll subjects to receive treatment. The MTD is defined as the dose level for which the DLT incidence within 21 days (28 days for Q2W dosing) after the subject's first administration of the investigational drug is closest to the target toxicity rate (i.e., 0.3). Decisions for dose escalation/de-escalation or elimination depend on the posterior probability of the toxicity rate. If the posterior probability that the DLT incidence at the current dose exceeds the target toxicity rate is greater than 95%, the current and higher doses will be eliminated. The specific rules for dose escalation/de-escalation or elimination are shown in the following table.

| **Decision** | **Total number of subjects treated at the current dose** | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Increase dose if number of DLT subjects ≤ | 0 | 0 | 0 | 0 | 1 | 1 | 1 | 1 | 2 | 2 | 2 | 2 |
| Reduce dose if number of DLT subjects ≥ | 1 | 1 | 2 | 2 | 2 | 3 | 3 | 3 | 4 | 4 | 4 | 5 |
| Eliminate dose if number of DLT subjects ≥ | NA | NA | 3 | 3 | 4 | 4 | 5 | 5 | 5 | 6 | 6 | 7 |

Footnote: In extreme cases where more than 12 patients are treated at a single dose level, refer to the dose rules table of BOIN design on https://trialdesign.org/.

The criteria for early termination of the dose escalation study are: 1) elimination of the lowest dose due to toxicity; or 2) the number of subjects at a certain dose reaching 6 and the decision for the next cohort is to maintain the current dose; or 3) the total number of subjects reaches the maximum sample size of 48.

During the dose escalation phase, if a subject experiences DLT, based on human pharmacokinetics, safety data, and preclinical data, and after discussion with investigators, the dosing interval for subsequent administration to that subject may be extended. During the dose escalation phase, in situations including but not limited to the inability to conduct QW dose ramp-up for subsequent subjects, the dosing frequency may be adjusted after discussion with investigators based on human pharmacokinetics, safety data, and preclinical data, to dose escalation with dosing every two weeks (Q2W) or every three weeks (Q3W). The DLT observation periods are 28 days after the first dose (Q2W) and 21 days (Q3W), respectively.

During the dose escalation phase, if MTD is not observed, higher dose administration may be explored after discussion with investigators based on human pharmacokinetics, safety data, preclinical data, and other factors.

### ➢Phase Ia Part B:

The sponsor may, based on the PK, safety, and preliminary efficacy data from Phase Ia Part A, further expand enrollment to observe safety and conduct dose optimization studies (including but not limited to extending the dosing interval). Specifically:
(1) Select dose groups with confirmed safety from Phase Ia Part A, enrolling 3-20 subjects per dose group for QW/Q2W/Q3W administration studies.
(2) After safety evaluation for 600 µg/kg QW in Phase Ia Part A is completed, Part B dosing of 1 mg/kg Q3W and 1 mg/kg Q2W is performed. First enroll 3-6 subjects for safety evaluation; if no intolerable safety signals occur, continue enrollment for that dose group. If intolerable safety signals appear, the safety evaluation committee will decide on exploring other dose administrations or dosing intervals based on human pharmacokinetics, safety data, preclinical data, and other considerations. Each dose group may enroll 10-30 subjects. Enrollment of various dose groups may proceed simultaneously or sequentially.
(3) After safety evaluations for 1 mg/kg Q3W and 1 mg/kg Q2W dosing in Part B are completed, proceed with 1.5 mg/kg Q3W dosing in Part B. First enroll 3-6 subjects for safety evaluation; if no intolerable safety signals occur, continue enrollment for that dose group. If intolerable safety signals appear, the safety evaluation committee will decide on exploring other dose administrations or dosing intervals based on human pharmacokinetics, safety data, preclinical data, and other considerations. Each dose group may enroll 10-30 subjects.
(4) After safety evaluation of 1.5 mg/kg Q3W in Part B, proceed with 2 mg/kg Q3W dosing. First enroll 3-6 subjects for safety evaluation; if no intolerable safety signals occur, continue enrollment for that dose group. If intolerable safety signals appear, the safety evaluation committee will decide on exploring other dose administrations or dosing intervals based on human pharmacokinetics, safety data, preclinical data, and other considerations. Each dose group may enroll 10-30 subjects.
(5) After completion of the safety evaluation for Part B 2 mg/kg Q3W, administration at 2 mg/kg Q2W and 3 mg/kg Q3W will be conducted. First enroll 3-6 subjects for safety evaluation; if no intolerable safety signals occur, continue enrollment for that dose group. If intolerable safety signals appear, the safety evaluation committee will decide on exploring other dose administrations or dosing intervals based on human pharmacokinetics, safety data, preclinical data, and other considerations. Each dose group may enroll 10-30 subjects. Enrollment of various dose groups may proceed simultaneously or sequentially.
(6) Dose exploration of Part B combination therapy: after completion of the safety evaluation for Part B 1.5 mg/kg Q3W monotherapy, dose exploration of Part B in combination with bevacizumab (trade name: Dayoutong) will be conducted. The dosage regimen of molecule **2149** includes three dose groups: 1.5 mg/kg Q3W, 2 mg/kg Q3W, and 3 mg/kg Q3W (it is also possible, based on accumulated safety data, efficacy data, PK/PD data, and other clinical study data of molecule **2149**, to start escalation from a molecule **2149** combination dose that has been verified to be safe, or to explore other administration doses), and the administration dose of bevacizumab is 7.5 mg/kg Q3W. First, 3-6 subjects will be enrolled for safety evaluation; if no intolerable safety signal occurs, this dose group may continue to be expanded for enrollment. If intolerable safety signals appear, the safety evaluation committee will decide on exploring other dose administrations or dosing intervals based on human pharmacokinetics, safety data, preclinical data, and other considerations. Each dose group may enroll 10-30 subjects. Based on the accumulated safety data of monotherapy, for dose escalation in combination therapy, 1 low dose of molecule **2149** (≤100 µg/kg) (C1D-7) may also be administered first as a priming dose, and 7 days later (C1D1, ±1 day), normal-dose molecule **2149** treatment will be administered as planned. For example, for the 2 mg/kg and 3 mg/kg dose groups, after discussion with the investigator, one 100 µg/kg molecule **2149** treatment may be administered at C1D-7, and 7 days later (C1D1, ±1 day), 2 mg/kg or 3 mg/kg molecule **2149** treatment will be administered, respectively. The sponsor may evaluate the benefits and risks of gradient dosing by comparing the safety and efficacy of the two administration methods.

For tumor types in which efficacy signals are observed, the same tumor type under the same dosage regimen (including treatment in combination with bevacizumab) may be further expanded by enrolling 20-50 subjects on the basis of the originally preset sample size. The investigator may also terminate exploration of a certain administration method in advance based on the obtained efficacy, safety, clinical pharmacology, and other data. During implementation, the sponsor may further adjust the number of enrolled subjects based on PK, preliminary efficacy, and safety data, and the actual number of enrolled subjects may differ from the planned number of enrolled subjects. Meanwhile, according to preliminary analysis results of antitumor efficacy and biomarkers, some populations with specific biomarkers may subsequently be prospectively enrolled in specific tumor types, for example, expanded enrollment of about 30 patients with advanced colorectal cancer having PD-L1 CPS≥1.

### ➢Phase Ia Part C

Part C of Phase Ia is the randomized controlled dose optimization part: after safety evaluation at 3 mg/kg Q3W (generally requiring at least 6 subjects to have received this dose for more than one cycle) is completed, this dose optimization part can proceed. This part will enroll 60-90 subjects with advanced non-small cell lung cancer without known driver gene mutations who have failed prior immunotherapy. Subjects will be randomized in a 1:1:1 ratio into three groups to receive molecule**2149** at 600 µg/kg Q3W, 1.5 mg/kg Q3W, or 3 mg/kg Q3W treatment respectively [subjects will receive one low dose of **2149** ≤100 µg/kg at C1D-7 as priming dose, followed 7 days later (C1D1, ±1 day) by the planned normal dose of **2149** treatment]. Randomization stratification factors include: tumor histological type (adenocarcinoma vs. squamous cell carcinoma), number of prior systemic therapy lines (1 line vs. ≥2 lines). During the process, the sponsor may decide to stop enrollment of a certain dose group based on a comprehensive evaluation of preliminary data. The sponsor may decide whether to further expand enrollment based on PK, preliminary efficacy, and safety data.

### ➢Phase Ib

The investigator and sponsor will comprehensively determine the recommended dosing dose and dosing frequency of molecule **2149** in Phase Ib based on PK, safety, and efficacy data obtained from Phase Ia. Phase Ib preliminary evaluation of the safety and efficacy of molecule **2149** in treating subjects with advanced or metastatic solid tumor or lymphoma includes the following cohorts:
Cohort A: subjects with advanced colorectal cancer who have failed standard treatment:
   Cohort A2: approximately 30-60 subjects with advanced colorectal cancer who have failed standard treatment, receiving molecule **2149** combined with bevacizumab treatment.
   *Note:*
      *1) The doses of molecule **2149** and bevacizumab will be discussed and determined by the sponsor and investigators after reviewing clinical data from Phase Ia and other clinical study data of molecule **2149**. During the trial, based on comprehensive analysis of safety, efficacy, PK, and PD data of enrolled subjects, subsequent adjustments to the dosing dose or frequency of molecule **2149** and*/*or bevacizumab may be made;*
      *2) During enrollment, based on preliminary analysis of antitumor efficacy and biomarkers, specific populations with certain biomarkers, for example those with PD-L1 CPS≥1, may be prospectively enrolled.*
      *3) During the trial, with data accumulation and after discussion between the sponsor and investigators, further enrichment of subjects with specific disease characteristics (for example subjects without liver metastasis) may be conducted.*
Cohort B: subjects with advanced melanoma:
   Cohort B1: 20-30 subjects with advanced melanoma who are immunotherapy-naïve will be enrolled to receive molecule **2149** combined with bevacizumab treatment.
   Cohort B2: 20-40 subjects with advanced melanoma who failed prior immunotherapy are enrolled and treated with molecule **2149** combined with bevacizumab.
   Cohort B3: 20-30 subjects with advanced melanoma without systemic therapy are enrolled and treated with molecule **2149** combined with dacarbazine.
Cohort C: Subjects with advanced non-small cell lung cancer who have failed or are intolerant to standard therapy:
   Cohort C1: 20-60 subjects with advanced non-small cell lung cancer failed or intolerant to standard therapy are enrolled and treated with molecule **2149** as monotherapy.
   Cohort C2: 20-80 subjects with advanced non-squamous non-small cell lung cancer failed or intolerant to standard therapy are enrolled and treated with molecule **2149** combined with bevacizumab.
   Cohort C3: 20-80 subjects with advanced non-small cell lung cancer failed or intolerant to standard therapy are enrolled and treated with molecule **2149** combined with anlotinib.
Cohort D: 20-60 subjects with advanced hepatocellular carcinoma without systemic therapy are enrolled and treated with molecule **2149** combined with bevacizumab.
Cohort E: 20-60 subjects with advanced renal cell carcinoma failed or intolerant to standard therapy are enrolled and treated with molecule **2149** combined with bevacizumab.
Cohort F: 20-40 subjects with other advanced solid tumors or lymphomas failed or intolerant to standard therapy are enrolled and treated with molecule **2149** combined with bevacizumab.

During the enrollment of the above cohorts, based on preliminary analysis results of antitumor efficacy and biomarkers, subsequent prospective enrollment may be directed to populations with specific biomarkers, for example, those with tumor-associated CD8⁺ T cells ≥1%, or populations enriched based on tumor tissue CD8⁺ T cell cut-off values corresponding to optimal clinical benefit; or populations with PD-L1 CPS ≥1.

If during the trial any cohort exhibits unfavorable changes in risk/benefit, after discussion between investigators and sponsors, further enrollment in that cohort may be stopped to protect participant's safety.

### Supplementary notes

This study permits subjects to perform self-dose escalation. Investigators and sponsors will comprehensively assess the potential clinical benefit and possible safety risks for subjects to decide whether they can proceed to treatment at higher dose levels confirmed as safe. After self-dose escalation, all subject safety monitoring, PK sampling, tumor imaging visits, and other assessments should continue to follow previous arrangements. For subjects receiving molecule **2149** monotherapy, investigators and sponsors may adjust treatment in subsequent treatment cycles to molecule **2149** combined with bevacizumab after comprehensive assessment of potential clinical benefit and safety risks.

For dose groups with single doses ≥600µg/kg, it is recommended to administer one low dose of molecule **2149** (≤100 µg/kg) on C1D-7 as priming dose, followed 7 days later (C1D1, ±1 day) by the planned normal dose of molecule **2149** treatment. For example, in the 2 mg/kg Q3W, 2 mg/kg Q2W, and 3 mg/kg Q3W dose groups, after discussion with investigators, it is recommended to administer one 100µg/kg dose of molecule **2149** on C1D-7, then 7 days later (C1D1, ±1 day) administer 2 mg/kg Q3W, 2 mg/kg Q2W, and 3 mg/kg Q3W molecule **2149** treatments, respectively. The sponsor will evaluate the benefits and risks of gradient dosing by comparing the safety and efficacy of two dosing methods (with or without priming dose). It may also compare the differences in safety, efficacy, PK, and biomarkers between different priming dosing doses (for example 30 µg/kg and 100 µg/kg) to select the optimal priming dosing dose.

Subjects will receive the investigational drug treatment until progressive disease, intolerable toxicity, unwillingness to continue participating, occurrence of other reasons requiring treatment discontinuation, or treatment duration reaching 24 months (whichever occurs first).

As the study progresses and data accumulate, after discussion between the sponsor and investigators, subjects who show clinical benefit (for example achieving SD or above efficacy and lasting more than 2 months) may have the dosing interval of molecule **2149** extended (for example 3 to 8 weeks) for maintenance therapy, or switch to sintilimab for maintenance therapy; it is also possible to adopt a regimen of fixed treatment cycles of molecule **2149** followed by sequential sintilimab maintenance therapy, for example: molecule **2149** induction treatment for 4 to 6 cycles, followed by sequential sintilimab maintenance therapy.

This study evaluates the efficacy of subjects according to RECIST v1.1 or Lugano 2014 evaluation criteria. During the study, subjects will undergo tumor imaging assessments every 6 weeks (±7 days) after C1D1 until progressive disease, initiation of new antitumor treatment, unwillingness to continue, loss to follow-up, death, or study termination (whichever occurs first).

Subjects will have an end-of-treatment visit within 10 days of treatment discontinuation confirmation. Safety follow-up should be conducted 30 (+7) days after the last dose, after which subjects will enter survival follow-up (every 12 weeks ±7 days).

### 2.2. Dose-limiting toxicity (DLT)

In this study, DLT refers to any of the following adverse events related to **2149** occurring within days 1 to 21 of the phase Ia accelerated titration and BOIN dose escalation stages (28 days for Q2W dosing) and matching the specified severity. Adverse event severity will be graded according to the Common Terminology Criteria for adverse events (CTCAE) version 5.0 (CRS will be graded according to the CRS criteria). During the accelerated titration stage, if a subject receives <75% of the planned dose in the first cycle due to reasons other than toxicity (for example progressive disease, missed appointments, non-compliance, subject withdrawal), the sponsor may replace the subject with a new one.
(1) Hematologic toxicity:
   - grade 4 neutropenia lasting more than 5 days;
   - febrile neutropenia, defined as an absolute neutrophil count (ANC) <1.0×10⁹/L with a single temperature >38.3°C or sustained temperature (>1 hour) ≥38°C;
   - grade 3 thrombocytopenia with bleeding or requiring transfusion;
   - grade 4 thrombocytopenia;
   - grade 4 anemia unexplained by underlying disease;
(2) Non-hematologic toxicity:
   - grade ≥ 3 irAE that do not improve to grade ≤ 2 within 7 days after interventional treatment or to grade ≤ 1 within 21 days (excluding well-controlled grade 3 endocrine disorders and grade 3 tumor site inflammatory responses).
   - Any other grade ≥ 3 non-hematologic toxicity, except for the following conditions:
      √ grade 3 fatigue, fever, or constipation;
      √ grade 3 nausea, vomiting, or diarrhea with a duration ≤3 days after optimal supportive treatment;
      √ transient grade 3 CRS occurring for the first time, for example, recovery to baseline level or grade ≤ 1 within 72 hours;
      √ grade 3 skin toxicity improving within 24 hours with treatment (for example chlorpheniramine maleate);
      √ grade 3 hypotension or hypertension;
      √ grade 3 vascular leak syndrome lasting ≤7 days;
      √ grade 3 allergic reactions, for example rapid-onset allergic reactions that can be quickly controlled by symptomatic treatment;
      √ grade 3 QTc interval prolongation (QTcF≥501ms) occurring in asymptomatic subjects, immediate repeat testing is recommended, professional reassessment, and correction of reversible underlying causes for example electrolyte abnormalities or hypoxia. If grade 3 QTc prolongation persists after correction of any reversible causes, the event is considered a DLT;
      √ grade 3 asymptomatic electrolyte abnormalities lasting ≤3 days without clinical complications, capable of spontaneous recovery or responsive to appropriate medical intervention;
      √ asymptomatic grade 3 amylase or lipase elevation; grade 3 glutamyl transferase elevation, grade 3 ALT or AST elevation lasting <7 days with interventional treatment;
      √ grade 3 bone/joint pain lasting ≤3 days without clinical complications, capable of spontaneous recovery or responsive to appropriate medical intervention;
      √ Other grade 3 asymptomatic, clinically insignificant laboratory test abnormalities considered by investigators to be spontaneously reversible or resolved with appropriate medical interventions.
(3) Liver function results meeting Hy's law criteria:
   - AST and/or ALT elevation >3×ULN (or if elevated at study entry due to tumor liver involvement, then >3× baseline), and
   - total bilirubin ≥ 2×ULN, without cholestasis (i.e., serum alkaline phosphatase [ALP] < 2×ULN), and
   - the above results cannot be explained, for example by viral liver injury, past or acute liver diseases, or other drugs or conditions that can cause liver damage.
(4) Any death that cannot be excluded as related to the investigational drug.
(5) Other toxicities at any level deemed necessary for early termination after joint discussion between the investigator and the sponsor.

Note:
- grade 3 or grade 4 infusion related reaction (IRR) is not considered a DLT. However, if grade 4 IRR or grade 3 IRR occurs after premedication, the subject will need to discontinue study treatment and be replaced by a new subject;
- All grade ≥ 3 irAEs must be reviewed by the safety evaluation committee to determine if they constitute a DLT.

To evaluate DLT, the subject must meet any of the following conditions:
(a) During the dose escalation phase, a DLT occurred within the DLT observation window.
(b) Receiving at least 75% of the planned dose in cycle 1 and completing the observation within the DLT window. If treatment-related toxicity leads to dosing delays during the DLT observation period, the DLT observation period will be extended accordingly, as determined by the safety evaluation committee.

During the accelerated titration phase, new subjects must be enrolled to supplement those who do not meet the above conditions.

### 2.3. Maximum tolerated dose (MTD) and maximum administered dose (MAD)

MTD is defined as the dose level at which the DLT incidence rate is closest to the target toxicity rate (i.e., 0.3) within 21 days after subjects' first administration of the investigational drug (28 days for Q2W dosing).

In the dose escalation phase, once the MTD is reached, that dose is considered the maximum administered dose (MAD); if MTD is never reached, the highest dose level in all parts of the study design will be the MAD. In this study, if MTD is never reached, the maximum administered dose in phase Ia dose escalation will be 1 mg/kg.

### 3. Study population

### 3.1 Inclusion Criteria

Enrolled subjects (subjects must meet all the following criteria):
(1) Signed written informed consent and able to comply with the protocol-specified visit schedule and related procedures.
(2) Age ≥18 years, any gender.
(3) Histologically or cytologically confirmed unresectable locally advanced or metastatic solid tumor or lymphoma;

### Phase Ia study:

Priority enrollment of subjects with advanced colorectal cancer, melanoma, non-small cell lung cancer, triple-negative breast cancer, pancreatic cancer, cholangiocarcinoma, ovarian cancer, cervical cancer, head and neck squamous cell carcinoma, etc. In the Phase Ia Part B specific biomarker-enriched cohort: subjects with advanced colorectal cancer confirmed by histopathological examination with PD-L1 CPS ≥1, and confirmed by local standards as proficient mismatch repair (pMMR) or microsatellite stable (MSS).

Phase Ia Part C dose optimization: locally advanced or metastatic non-small cell lung cancer confirmed by histology or cytology, which is inoperable and not suitable for radical concurrent chemoradiotherapy, staged according to the 8th edition of the lung cancer TNM staging by the International Association for the Study of Lung Cancer and the American Joint Committee on Cancer. For non-squamous non-small cell lung cancer, absence of known driver gene mutation must be confirmed by histological or cytological specimens (including at least EGFR, ALK, ROS1, and cMET with written evidence provided); for squamous non-small cell lung cancer, if driver gene mutation testing was previously performed, absence of known driver gene mutation must be confirmed by histological or cytological specimens (including at least EGFR, ALK, ROS1, and cMET with written evidence provided);

Phase Ib study comprising 6 cohorts, among which:
**cohort A**: subjects with advanced colorectal cancer confirmed by histopathology, recognized by local standards as proficient mismatch repair (pMMR) or microsatellite stable (MSS);
**cohort B**: subjects with advanced melanoma confirmed by histopathology;
**cohort C**: subjects with advanced non-small cell lung cancer confirmed by histopathology;
**cohort D**: subjects with unresectable or metastatic hepatocellular carcinoma confirmed by histopathology;
**cohort E**: subjects with advanced renal cell carcinoma confirmed by histopathology;
**cohort F**: subjects with other advanced solid tumors or lymphoma confirmed by histology or cytology.

(4) Phase Ia and Ib cohorts A, C, E, and F: subjects with progressive disease after at least one line of standard therapy, or unsuitable for standard therapy due to intolerable toxicity or other reasons, or without standard therapy options (Note: patients who failed PD-1/PD-L1 therapy are permitted to enroll).

Phase Ia part C dose optimization: prior failure of at least one line of systemic antitumor therapy, including immunotherapy.

Phase Ib cohort B3, D: no prior systemic antitumor therapy for advanced melanoma or hepatocellular carcinoma.

Phase Ib cohort B1: no prior systemic immunotherapy for advanced melanoma.

Phase Ib cohort B2: prior failure of at least one line of systemic antitumor therapy, including immunotherapy.

(5) At least one measurable lesion according to RECIST v1.1 (solid tumor) or Lugano 2014 (lymphoma) criteria.

(6) Eastern Cooperative Oncology Group performance status score (ECOG PS) was 0 or 1.

(7) The survival time was expected ≥ 3 months.

(8) Female subjects of childbearing potential or male subjects with partners who are females of childbearing potential agree to strictly use effective contraception during the entire treatment period and for 6 months after treatment.

### 3.2 Exclusion Criteria

Subjects meeting any of the following criteria should not be enrolled in this study:
(1) Pregnant or lactating women, or women planning to become pregnant before, during, or within 6 months after the last administration of the investigational drug.
(2) Active or untreated central nervous system metastases (for example brain or leptomeningeal metastases) confirmed by imaging assessment during screening or prior imaging assessments. Patients with asymptomatic brain metastases may participate in this study. Patients with brain metastases treated and stable symptoms for ≥2 weeks, with no increase in brain metastases and no further enlargement after treatment, may also participate if all the following criteria are met:
   ◆ measurable lesions outside the central nervous system;
   ◆ no meningeal, midbrain, pons, medulla oblongata, or spinal cord metastases, nor multiple cerebellar metastases;
   ◆ no compression of the cerebral aqueduct, no compression of the third or fourth ventricles, and no spinal cord compression;
   ◆ hormone therapy discontinued for 14 days prior to the first dose of investigational drug.
(3) For lymphoma: patients with hematologic malignant tumors other than lymphoma.
(4) Any hematological abnormalities present at baseline * (within 7 days prior to the first administration of the investigational drug) (if lymphoma involves bone marrow, investigators may adjust enrollment criteria at their discretion):
   ◆ $hemoglobin < 90 g / L$
   ◆ $absolute neutrophil count \left(ANC\right) < 1.5 \times 109 / L$
   ◆ $platelet count < 100 \times 109 / L$
   ◆ $eosinophils > 1.5 \times ULN \left(Upper limit of normal, ULN\right)$
      *Throughout the protocol, "baseline" is defined as the last available observation prior to the first administration of the investigational drug. Subjects must not have received blood product transfusions (including packed red blood cells, apheresis platelets, cryoprecipitate, etc.), erythropoietin, or colony-stimulating factor support therapy within 7 days prior to blood sample collection.
(5) Any serum biochemical abnormalities present at baseline (within 7 days prior to first administration):
   ◆ total bilirubin >1.5×ULN (Upper limit of normal, ULN) (if conjugated bilirubin ≤ ULN concurrently, inclusion is permitted);
   ◆ aspartate aminotransferase (AST) or alanine aminotransferase (ALT) >3×ULN;
   ◆ serum creatinine >1.5×ULN or clearance of creatinine (CCr) <45 mL/min, calculated using the Cockcroft-Gault formula (using actual body weight);
   ◆ $albumin < 32 g / L .$
(6) Any coagulation parameter abnormalities present at baseline (within 7 days prior to first administration):
   ◆ international normalized ratio (INR) >1.5×ULN (if on stable dose anticoagulation therapy, >3×ULN);
   ◆ partial thromboplastin time (PTT) (or activated partial thromboplastin time, aPTT) >1.5×ULN (if on stable dose anticoagulation therapy, >3×ULN).
(7) History of active thrombosis, deep vein thrombosis or pulmonary embolism within 4 weeks prior to the first administration of the investigational drug, unless adequately treated and the investigator considers the condition stable.
(8) Active uncontrolled bleeding or known bleeding tendency.
(9) Clinically significant cardiovascular or cerebrovascular diseases, including:
   ◆ antiarrhythmic drug treatment required due to ventricular arrhythmia or other uncontrolled arrhythmias;
   ◆ severe conduction disorders (for example third-degree atrioventricular block);
   ◆ HR corrected QT interval (QTc interval, calculated using Fridericia's method) ≥480ms;
   ◆ uncontrolled arterial hypertension despite standard treatment (systolic blood pressure ≥160mmHg or diastolic blood pressure ≥100mmHg);
   ◆ history of myocarditis;
   ◆ left ventricular ejection fraction (LVEF) <50%;
   ◆ currently requiring treatment for congestive heart failure;
   ◆ cardiovascular disease classified as NYHA functional class III or IV according to the New York Heart Association (NYHA);
   ◆ history of acute coronary syndrome (including myocardial infarction and unstable angina), coronary angioplasty, or stent implantation within 6 months before the first administration of the investigational drug.
   ◆ history of cerebrovascular accident or transient ischemic attack within 6 months prior to the first administration of the investigational drug;
   ◆ known active epileptic seizures.
(10) Interstitial pneumonia, pulmonary fibrosis, pneumoconiosis, drug-related pneumonia, radiation pneumonia, etc., requiring steroid hormones or other treatment, as well as history of severe pulmonary function abnormality or other forms of restrictive lung disease.
(11) History of allergic constitution, asthma, or atopic dermatitis.
(12) Accompanying pleural, peritoneal, or pericardial effusion requiring repeated drainage or causing significant symptoms.
(13) Subject currently or recently (within 6 months) having major gastrointestinal diseases or conditions, including:
   ◆ history of inflammatory bowel disease;
   ◆ occurrence of grade ≥2 diarrhea within 2 weeks prior to the first administration of the investigational drug.
(14) Active autoimmune disease requiring systemic treatment (for example use of disease-modifying drugs, corticosteroids, or immunosuppressants) within 2 years before the first dosing. Substitution therapy (for example thyroxine, insulin, or physiological corticosteroids for adrenal or pituitary insufficiency) is not considered systemic treatment;
(15) known history of allogeneic organ transplantation and allogeneic hematopoietic stem cell transplantation.
(16) Subjects known or suspected to be allergic to the investigational drug and any excipients.
(17) Subjects with a prior significant toxicity history related to immune checkpoint inhibitor administration requiring permanent discontinuation of the therapy.
(18) Subjects with any prior antitumor treatment related unresolved >Grade 1 toxicity, except for persistent Grade 2 alopecia, peripheral neuropathy, hypomagnesemia, expected irreversible but drug-stabilized toxicities (for example hypothyroidism stabilized with substitution therapy, hypertension controlled below 160/100 mmHg with antihypertensive medication).
(19) Subjects not fully recovered from prior surgery or having undergone any major surgery within 4 weeks prior to first dosing of the investigational drug.
(20) Subjects with uncontrolled tumor-related pain or symptomatic hypercalcemia.
(21) Known HIV-positive, active hepatitis B, hepatitis C virus (HCV), or tuberculosis.
   ◆ Patients positive for hepatitis B surface antigen (HBsAg) or hepatitis B core antibody (HBcAb) require hepatitis B virus (HBV) DNA testing; enrollment is permitted if HBV DNA copies ≤2.5×10³ copies/ml or ≤500 IU/ml or below the detection limit. HBsAg(+) subjects should receive anti-HBV therapy during the entire investigational drug treatment period to prevent virus reactivation. Subjects who are anti-HBc(+), HBsAg(-), anti-HBs(-), and HBV viral load (-) do not require prophylactic anti-HBV treatment but require close monitoring for viral reactivation;
   ◆ subjects seropositive for hepatitis C virus but with HCV RNA testing negative or below detection limit are permitted to be enrolled;
   ◆ subjects treated for HCV with undetectable viral load results are permitted to be enrolled.
(22) Subjects with severe/active/uncontrolled infections, infections requiring systemic intravenous antibiotic treatment, or unexplained fever (>38°C) within 2 weeks before first dosing of the investigational drug.
(23) History or current evidence of any disease, treatment or laboratory abnormality that, in the investigator's judgment, may jeopardize subject safety, interfere with obtaining informed consent, affect subject compliance, or impact safety evaluation of the investigational drug.
(24) Subjects with mental illness, altered mental status, or drug abuse that impairs understanding of the informed consent process and/or completion of necessary study-related assessments.
(25) The investigator considers that the subject is unable to meet protocol requirements based on known or foreseeable reasons.
(26) Diagnosed with other malignant tumors within 5 years prior to the first administration, exceptions include treated basal cell carcinoma of the skin, squamous cell carcinoma of the skin and/or treated carcinoma in situ, as well as locally treated prostate cancer, papillary thyroid carcinoma, etc.
(27) Excluded drugs and other treatments (subjects must not receive any of the following treatments):
   (a) IL-2/IL-15 class cytokines. Use of IL-2/IL-15 as an adjuvant component of adoptive cell therapy or as immunomodulatory treatment for immunocompromised patients is permitted;
   (b) received any chemotherapy or small molecule targeted therapy within 2 weeks or 5 half-life periods (whichever is shorter) before the first administration of the investigational drug without delayed toxicity, except for the following situations:
      ◆ use of nitrosourea antitumor drugs and mitomycin C within 6 weeks before the first administration of the investigational drug.
   (c) use of any antibody therapy within 4 weeks before the first administration of the investigational drug;
   (d) participation in any clinical trial involving medical devices or other interventional therapies within 2 weeks before the first administration of the investigational drug;
   (e) received palliative radiotherapy within 2 weeks before the first administration of the investigational drug;
   (f) use of live vaccines for infectious disease prevention within 28 days before the first administration of the investigational drug;
   (g) received immunosuppressive or systemic steroid therapy (>10 mg/day prednisone equivalent) within 2 weeks before the first administration of the investigational drug.

### Exclusion criteria applicable to specific cohorts of phase 1b:

### cohorts A2, B1, B2, C2, D, E, and F:

(1) subjects with a history of significant toxicity associated with bevacizumab administration requiring permanent discontinuation of such treatment.

### Cohort C3:

(1) gastrointestinal diseases or surgical history that investigators consider may affect the absorption of the oral investigational drug anlotinib.
(2) Subjects with a history of significant toxicity associated with anlotinib-class drugs requiring permanent discontinuation of such treatment.

### 4. Study treatment and concomitant therapy

### 4.1 Treatment regimen

| | **Dose/amount** | **Administration frequency** | **Administration method** | **Treatment course/treatment cycle** |
|---|---|---|---|---|
| **Phase Ia Part A** | | | | |
| Accelerated titration phase | | | | |
| **2149** | 0.2 µg/kg, 2 µg/kg, 10 µg/kg, 30 µg/kg | Once weekly | Intravenous infusion | Three weeks per cycle; the first treatment cycle (21 days) is the DLT observation period |

| BOIN design escalation phase | | | | |
|---|---|---|---|---|
| **2149** | 100 µg/kg, 300 µg/kg, 600 µg/kg, 1 mg/kg | Once weekly | Intravenous infusion | Three weeks per cycle; the first dosing cycle (21 days) is the DLT observation period* |

| **Phase Ia Part B*** | | | | |
|---|---|---|---|---|
| **2149** | Safety dose determined in Phase Ia Part A, 1 mg Q2W/Q3W, 1.5 mg/kg Q3W, 2 mg/kg Q2W/Q3W, 3 mg/kg Q3W | Once weekly, Once every two weeks, or Once every three weeks | Intravenous infusion | Once weekly, once every three weeks, three weeks per cycle; once every two weeks, four weeks per cycle |

| **Phase Ib** | | | | |
|---|---|---|---|---|
| **2149** | RP2D | Determined based on Phase Ia results | Intravenous infusion | Determined based on Phase Ia results |
| Bevacizumab (cohorts A2, B1, B2, F) | 5 mg/kg or 7.5 mg/kg | Once every two weeks or once every three weeks | Intravenous injection | Four weeks per cycle or three weeks per cycle |
| Bevacizumab (cohorts B1, B2, C2, D, E, F) | 10 mg/kg or 15 mg/kg | Once every two weeks or once every three weeks | Intravenous injection | Four weeks per cycle or three weeks per cycle |
| Dacarbazine (cohort B3) | 850 mg/m2, d1 or 250 mg/m2, d1-5 | Once every four weeks or once every three weeks | Intravenous injection | Four weeks per cycle or three weeks per cycle |
| Anlotinib (cohort C3) | 12 mg | Once daily | Oral administration | Day 1 to 14/21 |

| | | | | |
|---|---|---|---|---|
| *: Specific dosage regimen is detailed in "Study Design" | | | | |

### 4.2 Formulation and administration of the investigational drug

The drug **2149** is a sterile injection packaged in vials, administered by intravenous infusion, available in specifications of 3mg (3ml)/vial and 12mg (12ml)/vial. The drug presents as a clear to microemulsion light, colorless to pale yellow liquid, with no foreign matters.

Administer **2149** following the above dosage regimen; dilute if necessary with 0.9% sodium chloride solution, maintaining the final concentration of **2149** between 0.02-0.80 mg/ml. If no other clinical indications exist, the infusion time should be controlled within 30-60 (+10) minutes. The prepared solution is recommended to be used immediately; if immediate use is not possible, it can be stored protected from light at 2~8°C for 8 hours, and at room temperature for 4 hours (including infusion time).

### 4.3 Concomitant therapy

### 4.2.1 Permitted concomitant therapies

Other therapies permitted during the trial include:
(1) medications judged by the investigator to be consistent with the protocol (for example concomitant therapies for treating disease-related symptoms and various AEs related to treatment).
(2) Subjects requiring long-term medication for underlying diseases for example hypertension and diabetes may continue such medications.
(3) Prophylactic treatment or therapy for infusion-related and/or allergic reactions
(4) Prophylactic use of growth factors is permitted based on clinical indications and in accordance with guidelines (except during the first cycle of dosing). Therapeutic use of growth factors during the study period is permitted.
(5) Palliative local radiotherapy may be applied to isolated lesions (excluding target lesions) during the study treatment.
(6) Supportive treatments to relieve tumor-related symptoms are permitted, for example bisphosphonate therapy for bone metastases.
(7) Hormone therapies for pre-treatment before investigational drug administration, allowing the use of immunosuppressive or systemic hormone therapy not exceeding 10 mg prednisone equivalent per day for reasons other than pre-treatment, as well as appetite-stimulating hormone therapy:
   - Hormone therapy for pre-treatment before investigational drug administration
   - Nasal, ophthalmic, inhalation, and topical glucocorticoid preparations
   - Stable hormone therapy for prostate cancer
   - Stable ovarian suppression hormone therapy, hormonal contraceptive therapy, or postmenopausal hormone replacement therapy
   - Intra-articular steroid injections
   - Low-dose hormone maintenance therapy for other diseases (for example asthma acute exacerbations, cerebral edema, etc.)
   - In subjects with stable disease or ongoing remission, high-dose corticosteroid treatment of acute comorbid diseases (for example immune-related adverse events) is permitted. Study drug treatment should be interrupted in these cases.
   - Subjects with neuroendocrine tumors receiving stable hormone therapy with somatostatin analogs

### 4.2.2 Prohibited concomitant therapies

Subjects are prohibited from receiving the following therapies during study treatment:
- Other systemic chemotherapy or biological therapy with antitumor effects.
- Immunotherapies not specified in this protocol.
- Investigational drugs other than the study drug.
- Immunosuppressive or immunostimulatory drugs, unless otherwise specified under "Allowed concomitant therapies."
- Any vaccine therapy for prevention of infectious diseases (for example human papillomavirus vaccine), except inactivated vaccines (for example influenza vaccine).
- Radiotherapy for tumor control (palliative radiotherapy is permitted provided it is not targeted at the target lesions, for example radiotherapy to relieve bone metastasis pain or brain metastasis symptoms).
- Drugs and other treatments explicitly excluded in the exclusion criteria.

It is important for investigators to review each drug (prescription and over the counter) that subjects received before study initiation and at each study visit.
- At each visit, any new medications received by the subject must be inquired about;
- to reduce the risk of adverse drug interactions, all measures must be taken to limit the number of concomitant drugs used to those truly necessary;
- during administration, drugs with hepatotoxicity (i.e., drugs warned for hepatotoxicity in the product label) should be avoided. Investigators are encouraged to review each potential hepatotoxic drug by searching the www.livertox.nih.gov website.
- Prohibited drugs listed in the exclusion criteria are not permitted.

### 5. Efficacy evaluation

### 5.1 Efficacy evaluation of subjects with solid tumors

Tumor assessment is performed using RECIST v1.1. Tumor imaging examinations generally include contrast-enhanced CT or MRI, with the chest, abdomen, and pelvis as the examination sites; if subjects are allergic to contrast agents for enhanced CT or MRI, non-contrast CT or MRI may be considered; additional CT or MRI scans of other sites may be performed if necessary. Patients suspected of having bone metastasis may undergo bone scan examination. The imaging techniques used for the same subject during the study should be consistent. Baseline assessments will be conducted within 28 days before C1D1; investigators may collect imaging results within 28 days prior to C1D1 for evaluation. Tumor imaging evaluations are performed every 6 weeks (±7 days) after administration of the investigational drug on C1D1. After 48 weeks, tumor evaluations are performed every 12 weeks (±14 days); until progressive disease, initiation of new antitumor treatment, subject's unwillingness to continue participation, loss to follow-up, death, or study termination, whichever occurs first. If treatment discontinuation occurs early for reasons other than progressive disease, subjects should still undergo imaging examinations according to the protocol-specified timing. Scheduled tumor imaging examinations should not be delayed due to treatment delays, holidays, or any other reasons. If progressive disease is suspected based on clinical or laboratory findings before the next scheduled assessment, an unscheduled evaluation should be performed. For subjects with the first recorded remission of disease [complete response (CR) or partial response (PR)], imaging assessment shall be performed 4-6 weeks (+7 days) later to confirm the remission. Thereafter, imaging assessments shall continue according to the originally established assessment schedule until imaging-confirmed progressive disease is recorded.

For subjects who discontinue treatment due to progressive disease, one tumor imaging examination shall be performed at the time of treatment discontinuation. If the time of the previous scan is within 4 weeks of treatment discontinuation, a scan at the time of treatment discontinuation is not mandatory. For subjects who stop treatment due to confirmed progressive disease, this scan is the last tumor imaging assessment required by the study. For subjects who discontinue treatment without evidence of progressive disease, efforts shall be made to continue monitoring disease status by tumor imaging in accordance with their tumor assessment schedule during treatment, until progressive disease, intolerable toxicity, subject unwillingness to continue participation, death, or other protocol-specified criteria requiring treatment discontinuation, whichever occurs first.

### 5.2 Efficacy evaluation of lymphoma subjects

### 5.2.1 Bone marrow examination

Except for the following circumstances, subjects are required to undergo bone marrow examination, including bone marrow smear cytology and bone marrow biopsy, at baseline assessment and subsequently continue bone marrow examination according to the schedule:
- Subjects had a bone marrow examination performed according to clinical practice guidelines within 60 days before first dosing of investigational drug.
- Subjects had a bone marrow examination after the last treatment, with results showing lymphoma infiltration of bone marrow.

For subjects without bone marrow infiltration by lymphoma at baseline, repeated bone marrow examination to confirm imaging CR criteria is not required. For subjects who were bone marrow positive at baseline, if imaging complete response occurs during treatment, bone marrow reexamination shall be performed within 2 weeks after imaging assessment. Whenever feasible, all bone marrow examinations should include unilateral aspiration and biopsy; if sufficient material is available, bone marrow flow cytometric analysis to evaluate lymphoma bone marrow infiltration may also be performed.

### 5.2.2 Imaging assessment

lymphoma subject imaging assessment is conducted according to the Lugano 2014 criteria. Baseline period should be within 28 days prior to priming study drug administration. Investigators shall identify at least one measurable lesion based on PET-CT and contrast-enhanced CT scans (covering whole body including neck, thorax, abdomen, and pelvis, contrast-enhanced MRI may be used for subjects allergic to CT contrast agents). Measurable lesions are defined as lymph node lesions assessed by CT/MRI with a longest diameter >15 mm and extranodal lesions with a longest diameter >10 mm. PET scans shall be performed at baseline, every 12 weeks (±7 days) after C1D1 dosing, at confirmation of CR or PD, whenever deemed necessary by investigators, and at treatment termination (±7 days). Contrast-enhanced CT scans shall be performed at baseline, every 6 weeks (±7 days) after C1D1 dosing, every 12 weeks (±14 days) after 48 weeks, whenever deemed necessary by investigators, until progressive disease, start of new antitumor treatment, subject withdrawal, loss to follow-up, death, or study termination, whichever occurs first.

### 6. pharmacokinetics

3.5 ml whole blood was collected, serum was separated, aliquoted and frozen for PK analysis. If necessary, PK samples will be used for immunogenicity testing. During the clinical study, the sponsor may adjust, reduce or stop PK sampling and/or testing based on clinical development needs, overall development strategy, and subject protection considerations.

### 7. Immunogenicity analysis

5 ml whole blood was collected using coagulation-promoting vacuum blood collection tubes, serum was separated, aliquoted and frozen for molecular **2149** ADA and/or NAb analysis. If necessary, immunogenicity samples would be used for molecular **2149** concentration testing. During the clinical study, the sponsor may reduce or stop sampling and/or testing based on clinical development needs, overall development strategy, and subject protection considerations.

### 8. Pharmacodynamics analysis

Approximately 4 mL whole blood was collected for receptor occupancy and immune cell subtype analysis. During the clinical study, the sponsor may reduce or stop sampling and/or testing based on clinical development needs, overall development strategy, and subject protection considerations.

### 9. Safety evaluation

### 9.1 Laboratory tests

### 9.1.1 Routine laboratory safety evaluation

| | |
|---|---|
| **Hematology** | Red blood cell count (RBC), HGB, white blood cell count (WBC), platelet count (PLT), white blood cell differential [lymphocyte count (LYM), ANC, eosinophil count (EOS), basophil count (BASO)] |
| **Blood biochemistry** | Liver function [TBIL, ALT, AST, γ-glutamyl transferase (γ-GT), alkaline phosphatase (ALP), albumin (ALB), total protein (TP), lactic dehydrogenase (LDH)], kidney function [urea (Urea), Cr], blood electrolytes (Na, K, Cl, Mg, Ca, P), fasting blood glucose (FBG), C-reactive protein and ferritin |
| **Myocardial enzyme spectrum and troponin** | Creatine Kinase (Creatine Kinase, CK), Creatine Kinase Isoenzyme (CK-MB), Troponin (Troponin T [cTnT] or Troponin I [cTnI]), BNP |
| **Urinalysis** | pH, Urine Specific Gravity, Urine Glucose, Urine Protein, Urine Red Blood Cells, Urine White Blood Cells, Urine Bilirubin, Urine Urobilinogen, and Occult Blood |
| **Viral Serology** | HBsAg, HBsAb, HBcAb, HBeAb, HBeAg, HCV Antibody, HIV Antibody, HBV DNA, HCV RNA |
| **Coagulation Function Indicators** | Fibrinogen, D-dimer, Prothrombin Time (PT), Activated Partial Thromboplastin Time (APTT), International Normalized Ratio (INR) |
| **Thyroid Function** | TSH, FT3, and FT4 |
| **Stool Routine** | Fecal Occult Blood Test |
| **Cytokines** | IFN-γ, TNF-α, IL-8, IL-6, IL-10 |

HBcAb: Hepatitis B Core Antibody; HBeAb: Hepatitis B E Antibody; HBeAg: Hepatitis B E Antigen; HBsAb: Hepatitis B Surface Antibody; HBsAg: Hepatitis B Surface Antigen; HBV: Hepatitis B Virus; HCV: Hepatitis C Virus; HGB: Hemoglobin content; HIV: Human Immunodeficiency Virus. TBIL: Total Serum Bilirubin; DBIL: Direct Bilirubin; IBIL: Indirect Bilirubin; ALT: Alanine Aminotransferase; AST: Aspartate Aminotransferase; BNP: B-type Natriuretic Peptide; TSH: Thyroid Stimulating Hormone; FT3: Free Triiodothyronine; FT4: Free Thyroxine; IFN-γ: Interferon gamma; TNF-α: Tumor Necrosis Factor alpha; IL-8: Interleukin 8; IL-6: Interleukin 6; IL-10: Interleukin 10.

### 9.1.2 Liver Function Testing

Researchers need to regularly check patients' liver enzyme levels. If
(1) in patients with normal baseline levels, AST or ALT increases to >3×ULN;
(2) in patients with abnormal baseline levels, if baseline AST or ALT ≤2.5×ULN, it increases to ≥3 times the baseline level after medication; or if baseline level 2.5×ULN<AST or ALT<5×ULN, it increases to ≥2 times the baseline level after medication;
(3) total bilirubin elevates to ≥2×ULN in the absence of cholestasis

Reexamination of four common serum measurements (ALT, AST, ALP, and TBL) within 48 to 72 hours to confirm abnormalities and determine whether they are elevated or decreased. If the reexamination results are consistent with the previous ones and meet (1)+(3) or (2)+(3), it is then defined as a potential Hy's law case. A liver toxicity questionnaire must be completed in the appendix of the CRF to collect further information to determine whether it is a potential Hy's law case or a true Hy's law case.

**Hy's Law (HL)**: Besides the investigational drug, no other reason can reasonably explain AST or ALT ≥3×ULN combined with total bilirubin ≥2×ULN, including reasons for example ALP elevation caused by cholestasis, viral hepatitis, other drugs, etc.

### 9.2 Clinical examination

### 9.2.1 Physical examination

A complete physical examination includes: general condition, respiratory system, cardiovascular system, abdomen, skin, head and neck (including ears, eyes, nose, and throat), lymph nodes, thyroid, musculoskeletal system (including spine and limbs), genital/anus (if applicable), and neurological assessment.

### 9.2.2 Vital signs examination

Vital signs examination will be conducted according to the visit schedule description. Vital signs include temperature, pulse, respiratory rate, and blood pressure.

### 9.2.3 Lead Electrocardiogram (ECG) Examination

Resting 12-lead ECG will be analyzed at the local laboratory according to the visit schedule.

Subjects should participate in the examination in a resting state. All 12-lead ECGs should be recorded while the subject is resting in a supine position. Further ECG examinations will be conducted if clinically necessary, for example upon occurrence of cardiac-related adverse events. The investigator completes the ECG assessment on the day of examination and records the assessment results on the electrocardiogram. The same assessment method should be used throughout the study duration.

The investigator should evaluate all ECGs according to categories of clinically significant abnormalities/non-clinically significant abnormalities. If the result is a clinically significant abnormality, the investigator shall record the result as an AE in the eCRF.

### 9.2.4 Assessments and examinations during and after infusion

During investigational drug infusion, carefully observe patients for any evidence of treatment-related AEs by collecting pulse, blood pressure, respiratory rate, and body temperature for at least 2 hours after the first administration of the investigational drug and at least 1 hour after subsequent infusions. Collect pulse oximetry (SaO2) within at least 2 hours after the first administration of the investigational drug and at least 1 hour after subsequent infusions.

### 9.3 Safety evaluation committee

The safety evaluation committee consists of the sponsor and investigators. Before completion of the dose escalation phase of part Ia, after DLT observation in the previous dose group is completed, the safety evaluation committee evaluates and decides whether treatment may proceed to the next dose group. In part B of phase Ia, unexpected safety events or intolerable safety signals may also trigger discussion by the safety evaluation committee. If specific safety concerns are identified, external experts may be invited to jointly form an independent safety evaluation committee with the sponsor to evaluate, judge, and provide guidance on safety issues.

### 10. Tissue biomarker analysis

With ethics committee approval, tumor tissue slices (including archived or freshly collected) will be collected according to the visit schedule for biomarker analysis. 10 tumor tissue slices were collected for PD-L1 expression level and tumor microenvironment detection. If approved by the ethics committee, with subject consent and investigator's judgment that conditions allow, subjects should provide 10 tumor tissue slices prepared from fresh tumor biopsy or puncture specimens (or tumor tissue specimens obtained after neoadjuvant therapy surgery) as much as possible when objective response occurs or progressive disease happens, for PD-L1 expression level and tumor microenvironment detection. If testing failure occurs, and residual slices are available at the clinical center, additional slices may be requested.

### 11. Results

### Solid tumor

Patients eligible who have failed or are intolerant to standard therapy were enrolled. Intravenous administration of **2149** was performed with accelerated titration (0.2, 2, 10, 30 µg/kg QW) and Bayesian optimal interval (BOIN) design (0.1, 0.3, 0.6, 1 mg/kg QW). Further dose optimization was evaluated at 0.3, 0.6, 1 mg/kg Q2W and 0.6, 1, 1.5, 2, 3 mg/kg Q3W. The endpoints were safety and efficacy according to RECIST v1.1.

Among 326 enrolled patients (male: 60.7%, ECOG PS 1: 63.3%), 168 patients received a dose of 1 mg/kg, 99 patients received a dose below 1 mg/kg, and 59 patients received a dose above 1 mg/kg. A total of 272 patients (≥0.1 mg/kg, including 77 non-small cell lung cancer [NSCLC], 67 melanoma, 79 colorectal cancer, and 49 other tumors) underwent at least one post-baseline tumor assessment. The overall ORR was 17.6% (95% CI: 13.3-22.7), and DCR was 57.0% (95% CI: 50.9-62.9). The ORR for IO-treated patients (n=185) was 17.8% (95% CI: 12.6-24.1), and the DCR was 62.2% (95% CI: 54.8-69.2). In NSCLC (n=77, prior tx ≥2L: 79.2%, including 8 with EGFR mutation), the ORR was 22.1% (95% CI: 13.4-33.0), and DCR was 67.5% (95% CI: 55.9-77.8). Among them, IO-treated patients (n=72) had an ORR of 22.2% (95% CI: 13.3-33.6) and a DCR of 68.1% (95% CI: 56.0-78.6). In squamous NSCLC (n=36, prior tx ≥2L: 69.4%, 35 IO-treated, 1 IHC positive for EGFR), ORR was 30.6% (95% CI: 16.3-48.1), DCR was 75.0% (95% CI: 57.8-87.9), median PFS was 5.5 months (95% CI: 4.0-NC), with 14 patients (38.9%) experiencing events. All squamous NSCLC patients (3 mg/kg Q3W) experienced partial response (n=4). In mucosal melanoma (n=25), ORR was 32.0% (95% CI: 14.9-53.5), DCR was 68.0% (95% CI: 46.5-85.1).

The 1 mg/kg Q2W dose group and 3 mg/kg Q3W dose group enrolled 145 and 7 subjects, respectively. All subjects received a priming dose treatment of 100 µg. Overall, 1 mg/kg Q2W and 3 mg/kg Q3W showed manageable safety profiles. The TEAE incidence in the 1 mg/kg Q2W dose group was 90.3%, with grade 3 or higher TEAE incidence of 21.4%, and grade 3 or higher treatment-related adverse event (TRAE) incidence related to 2149 was 17.9%. The treatment emergent adverse event (TEAE) incidence in the 3 mg/kg Q3W dose group was 71.4%, with grade 3 or higher treatment emergent adverse event (TEAE) incidence of 28.6%, and grade 3 or higher TRAE incidence related to 2149 was 14.3%.

Molecule 2149 exhibits favorable efficacy in patients with advanced malignant tumors. 2149 demonstrates significant clinical benefit in patients with advanced melanoma who have failed or are intolerant to standard therapy. As of January 11, 2024, a total of 57 subjects received at least one tumor assessment, with 16 subjects achieving a best overall response of partial response (PR), resulting in an overall ORR of 28.1%.

In the ongoing study, a total of 347 patients with advanced solid tumors failed or intolerant to standard therapy were enrolled, including 100 patients with non-small cell lung cancer (NSCLC), 89 patients with melanoma, 102 patients with colorectal cancer (CRC), and 56 patients with other tumors. Baseline patient characteristics are as follows: median age 58.0; ECOG PS 1: 64.5%; prior lines of systemic therapy ≥2: 81.8%; prior immunotherapy: 67.7%; liver metastasis: 33.1%; lung metastasis: 57.3%; bone metastasis: 19.6%; brain metastasis: 3.2%. Intravenous administration of molecule **2149** was performed following accelerated titration (0.2, 2, 10, 30 µg/kg QW) and BOIN design (100, 300, 600, 1000 µg/kg QW), with further dose optimization evaluated at 300, 600, 1000 µg/kg Q2W and 600, 1000, 1500, 2000, 3000 µg/kg Q3W (see FIG. 2). In the dose groups ≥600 µg/kg, patients received a priming dose of 100 µg/kg 7 days before the first planned full dose. The endpoints were safety and efficacy according to RECIST v1.1. Overall efficacy is shown in Table 1 below.

**Table 1. Overall efficacy in patients treated at a dose level ≥0.1 mg/kg**

| Investigator-assessed response | | All patients (N=300)*^{#} | Patients treated with IO therapy (N=204) | 1 mg/kg Q2W (N=151) | 3 mg/kg Q3W (N=15) |
|---|---|---|---|---|---|
| Best overall response, n (%) | | | | | |
| | CR (complete response) | 3 (1.0) | 1 (0.5) | 2 (1.3) | 0 |
| | PR (partial response) | 49 (16.3) | 35 (17.2) | 24 (15.9) | 7 (46.7) |
| | SD (stable disease) | 117 (39.0) | 90 (44.1) | 62 (41.1) | 5 (33.3) |
| | PD (progressive disease) | 128 (42.7) | 76 (37.3) | 61 (40.4) | 3 (20.0) |
| | Not evaluable | 3 (1.0) | 2 (1.0) | 2 (1.3) | 0 |
| ORR (objective response rate), % (95% CI) | | 17.3 (13.2-22.1) | 17.6 (12.7-23.6) | 17.2 (11.6-24.2) | 46.7 (21.3-73.4) |
| DCR (disease control rate), % (95% CI) | | 56.3 (50.5-62.0) | 61.8 (54.7-68.5) | 58.3 (50.0-66.2) | 80.0 (51.9-95.7) |

| | | | | | |
|---|---|---|---|---|---|
| *Patients underwent at least 1 tumor assessment. #Including 81 NSCLC patients, 77 melanoma patients, 98 CRC patients, and 44 patients with other tumors. | | | | | |

Durable DoR (duration of response) was observed. Among 52 responders (CR and PR), 38 had no progressive disease, and 9 patients maintained tumor response even after end of treatment (EOT). Median DoR (95% CI) was 8.1 months (4.2, NC).

**Table 2. Safety evaluation**

| n, (%) | | Total patients (N=347) | 1 mg/kg Q2W (N=162) | 3 mg/kg Q3W (N=38) |
|---|---|---|---|---|
| Any TEAEs¹ | | 326 (93.9) | 161 (99.4) | 22 (57.9) |
| | Grade 3-5 TEAEs | 111 (32.0) | 47 (29.0) | 6 (15.8) |
| Any TRAEs | | 309 (89.0) | 157 (96.9) | 19 (50.0) |
| | Grade 3-5 TRAEs | 83 (23.9) | 39 (24.1) | 5 (13.2) |
| Any TRSAE² | | 79 (22.8) | 45 (27.8) | 3 (7.9) |
| TRAEs leading to treatment discontinuation | | 11 (3.2) | 7 (4.3) | 0 |
| TEAEs leading to death | | 4 (1.2) | 1 (0.6) | 1 (2.6) |
| TRAEs leading to death | | 2 (0.6) | 1 (0.6) | 0 |
| Any irAEs | | 126 (36.3) | 67 (41.4) | 6 (15.8) |
| | Grade 3-5 irAEs | 36 (10.4) | 22 (13.6) | 0 |

| | | | | |
|---|---|---|---|---|
| ¹ Graded according to the Common Terminology Criteria for adverse event (CTCAE) version 5.0. ² TRSAE: treatment-related serious adverse event. | | | | |

Conclusion: **2149** was well tolerated in all patients, and its efficacy in solid tumors, especially in advanced NSCLC and melanoma, is encouraging. Moreover, molecular **2149** demonstrated good safety in patients.

### Intestinal cancer

The prognosis of advanced colorectal cancer (CRC) is poor, and available treatment options are limited. This study enrolled eligible patients with failed or intolerant to standard therapy locally advanced or metastatic colorectal cancer (CRC) who received intravenous injection of **2149** at dose levels of 0.1-3 mg/kg QW/Q2W/Q3W (see FIG. 3). In the dose groups ≥600 µg/kg, patients received a priming dose of 100 µg/kg 7 days before the first planned full dose.

Among the 68 enrolled patients (male: 55.9%, median age: 55.5 years, ECOG PS 1: 50.0%, liver metastasis: 61.8%; prior treatments ≥3 lines: 76.5%; prior immunotherapy: 27.9%), 24 patients received 0.6 mg/kg Q2W, and 20 patients received 1 mg/kg Q2W. 57 (83.8%) patients were microsatellite stable (MSS)/proficient mismatch repair (pMMR), while the microsatellite status of the remaining 11 (16.2%) patients was unknown. The median follow-up time was 5.3 months (95% CI: 4.4-6.9). 65 patients (95.6%) reported treatment emergent adverse events (TEAEs), among whom 22 patients (32.4%) reported TEAEs of grade ≥3. Common TEAEs (≥20%) included arthralgia (35.3%), anemia (32.4%), pyrexia (22.1%), and decreased blood albumin (20.6%). 62 patients (91.2%) reported treatment-related adverse events (TRAEs), with 16 patients (23.5%) reporting TRAEs of Grade ≥3. 22 patients (32.4%) reported immune-related adverse events (irAE), of whom 4 patients (5.9%) reported irAEs of Grade ≥3. 12 patients (17.6%) reported serious TRAEs. 25 patients (36.8%) reported TRAEs leading to treatment interruption, and 2 patients (2.9%) reported TRAEs leading to treatment discontinuation. No TRAEs leading to death were reported. Patients who had undergone at least 1 post-baseline tumor assessment were included in the efficacy evaluation group. Among all evaluable patients (n=63), the overall ORR was 12.7% (95% CI: 5.6-23.5). Among patients with liver metastasis (n=38), the ORR was 13.2% (95% CI: 4.4-28.1). Among patients with PD-L1 CPS≥1 (n=13), the ORR was 30.8% (95% CI: 9.1-61.4), and the DCR was 76.9% (95% CI: 46.2-95.0). A prospective cohort of CRC patients with PD-L1 CPS≥1 has been initiated.

Conclusion: **2149** showed favorable efficacy and controllable safety in patients with advanced colorectal cancer. Preliminary efficacy of **2149** was observed, particularly in patients with PD-L1 CPS≥1.

In the study of molecule **2149** + bevacizumab, eligible patients with locally advanced or metastatic colorectal cancer (CRC) who failed or intolerant to standard therapy were enrolled. The baseline characteristics of the patients are shown in Table 3 below. Patients were administered by intravenous injection according to the following regimens, respectively: 1.5 mg/kg Q3W **2149** + 7.5 mg/kg Q3W bevacizumab (Beva), 2 mg/kg Q3W **2149** + 7.5 mg/kg Q3W bevacizumab (Beva), 3 mg/kg Q3W **2149** + 7.5 mg/kg Q3W bevacizumab (Beva) (see FIG. 3, Phase Ia Part B combination). In the dose groups ≥600 µg/kg, patients received a priming dose of 100 µg/kg 7 days before the first planned full dose. The endpoints were safety and efficacy according to RECIST v1.1. The median follow-up time was 3.8 months (range: 0-125). The preliminary treatment effects are shown in Table 4 below.

**Table 3. Baseline characteristics of CRC patients enrolled in the study of molecule 2149 + bevacizumab**

| | 2 mg/kg + Beva | 3 mg/kg + Beva | **Total** |
|---|---|---|---|
| Number of enrolled patients | 7 | 19 | **26** |
| Age ≤65, n (%) | 7 (100) | 12 (63.2) | **19 (73)** |
| Male, n (%) | 6 (85.7) | 10 (52.6) | **16 (61.5)** |
| MSS/pMMR, n (%) | 7 (100) | 16 (84.2) | **23 (88.5)** |
| MSI/MMR status unknown, n (%) | 0 | 3(15.8) | 3(11.5) |
| RAS exon 2/3/4 mutation, n (%) | 7 (100) | 14(73.7) | **21(80.8)** |
| RAS wild type, n (%) | 0 | 5(26.3) | 5(19.2) |
| Liver metastasis, n (%) | 6 (85.7) | 10 (52.6) | **16 (61.5)** |
| Previous therapy lines ≥ 2, n (%) | 6 (85.7) | 8(42.1) | **14(53.8)** |
| Prior IO therapy, n (%) | 1(14.3) | 5(26.3) | **6(23.1)** |

**Table 4. Preliminary treatment effect of 2149+bevacizumab combination in CRC patients**

| | 2 mg/kg + Beva | 3 mg/kg + Beva | Total | No liver metastasis |
|---|---|---|---|---|
| Patients with at least 1TA** | 7 | 11 | 18 | 5 |
| PR (partial response) | 0 | 3 | 3 | 3 |
| SD (stable disease) | 2 | 7* | 9 | 2 |
| PD (progressive disease) | 4 | 1 | 5 | 0 |
| NE (not evaluable) | 1 | 0 | 1 | 0 |
| ORR (objective response rate) | 0% | 27.3% | 16.7% | 60% |
| DCR (disease control rate) | 28.6% | 90.9% | 66.7% | 100% |

| | | | | |
|---|---|---|---|---|
| * Among patients with SD, 5 patients had tumor shrinkage >20%. ** indicates patients experiencing at least one treatment emergent adverse event (Treatment-emergent Adverse Events). | | | | |

It can be seen that molecule **2149** in combination with bevacizumab shows encouraging efficacy and controllable safety in patients with advanced CRC, especially in those without liver metastasis. Specifically, molecule **2149** at a 3 mg/kg dose shows superior therapeutic effect compared to 2149 monotherapy.

### Melanoma

Eligible advanced melanoma patients who failed or intolerant to standard therapy were enrolled and received intravenous injection of **2149** at dose levels of 0.1-3 mg/kg QW/Q2W/Q3W (see FIG. 2). In the dose groups ≥600 µg/kg, patients received a priming dose of 100 µg/kg 7 days before the first planned full dose.

Among the 67 enrolled patients (female: 55.2%, median age: 59.0 years, ECOG PS 1: 74.6%, prior lines of therapy ≥2: 59.7%, prior IO: 89.6%), there were 17 cases of cutaneous melanoma, 22 cases of acral melanoma, 25 cases of mucosal melanoma, and 3 cases of unknown primary melanoma. The median treatment duration was 12.0 weeks (range: 2.0-43.6), with 38 cases (56.7%) still under treatment. All patients were included in the safety analysis. Treatment-emergent adverse events (TEAEs) occurred in 63 patients (94.0%). TEAEs of grade ≥3 occurred in 16 patients (23.9%), and TRAEs of grade ≥3 occurred in 12 patients (17.9%). Common TEAEs (≥20%) were arthralgia (34.3%), hyperthyroidism (29.9%), and anemia (25.4%). 1 patient (1.5%) experienced a TEAE leading to treatment interruption. No patient experienced TEAEs leading to death. Patients with at least 1 post-baseline tumor assessment were included in the efficacy-evaluable set.

Among all evaluable patients (n=57), the best overall response was partial response (PR) in 16 patients, stable disease (SD) in 25 patients, and progressive disease (PD) in 16 patients. The overall ORR was 28.1% (95% CI: 17.0-41.5%), and the DCR was 71.9% (95% CI: 58.5-83.0). Among patients with prior IO (n=52), the ORR was 21.2% (95% CI: 11.1-34.7), and the DCR was 67.3% (95% CI: 52.9-79.7). Among patients with prior IO receiving 1 mg/kg Q2W (n=25), the ORR was 32.0% (95% CI: 14.9-53.5), and the DCR was 80.0% (95% CI: 59.3-93.2). Biomarker analysis of baseline tumor regions showed that CD8 T cell infiltration (measured by cell positivity and density) was significantly higher in PR/SD patients than in PD patients (p<0.05).

This study is ongoing. As of April 16, 2024, this study further provided efficacy data of molecule 2149 in patients with melanoma; see Tables 5-7.

**Table 5. Efficacy of molecule 2149 (1 mg/kg Q2W) in patients with immunotherapy-treated melanoma**

| | | Cutaneous melanoma (N=19) | Acral melanoma (N=7) | Mucosal melanoma (N=11) | Total* (N=37) |
|---|---|---|---|---|---|
| Previous therapy lines ≥ 2, n (%) | | 13 (68.4) | 7 (100) | 7 (63.6) | 27 (73.0) |
| Median prior IO therapy duration, months | | 5.6 | 11.3 | 5.2 | 5.8 |
| Best overall response, n (%) | | | | | |
| | CR (complete response) | 1 (5.3) | 0 | 0 | 1 (2.7) |
| | PR (partial response) | 5 (26.3) | 3 (42.9) | 2 (18.2) | 10 (27.0) |
| | SD (stable disease) | 7 (36.8) | 2 (28.6) | 7 (63.6) | 16 (43.2) |
| | PD (progressive disease) | 6 (31.6) | 2 (28.6) | 1 (9.1) | 9 (24.3) |
| | Not evaluable | 0 | 0 | 1 (9.1) | 1 (2.7) |
| ORR (objective response rate), % (95% CI) | | 31.6 (12.6-56.6) | 42.9 (9.9-81.6) | 18.2 (2.3-51.8) | 29.7 (15.9-47.0) |
| DCR (disease control rate), % (95% CI) | | 68.4 (43.4-87.4) | 71.4 (29.0-96.3) | 81.8 (48.2-97.7) | 73.0 (55.9-86.2) |

| | | | | | |
|---|---|---|---|---|---|
| * Patients underwent at least one post-baseline tumor assessment. | | | | | |

Sustained responses were observed. Among the 11 responders (CR and PR), 9 had confirmed PR, 1 was pending confirmation, and 1 was lost to follow-up. Median DoR (duration of response, months) (95% CI) was not computable (NC) because at the data cutoff, patients were still in progression free survival, thus no definitive longest progression free survival was identified.

Median PFS of molecule **2149** in various melanoma is shown in Table 4, with a median follow-up of 4.2 months.

**Table 6.**

| | | **Cutaneous melanoma** | **Acral melanoma** | **Mucosal melanoma** | **Total** |
|---|---|---|---|---|---|
| Number, N | | 21 | 8 | 11 | 40 |
| | Events, n (%) | 10 (47.6) | 4 (50.0) | 6 (54.5) | 20 (50.0) |
| Median PFS, months (95% CI) | | 3.0 (1.5, NC) | 5.6 (1.4, 7.6) | 4.2 (2.7, NC) | 4.2 (2.8, NC) |

**Table 7. Efficacy of molecule 2149 (≥0.3 mg/kg) in mucosal melanoma patients who have not received immunotherapy (IO)**

| | 300 ug/kg QW (N=1) | 600 ug/kg Q2W (N=1) | 1 mg/kg Q2W (N=5) | 1.5 mg/kg Q3W (N=1) | Total (N=8)* |
|---|---|---|---|---|---|
| Best overall response, n (%) | | | | | |
| CR | 0 | 0 | 0 | 1 (100) | 1 (12.5) |
| PR | 1 (100) | 1 (100) | 3 (60.0) | 0 | 5 (62.5) |
| SD | 0 | 0 | 2 (40.0) | 0 | 2 (25.0) |
| ORR, % | 100 | 100 | 60.0 | 100 | 75.0 |

| | | | | | |
|---|---|---|---|---|---|
| *Patients underwent at least 1 post-baseline tumor assessment. | | | | | |

Among 6 respondents (CR and PR), 5 hand confirmed PR and 1 was pending confirmation.

Conclusion: In patients with advanced melanoma, **2149** demonstrates encouraging efficacy across different tumor subtypes and in patients with prior IO, with an acceptable and manageable safety profile.

### Lung cancer

This study enrolled qualified advanced non-small cell lung cancer (NSCLC) patients who had failed standard therapy. Molecule **2149** was administered by intravenous injection as follows: 2-600 µg/kg QW, 0.3/0.6/1 mg/kg Q2W, or 1.5/2/3 mg/kg Q3W. In the dose groups ≥600 µg/kg, patients received a priming dose of 100 µg/kg 7 days before the first planned full dose. Endpoints included safety, objective response rate (ORR), disease control rate (DCR), duration of response (DoR), and progression free survival (PFS) according to RECIST v1.1. The median duration of exposure to molecule 2149 was 10 weeks (range: 2.0-56.7). The results are as follows.

**Table 8. Effect of molecule 2149 on NSCLC**

| Patients with at least 1 tumor assessment | | NSCLC | sqNSCLC | | |
|---|---|---|---|---|---|
| | | All tested doses (N=125) | 1/1.5 mg/kg (N=27) | 3 mg/kg (N=29) | 3 mg/kg (N=18**) |
| Best overall response, n (%) | | | | | |
| | PR (partial response) | 26 | 6 | 10 | 9 |
| | SD (stable disease) | 67 | 13 | 16 | 7 |
| | PD (progressive disease) | 30 | 8 | 2 | 2 |
| | NE (not evaluable) | 2 | 0 | 1 | 0 |
| ORR, % (95% CI) | | 20.8% (14.1, 29.0) | 22.2% (8.6, 42.3) | 34.5% (17.9, 54.3) | 50.0% (26.0, 74.0) |
| DCR, % (95% CI) | | 74.4% (65.8, 81.8) | 70.4% (49.8, 86.2) | 89.7% (72.6, 97.8) | 88.9% (65.3, 98.6) |

| | | | | | |
|---|---|---|---|---|---|
| **Patients underwent at least 1 tumor assessment and were followed up for at least 12 weeks or until study end. | | | | | |

The median PFS (progression free survival) of molecule **2149** (1 mg/kg and 1.5 mg/kg) in sqNSCLC (n=28) was 5.5 months (95% CI: 1.5, 8.3), with a median follow-up of 7.5 months (95% CI: 7.0-11.0).

The results of best overall response of PD-L1 expression in sqNSCLC for molecule **2149** (1 mg/kg, 1.5 mg/kg, and 3 mg/kg) (n=56) are shown in Table 9. It can be seen that responses were similar in PD-L1 TPS <1% and TPS ≥1%.

**Table 9.**

| Patients with at least 1 tumor assessment | | sqNSCLC (N=56, 1/1.5/3 mg/kg) | |
|---|---|---|---|
| | | TPS <1% (N=22) | TPS ≥1% (N=22) |
| Best overall response, n (%) | | | |
| | PR (partial response)* | 8 | 7 |
| | SD (stable disease) | 13 | 8 |
| | PD (progressive disease) | 1 | 6 |
| | NE (not evaluable) | 0 | 1 |
| ORR, % (95% CI) | | 36.4% (17.2, 59.3) | 31.8% (13.9, 54.9) |
| DCR, % (95% CI) | | 95.5% (77.2, 99.9) | 68.2% (45.1, 86.1) |

**Table 10. Safety evaluation**

| **n (%)** | | **1/1.5 mg/kg (N=62)** | **3 mg/kg (N=57)** | **Total (N=134)** |
|---|---|---|---|---|
| Treatment-emergent adverse events (TEAEs) | | 61 (98.4) | 56 (98.2) | 132 (98.5) |
| | TEAEs of grade ≥3 | 18 (29.0) | 17 (29.8) | 43 (32.1) |
| Treatment-related adverse events (TRAEs) | | 58 (93.5) | 54 (94.7) | 124 (92.5) |
| | TRAEs of grade ≥3 | 11 (17.7) | 10 (17.5) | 27 (20.1) |
| TEAEs leading to death | | 0 | 1 (1.8) | 2 (1.5) |
| TRAEs leading to death | | 0 | 0 | 1 (0.7) |
| TEAEs leading to treatment discontinuation | | 4 (6.5) | 3 (5.3) | 8 (6.0) |
| TRAEs leading to treatment discontinuation | | 4 (6.5) | 3 (5.3) | 8 (6.0) |
| TEAEs leading to treatment interruption | | 33 (53.2) | 18 (31.6) | 61 (45.5) |
| TRAEs leading to treatment interruption | | 28 (45.2) | 12 (21.1) | 49 (36.6) |
| Immune-mediated adverse events (irAEs) | | 20 (32.3) | 21 (36.8) | 46 (34.3) |
| | irAEs of grade ≥ 3 | 5 (8.1) | 3 (5.3) | 11 (8.2) |

| | | | | |
|---|---|---|---|---|
| Note: 1 patient at the 1/1.5 mg/kg dose level and all 57 patients at the 3 mg/kg dose level received a priming dose of 0.1 mg/kg 7 days before the first anticipated full-dose treatment. | | | | |

### Results:

Molecule 2149 was well tolerated and demonstrated controllable safety in sqNSCLC. No new safety signals were observed. At all dose levels: TRAEs of ≥G3 were 20.1%, with TRAEs leading to treatment discontinuation at 6.0%. At the 3 mg/kg Q3W dose, TRAEs of ≥G3 were 17.5%, with TRAEs leading to treatment discontinuation at 5.3%.

Molecule 2149 demonstrated encouraging efficacy in patients with advanced NSCLC. In all NSCLC patients, ORR was 20.8%, and DCR was 74.4%. In sqNSCLC patients treated with 1 or 1.5 mg/kg, ORR was 22.2%, DCR was 70.4%, and median PFS was 5.5 months. In sqNSCLC patients treated with 3 mg/kg, ORR was 34.5%, and DCR was 89.7%.

### Example 2. Phase II study of safety, tolerability, and efficacy of PD-1/IL-2R bispecific antibody fusion protein 2149 in subjects with advanced melanoma

In preliminary clinical studies, **2149** showed encouraging activity in subjects with melanoma. Among 8 evaluable subjects, 2 subjects with mucosal melanoma and 1 subject with cutaneous melanoma achieved partial response at the first tumor assessment. The subject with cutaneous melanoma had previously been treated with pembrolizumab and experienced treatment failure, and the overall safety and tolerability were good. The study population consisted of subjects who had previously failed at least one line of systemic standard therapy to maximize treatment benefit.

Based on the existing safety and efficacy data of, the controlled safety profile and positive efficacy signals observed in clinical studies of **2149** suggest that the risk of treatment with **2149** in subjects with advanced melanoma is controllable and the benefits are expected.

### 1. Study objectives and endpoints

| **Study objectives** | **Study endpoints** |
|---|---|
| Primary objectives | Primary endpoints |
| • To evaluate the safety and tolerability of **2149** in subjects with advanced melanoma. | Safety evaluation: |
| | • Incidence and severity of all adverse events (AE), treatment emergent adverse events (TEAE), immune-related adverse events (irAE) and serious adverse events (SAE) (per CTCAE v5.0), treatment emergent adverse events leading to treatment discontinuation and death, and their relation to the investigational drug; |
| • To evaluate the preliminary efficacy of **2149** in subjects with advanced melanoma, including objective response rate (ORR), duration of response (DoR), progression free survival (PFS), disease control rate (DCR), time to response (TTR), time to progression (TTP) assessed by investigators according to RECIST V1.1, as well as DoR and PFS rates at 6 and 12 months. | |
| | Efficacy evaluation: |
| | • Investigator-assessed ORR according to RECIST v1.1. |
| | • Investigator-assessed DoR, PFS, DCR, TTR, TTP according to RECIST v1.1, as well as DoR and PFS rates at 6 and 12 months. |

| Secondary objectives | Secondary endpoints |
|---|---|
| • To evaluate overall survival (OS) of **2149** in subjects with advanced melanoma. | • Overall survival (OS) of subjects and OS rates at 6 months and 12 months. |
| | • Pharmacokinetics, descriptive statistics of blood concentration of **2149**. |
| • To evaluate the immunogenicity of **2149** in subjects with advanced melanoma. | |
| | • To evaluate the immunogenicity of **2149**. |
| • To evaluate pharmacokinetics characteristics of **2149** in subjects with advanced melanoma. | |

| Exploratory objectives | Exploratory endpoints |
|---|---|
| • To explore the optimal dosing regimen of **2149**. | • To explore the optimal dosing regimen of **2149**. |
| • To explore the relationship between PD-L1 expression levels in tumor tissue samples, tumor microenvironment, and efficacy. | • To explore the proportion of subjects at baseline with different PD-L1 expression levels in tumor tissue and the relationship between different expression levels and efficacy. |
| • To explore the relationship between pre-dose peripheral blood sCD25 levels and changes post-dose with efficacy. | |
| | • To explore the relationship between tumor microenvironment in tumor tissue at baseline and efficacy. |
| | • To explore the changes in PD-L1 expression levels and tumor microenvironment in tumor tissue after treatment compared to baseline and their relationship with efficacy. |
| | • To explore the relationship between pre-dose and post-dose changes in peripheral blood sCD25 and efficacy. |

### 2. Study design

This study is an open-label, multi-center Phase II study. Aimed to evaluate the safety, tolerability, and preliminary efficacy of **2149** in subjects with advanced melanoma. This study includes the following two cohorts, each enrolling no more than 60 subjects:
**Cohort A: unresectable locally advanced or metastatic melanoma (excluding uveal melanoma) that progressed or recurred after prior treatment with immune checkpoint inhibitors.**
   Failure of immune checkpoint inhibitor therapy in this study requires meeting the following criteria: 1) having received at least 2 cycles of immune checkpoint inhibitor therapy. Subjects who have progressive disease after only 1 cycle of immune checkpoint inhibitor therapy, excluding pseudo progression, are permitted to enroll. 2) progressive disease occurring during immune checkpoint inhibitor therapy or within 6 months after the last immune checkpoint inhibitor treatment. Subjects who received subsequent chemotherapy, targeted therapy, or other treatments following progression on immune checkpoint inhibitor therapy and who experienced progressive disease are eligible for enrollment.
**Cohort B: unresectable locally advanced or metastatic melanoma (excluding uveal melanoma) not previously treated with systemic immune checkpoint inhibitors. Prior immune checkpoint inhibitor adjuvant therapy is permitted, and prior other systemic treatments (for example chemotherapy, anti-angiogenic therapy, and targeted therapy for patients with driver gene mutation) are permitted.**

To determine the optimal dosing strategy of **2149** in the melanoma population, this study **2149** plans to explore two doses: 1 mg/kg Q2W or 3 mg/kg Q3W; employing a gradient dosing strategy where 1 administration of ≤100 µg/kg **2149** on C1D1, followed 7 days later by 1 mg/kg Q2W or 3 mg/kg Q3W **2149** treatment. Administration of 1 mg/kg Q2W or 3 mg/kg Q3W may be decided by the investigator based on the subject's actual condition and must be agreed upon with the sponsor.

This study permits subjects to perform self-dose escalation. If a subject receiving a low dose of **2149** (for example 1 mg/kg) does not experience drug interruption or discontinuation due to ≥ Grade 2 severity adverse events related to the investigational drug, then after review of all data by the investigator and sponsor, it will be decided whether the subject can receive higher dose **2149** (3 mg/kg) treatment. Post-dose escalation, subject safety monitoring, PK sampling, tumor imaging examinations, and other visits should continue to follow prior arrangements.

Subjects will receive investigational drug treatment until progressive disease, intolerable toxicity, unwillingness to continue participation, occurrence of other reasons requiring treatment cessation, or a treatment duration of 24 months (whichever occurs first).

This study allows investigators to reduce the investigational drug dose (for example to 600 µg/kg) or extend the dosing interval (for example Q3W) based on the subject's clinical condition, including but not limited to safety, tolerability, and efficacy. In principle, the investigational drug dose may be reduced by up to two dose levels; if the investigator assesses that further dose reduction would still benefit the subject, communication and discussion with the sponsor is necessary. The maximum treatment duration of the investigational drug is 24 months.

In this study, the first cycle of Q2W administration is 35 days after the priming dose (i.e., the starting dose of gradient dosing), and visits are conducted every 28 days from the second cycle onwards. For Q3W administration, the first cycle is 28 days after the priming dose, and visits are conducted every 21 days from the second cycle onwards.

Efficacy evaluation of subjects in this study is performed according to the RECIST v1.1 criteria. During the study period, subjects will undergo tumor imaging assessments every 6 weeks (±7 days) within 54 weeks of the first dose, and every 12 weeks (±7 days) after 54 weeks from the first dose, until progressive disease, initiation of new antitumor therapy, withdrawal of consent, loss to follow-up, death, or study termination (whichever occurs first).

Subjects will have an end-of-treatment visit within 10 days of treatment discontinuation confirmation. Safety follow-up should be conducted 30 (+7) days after the last dose.

### 3. Dose selection

This study uses gradient dosing (1 administration of ≤100 µg/kg **2149** on C1D1, followed 7 days later by 1 mg/kg Q2W or 3 mg/kg Q3W **2149** treatment) to explore safety, tolerability, and preliminary efficacy in subjects with advanced melanoma.

### 4. Study population

### 4.1 Inclusion Criteria

Enrolled subjects (subjects must meet all the following criteria):
(1) Signed written informed consent and able to comply with the protocol-specified visit schedule and related procedures.
(2) Age ≥18 years, any gender.
(3) Histologically or cytologically confirmed unresectable locally advanced or metastatic melanoma (according to the American Joint Committee on Cancer [AJCC] 8th edition staging, stage III-IV). Stage II subjects assessed by the investigator as not completely resectable may be enrolled after discussion and agreement with the sponsor.
(4) Cohort A: unresectable locally advanced or metastatic melanoma (excluding uveal melanoma) that progressed or recurred after prior treatment with immune checkpoint inhibitors. If prior adjuvant or neoadjuvant treatment included immune checkpoint inhibitors and progressive disease occurred during treatment or within 6 months after treatment cessation as unresectable or metastatic melanoma, such treatment can be considered as first-line systemic therapy.
   Cohort B: unresectable locally advanced or metastatic melanoma (excluding uveal melanoma) not previously treated with systemic immune checkpoint inhibitors. Prior immune checkpoint inhibitor adjuvant therapy is permitted. Prior receipt of other systemic treatments (for example chemotherapy, anti-angiogenic therapy; patients with driver gene mutation are permitted to receive targeted therapy) is permitted.
(5) According to RECIST v1.1 criteria, there is at least one measurable lesion (target lesion).
   ◆ For lesions previously treated with radiotherapy or intratumoral injection, progression of the lesion after treatment can be considered as a measurable lesion.
   ◆ The target lesions in this study must be selected from measurable lesions on imaging (enhanced CT or MRI); lesions located on skin or other superficial areas that cannot be repeatedly measured by imaging can only be considered non-target lesions.
(6) Eastern Cooperative Oncology Group performance status score (ECOG PS) was 0 or 1.
(7) The survival time was expected ≥ 3 months.
(8) Female subjects of childbearing potential or male subjects with partners who are females of childbearing potential agree to strictly use effective contraception during the entire treatment period and for 6 months after treatment.

### 4.2 Exclusion Criteria

Subjects meeting any of the following criteria should not be enrolled in this study:
(1) Pregnant or lactating women, or women planning to become pregnant before, during, or within 6 months after the last administration of the investigational drug.
(2) Active or symptomatic central nervous system metastases confirmed by imaging assessment during screening or prior imaging (including brain parenchyma, leptomeningeal, spinal cord metastases). For subjects with central nervous system metastases unexpectedly detected during screening, or clinically stable after treatment, participation in this study is permitted if all the following criteria are met:
   ◆ measurable lesions outside the central nervous system;
   ◆ no meningeal, midbrain, pons, medulla oblongata, or spinal cord metastases, nor multiple cerebellar metastases;
   ◆ no compression of the cerebral aqueduct, no compression of the third or fourth ventricles, and no spinal cord compression;
   ◆ for untreated subjects with central nervous system metastases: the investigator assesses no need for immediate treatment targeting the central nervous system metastases; number of central nervous system metastases ≤ 3, and the longest diameter of each lesion ≤ 5 mm.
   ◆ For subjects with treated central nervous system metastases, all the following criteria must be met: 1) treatment targeting the central nervous system metastases (for example whole brain radiotherapy, stereotactic radiotherapy, or other equivalent treatments) was completed ≥ 14 days before the first dose of the investigational drug; 2) repeat imaging after completion of treatment shows no new brain metastases or enlargement of existing brain metastases (the interval between imaging and pre-treatment head imaging must be ≥ 4 weeks and use the same imaging modality); 3) no steroid treatment is required ≥ 14 days before first dose of investigational drug and symptoms are stable.
(3) At baseline * (within 7 days before first administration of investigational drug), any of the following hematologic abnormalities are present:
   ◆ $hemoglobin < 90 g / L$
   ◆ $absolute neutrophil count \left(ANC\right) < 1.5 \times {10}^{9} / L$
   ◆ $platelet count < 100 \times {10}^{9} / L$
      *Throughout the protocol, "baseline" is defined as the last available observation prior to the first administration of the investigational drug. Subjects must not have received blood transfusion products (including red blood cells, single-donor platelets, cryoprecipitate, etc.), erythropoietin, or colony-stimulating factor support within 7 days before blood sample collection.
(4) Any serum biochemical abnormalities present at baseline (within 7 days prior to first administration):
   ◆ $total bilirubin > 1.5 \times upper limit of normal \left(ULN\right) ;$
   ◆ aspartate aminotransferase (AST) or alanine aminotransferase (ALT) >3×ULN; if tumor liver metastasis is present, AST or ALT >5.0×ULN;
   ◆ serum creatinine >1.5×ULN or clearance of creatinine (CCr) <45 mL/min, CCr calculated using the Cockcroft-Gault formula (using actual body weight);
   ◆ $albumin < 30 g / L .$
(5) Any coagulation parameter abnormalities present at baseline (within 7 days prior to first administration):
   ◆ international normalized ratio (INR) >1.5×ULN (if on stable dose anticoagulation therapy, >3×ULN);
   ◆ partial thromboplastin time (PTT) (or activated partial thromboplastin time, aPTT) >1.5×ULN (if on stable dose anticoagulation therapy, >3×ULN).
(6) History of active thrombosis, deep vein thrombosis or pulmonary embolism within 4 weeks prior to the first administration of the investigational drug, unless adequately treated and the investigator considers the condition stable.
(7) Active uncontrolled bleeding or known bleeding tendency.
(8) Clinically significant cardiovascular or cerebrovascular diseases, including:
   ◆ symptomatic, clinically unstable, or requiring clinical intervention arrhythmia;
   ◆ HR corrected QT interval (QTc interval, calculated using Fridericia's method) ≥480ms;
   ◆ arterial hypertension uncontrolled despite standard treatment (systolic blood pressure ≥160 mmHg or diastolic blood pressure ≥100 mmHg);
   ◆ history of myocarditis;
   ◆ left ventricular ejection fraction (LVEF) <50%;
   ◆ currently requiring treatment for congestive heart failure;
   ◆ cardiac insufficiency class II-IV according to the New York Heart Association (NYHA) classification;
   ◆ history of acute coronary syndrome (including myocardial infarction and unstable angina), coronary angioplasty, or stent implantation within 6 months before the first administration of the investigational drug.
   ◆ history of cerebrovascular accident or transient ischemic attack within 6 months prior to the first administration of the investigational drug;
   ◆ known history of epileptic seizures.
(9) Interstitial pneumonia, pulmonary fibrosis, pneumoconiosis, drug-related pneumonia, radiation pneumonia, etc., requiring steroid hormones or other treatment, as well as history of severe pulmonary function abnormality or other forms of restrictive lung disease.
(10) History of allergic constitution, asthma, or atopic dermatitis.
(11) Imaging showing massive ascites or symptomatic ascites requiring clinical intervention; those with small amount of ascites on imaging but asymptomatic may be included.
(12) Bilateral moderate pleural effusion, or unilateral large pleural effusion, or patients with respiratory function impairment requiring drainage.
(13) Subject currently or recently (within 6 months) having major gastrointestinal diseases or conditions, including:
   ◆ history of inflammatory bowel disease;
   ◆ occurrence of grade ≥2 diarrhea within 2 weeks prior to the first administration of the investigational drug.
(14) Active autoimmune disease requiring systemic treatment (for example use of disease-modifying drugs, corticosteroids, or immunosuppressants) within 2 years before the first dosing. Substitution therapy (for example thyroxine, insulin, or physiological corticosteroids for adrenal or pituitary insufficiency) is not considered systemic treatment;
(15) known history of allogeneic organ transplantation and allogeneic hematopoietic stem cell transplantation.
(16) Subjects known or suspected to be allergic to the investigational drug and any excipients.
(17) Subjects with a prior significant toxicity history related to immune checkpoint inhibitor administration requiring permanent discontinuation of the therapy.
(18) Subjects with unresolved > Grade 1 toxicity related to any prior antitumor therapy (excluding alopecia, fatigue, hypothyroidism requiring only thyroid hormone replacement, hyperglycemia requiring only insulin replacement, electrolyte abnormalities requiring only symptomatic treatment, and other conditions deemed by the investigator not to affect investigational drug treatment).
(19) Subjects not fully recovered from prior surgery or having undergone any major surgery within 4 weeks prior to first dosing of the investigational drug.
(20) Subjects with uncontrolled tumor-related pain or symptomatic hypercalcemia.
(21) Subjects known to be HIV positive, or suffering from active hepatitis B, hepatitis C (HCV), or tuberculosis.
   ◆ Patients positive for hepatitis B surface antigen (HBsAg) or hepatitis B core antibody (HBcAb) require hepatitis B virus (HBV) DNA testing; enrollment is permitted if HBV DNA copies ≤2.5×10³ copies/ml or ≤500 IU/ml or below the detection limit. HBsAg(+) subjects should receive anti-HBV therapy during the entire investigational drug treatment period to prevent virus reactivation. Subjects who are anti-HBc(+), HBsAg(-), anti-HBs(-), and HBV viral load (-) do not require prophylactic anti-HBV treatment but require close monitoring for viral reactivation;
   ◆ subjects seropositive for hepatitis C virus but with HCV RNA testing negative or below detection limit are permitted to be enrolled;
   ◆ subjects treated for HCV with undetectable viral load results are permitted to be enrolled.
   ◆ Subjects with previously cured or clinically stable old tuberculosis assessed by the investigator are permitted to be enrolled.
(22) Subjects with severe/active/uncontrolled infection, infection requiring systemic intravenous antibiotic treatment, or fever of unknown origin (>38.3°C lasting more than 3 weeks) within 2 weeks prior to the first administration of the investigational drug.
(23) History or current evidence of any disease, treatment or laboratory abnormality that, in the investigator's judgment, may jeopardize subject safety, interfere with obtaining informed consent, affect subject compliance, or impact safety evaluation of the investigational drug.
(24) Subjects with mental illness, altered mental status, or drug abuse that impairs understanding of the informed consent process and/or completion of necessary study-related assessments.
(25) The investigator considers that the subject is unable to meet protocol requirements based on known or foreseeable reasons.
(26) Diagnosis within 5 years prior to first administration of primary uveal or ocular melanoma, and other pathologically confirmed malignant tumors, except for completely treated basal cell carcinoma of the skin, squamous cell carcinoma of the skin and/or carcinoma in situ, locally treated prostate cancer after radical treatment, papillary thyroid carcinoma, and other cured malignant tumors with no known active disease and extremely low risk of recurrence for at least 2 years before study entry.
(27) Excluded drugs and other treatments (subjects must not receive any of the following treatments):
   (a) IL-2/IL-15 class cytokines. The use of IL-2/IL-15 was allowed as neoadjuvant or adjuvant therapy, or as a component of cell therapy;
   (b) received any chemotherapy or small molecule targeted therapy within 2 weeks or 5 half-life periods (whichever is shorter) before the first administration of the investigational drug without delayed toxicity, except for the following situations:
      ◆ use of nitrosourea antitumor drugs and mitomycin C within 6 weeks before the first administration of the investigational drug.
   (c) use of any antibody therapy within 4 weeks before the first administration of the investigational drug;
   (d) participation in any clinical trial involving medical devices or other interventional therapies within 2 weeks before the first administration of the investigational drug;
   (e) received palliative radiotherapy within 2 weeks before the first administration of the investigational drug;
   (f) use of live vaccines for infectious disease prevention within 28 days before the first administration of the investigational drug;
   (g) received immunosuppressive or systemic steroid therapy (>10 mg/day prednisone equivalent) within 2 weeks before the first administration of the investigational drug.

### 5. Study treatment

### 5.1 Treatment regimen

The treatment regimen of this study is as follows:

| **Investigational drug** | **Dose/amount** | **Administration frequency** | **Administration method** | **Treatment course/treatment cycle** |
|---|---|---|---|---|
| **Gradient dosing** | | | | |
| **2149** | Priming dose ≤100 µg/kg; subsequent 1 mg/kg or 3 mg/kg | Q2W or Q3W ² | Intravenous infusion (IV) | In the first cycle, after 1 priming dose administration, predetermined dose escalation is performed (the first cycle lasts 28 days (7+21); subsequent cycles administered every 21 days (1 cycle) until progressive disease, intolerable toxicity, subject unwillingness to continue participation, occurrence of other reasons requiring study treatment discontinuation, or treatment duration reaching 24 months (whichever occurs first) |

### Note:

1: If no drug interruption or termination-causing adverse event of severity ≥grade 2 related to the investigational drug occurs during low-dose **2149** treatment, the investigator and sponsor will review all data to decide whether the subject can subsequently receive high-dose **2149** treatment.
2: This study uses gradient dosing with 1 administration of ≤100 µg/kg **2149** on C1D1, followed by 1 mg/kg Q2W or 3 mg/kg Q3W after 7 days.

### 5.2 Formulation and administration of the investigational drug

The preparation and administration of the investigational drug are the same as in Example 1.

### 6. Safety evaluation

### 6.1. Laboratory tests

Laboratory tests include routine blood tests, blood biochemistry, myocardial enzymes and troponin, urinalysis, viral serology, coagulation function indicators, thyroid function, stool routine, cytokines (IFN-γ, TNF-α, IL-8, IL-6, IL-10), pregnancy test, and liver function tests.

### 6.2. Clinical examination

Clinical examinations include physical examination (general condition, respiratory system, cardiovascular system, abdomen, skin, head and neck (including ear, eye, nose, and throat), lymph nodes, thyroid, musculoskeletal system (including spine and limbs), genitals/anus (if applicable), and neurological evaluation); vital signs (temperature, pulse, respiratory rate, and blood pressure); 12-lead electrocardiogram (ECG); and assessments and examinations during and after infusion (any treatment-related adverse event).

### 6.3. Echocardiography

Echocardiography will be analyzed locally according to the visit schedule, including during screening and as clinically required. Further examinations will be conducted if clinically necessary, for example, in case of cardiac-related adverse events. On the day of examination, the investigator shall complete the echocardiography assessment and record the assessment results. The same assessment method should be used throughout the study duration.

The investigator shall evaluate all echocardiographic results based on categories of clinically significant abnormalities/clinically insignificant abnormalities. If the result is a clinically significant abnormality, the investigator shall record the result as an AE in the eCRF.

### 6.4. Pulmonary function

If baseline imaging identifies potential evidence of interstitial pneumonia, pulmonary fibrosis, or other drug-related pulmonary toxicity, or if there are pulmonary symptoms or signs, pulmonary function testing is recommended. Pulmonary function testing includes pulmonary ventilation and diffusion function. Pulmonary ventilation function testing shall at least include: FVC, FEV1, RV, and TLC; pulmonary diffusion function testing shall at least include DLCO. Pulmonary function testing shall be performed as assessed clinically necessary by the investigator. On the day of examination, the investigator shall complete the pulmonary function assessment and record the assessment results. The same assessment method should be used throughout the study duration. The investigator shall evaluate all pulmonary function results based on categories of clinically significant abnormalities/clinically insignificant abnormalities. If the result is a clinically significant abnormality, the investigator shall record the result as an AE in the eCRF.

Details can be referred to in Embodiment 1.

### 7. Efficacy evaluation

Baseline evaluation will be conducted within 28 days prior to C1D1. The investigator may collect imaging data within 28 days prior to C1D1 (bone scans within 3 months prior to C1D1 are acceptable). Baseline tumor imaging examination includes enhanced CT scans (covering chest, abdomen, and pelvis; enhanced MRI may be used for subjects allergic to CT contrast agents), enhanced brain MRI, and, if necessary, additional enhanced CT, enhanced MRI, or bone scans of other sites. If baseline contrast-enhanced brain MRI reveals brain metastases, subsequent tumor imaging assessments must include contrast-enhanced brain MRI; if baseline contrast-enhanced brain MRI does not detect brain metastases, the investigator must repeat contrast-enhanced brain MRI during treatment upon suspected brain metastases in the subject. Subjects with bone metastases at baseline are recommended to undergo bone scans regularly (for example every 12 weeks); subjects without bone metastases at baseline require bone scan reassessment during treatment if the investigator suspects bone metastases. Contrast-enhanced CT and MRI examinations are scheduled at baseline, every 6 weeks (±7 days) within the first 54 weeks of priming dosing, every 12 weeks (±7 days) after 54 weeks of priming dosing, and as deemed necessary by the investigator. Bone scan timing is determined by the investigator's assessment (subjects with bone metastases are recommended to be assessed every 12 weeks). All subjects undergo regular tumor imaging assessments until progressive disease, initiation of new antitumor therapy, withdrawal of consent, loss to follow-up, death, or study termination, whichever occurs first.

### 8. Pharmacokinetics analysis

3.5 ml whole blood was collected, serum was separated, aliquoted and frozen for PK analysis. If necessary, PK samples will be used for immunogenicity testing.

### 9. Immunogenicity analysis

5 ml whole blood was collected using coagulation-promoting vacuum tubes, serum was separated, aliquoted and frozen for **2149** ADA and/or NAb analysis. Immunogenicity samples will be used for **2149** concentration testing when necessary.

### 10. Tissue biomarker analysis

With ethics committee approval, tumor tissue slices (including archived or freshly collected) will be collected according to the visit schedule for biomarker analysis. Subjects successfully enrolled will provide 10 tumor tissue sections (optional) for PD-L1 expression level and tumor microenvironment analysis. With Ethics Committee approval, subject consent, and when deemed feasible by the investigator, subjects will be asked to provide up to 10 tumor tissue sections (optional) from fresh tumor biopsy or puncture specimens at the time of objective response, progressive disease, or clinical need (or tumor tissue specimens obtained post-neoadjuvant therapy surgery) for PD-L1 expression level and tumor microenvironment analysis. If testing failure occurs, and residual slices are available at the clinical center, additional slices may be requested.

### 11. Peripheral blood biomarker analysis

With Ethics Committee approval, approximately 3.5 mL of peripheral blood would be collected according to the visit schedule for sCD25 level analysis in peripheral blood.

### 12. Results

As of July 5, 2024, the study results are as follows:

**Table 11. Baseline characteristics of enrolled patients**

| **Baseline characteristics** | | **Total (N=45)** |
|---|---|---|
| Age, median (range) | Years | 61 (41-81) |
| Sex, n (%) | Male | 25 (55.6) |
| | Female | 20 (44.4) |
| E COG score, n (%) | 0 | 26 (57.8) |
| | 1 | 19 (42.2) |
| T umor staging, n (%) | III | 4 (8.9) |
| | IV | 41 (91.1) |
| P rior immunotherapy (IO), n (%) | Yes | 29 (64.4) |
| | No | 16 (35.6) |
| Melanoma subtype, n (%) | Mucosal type | 23 (51.1) |
| | Acral type | 11 (24.4) |
| | Cutaneous type | 9 (20.0) |
| | Primary lesion unknown | 2 (4.4) |
| D ose, n (%) | 1 mg/kg Q2W | 14 (31.1) |
| | 1.5 mg/kg Q3W* | 1 (2.2) |
| | 3 mg/kg Q3W | 30 (66.7) |

| | | |
|---|---|---|
| *: In the initial version of the clinical protocol, enrollment of the 1.5 mg/kg dose was permitted; this was the first patient enrolled in this study. To ensure data integrity, the patient was not excluded. | | |

**Table 12. Overall efficacy**

| | | All efficacy evaluable patients (N=37) | Efficacy evaluable patients not previously treated with immunotherapy (N=13) |
|---|---|---|---|
| Best overall response, n (%) | | | |
| | CR (complete response) | 0 | 0 |
| | PR (partial response) | 14(37.8) | 9 (69.2) |
| | SD (stable disease) | 13(35.1) | 1 (7.7) |
| | PD (progressive disease) | 7(18.9) | 2 (15.4) |
| | ND (not done) | 3(8.1) | 1 (7.7) |
| ORR (objective response rate), % (95% CI) | | 37.8 (22.5-55.2) | 69.2 (38.6-90.9) |
| DCR (disease control rate), % (95% CI) | | 73.0 (55.9-86.2) | 76.9 (46.2-95.0) |

**Table 13. Safety evaluation**

| **n (%)** | | **1 mg/kg Q2W (N=14)** | **1.5 mg/kg Q3W (N=1)** | **3 mg/kg Q3W (N=30)** | **Total (N=45)** |
|---|---|---|---|---|---|
| Any TEAE | | 14 (100) | 1 (100) | 22 (73.3) | 37 (82.2) |
| | TEAE of Grade ≥ 3 | 1 (7.1) | 0 | 6 (20.0) | 7 (15.6) |
| Any TRAE | | 13(92.9) | 0 | 19(63.3) | 32(71.1) |
| | TRAE of Grade ≥ 3 | 1 (7.1) | 0 | 5(16.7) | 6(13.3) |
| Any TESAE | | 5(35.7) | 1 (100) | 7(23.3) | 13(28.9) |
| Any TRSAE | | 1 (7.1) | 0 | 6 (20.0) | 7 (15.6) |
| TEAE leading to death | | 0 | 0 | 0 | 0 |
| TRAE leading to death | | 0 | 0 | 0 | 0 |
| TEAE leading to treatment discontinuation | | 0 | 0 | 0 | 0 |
| TRAE leading to treatment discontinuation | | 0 | 0 | 0 | 0 |
| TEAE leading to treatment interruption | | 4 (28.6) | 0 | 7(23.3) | 11 (24.4) |
| TRAE leading to treatment interruption | | 4 (28.6) | 0 | 6 (20.0) | 10 (22.2) |
| Any irAE | | 13(92.9) | 0 | 17 (56.7) | 30 (66.7) |
| | irAE of Grade ≥ 3 | 1 (7.1) | 0 | 3(10.0) | 4(8.9) |

Overall, the above dosages (1 mg/kg Q2W and 3 mg/kg Q3W) exhibited controllable safety and favorable efficacy. **2149** showed favorable efficacy in patients with advanced malignant tumors. Specifically, **2149** demonstrated good clinical benefit in patients with advanced melanoma failed or intolerant to standard therapy. In summary, **2149** exhibited excellent antitumor activity and manageable safety profile in melanoma subjects failed to standard therapy, reduced exposure of advanced tumor patients to ineffective dosage regimens and improved subject tolerance to the investigational drug.

### Sequence Listing:

| **SEQ ID NO** | **Description** | **Sequence** |
|---|---|---|
| 1 | Full-length native IL-2 | MYRMQLLSCIALSLALVTNSAPTSSSTKKTQLQLEHLLLD LQMILNGINNYKNPKLTRMLTFKFYMPKKATELKHLQCL EEELKPLEEVLNLAQSKNFHLRPRDLISNINVIVLELKGSET TFMCEYADETATIVEFLNRWITFCQSIISTLT |
| 2 | Mature IL-2 | |
| 3 | Wild-type IL-2 (including C125S) | APTSSSTKKTQLQLEHLLLDLQMILNGINNYKNPKLTRML TFKFYMPKKATELKHLQCLEEELKPLEEVLNLAQSKNFHL RPRDLISNINVIVLELKGSETTFMCEYADETATIVEFLNRWI TFSQSIISTLT |
| | | |

| **Molecule 2149** | **having T3A+N88R + S130R mutations, and IL15B'C' loop replacement** | |
|---|---|---|
| 4 | IL-2 mutein | APASSSTKKTQLQLEHLLLDLQMILNGINNYKNPKLTRML TFKFYMPKKATELKHLQCLEEELKPLEEVLNLAGDASIHD LISRINVIVLELKGSETTFMCEYADETATIVEFLNRWITFSQ SIIRTLT |
| 5 | Linker | GGGGSGGGGS |
| 6 | Fc-Knob S354C/T366 W+LALA | DKTHTCPPCPAPEAAGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPCRDELTKNQVSLWCLVKGFYPSDIA VEWESNGQPENNYKTTPPVLDSDGSFFLYSKLTVDKSRW QQGNVFSCSVMHEALHNHYTQKSLSLSPGK |
| 7 | IL-2m-linker-Fc-Knob | APASSSTKKTQLQLEHLLLDLQMILNGINNYKNPKLTRML TFKFYMPKKATELKHLQCLEEELKPLEEVLNLAGDASIHD LISRINVIVLELKGSETTFMCEYADETATIVEFLNRWITFSQ SIIRTLTGGGGSGGGGSDKTHTCPPCPAPEAAGGPSVFLFP PKPKDTLMISRTPEVTCVVVDVSHEDPEVKFNWYVDGVE VHNAKTKPREEQYNSTYRVVSVLTVLHQDWLNGKEYKC KVSNKALPAPIEKTISKAKGQPREPQVYTLPPCRDELTKNQ VSLWCLVKGFYPSDIAVEWESNGQPENNYKTTPPVLDSD GSFFLYSKLTVDKSRWQQGNVFSCSVMHEALHNHYTQKS LSLSPGK |

| | **Anti-PD-1 antibody portion** | |
|---|---|---|
| 8 | Anti-PD-1 heavy chain variable region | QVQLVQSGAEVKKPGSSVKVSCKASGGTFSSYAISWVRQ APGQGLEWMGLIIPMFDTAGYAQKFQGRVAITVDESTSTA YMELSSLRSEDTAVYYCARAEHSSTGTFDYWGQGTLVTV SS |
| 9 | Anti-PD-1 heavy chain variable region CDR1 | GGTFSSYAIS |
| 10 | Anti-PD-1 heavy chain variable region CDR2 | LIIPMFDTAGYAQKFQG |
| 11 | Anti-PD-1 heavy chain variable region CDR3 | AEHSSTGTFDY |
| 12 | Fc-Hole Y349C/T366 S/L368A/Y4 07V+LALA | DKTHTCPPCPAPEAAGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVCTLPPSRDELTKNQVSLSCAVKGFYPSDIA |
| | | VEWESNGQPENNYKTTPPVLDSDGSFFLVSKLTVDKSRW QQGNVFSCSVMHEALHNHYTQKSLSLSPGK |
| 13 | Anti-PD-1 heavy chain constant region | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVCTLPPSRD ELTKNQVSLSCAVKGFYPSDIAVEWESNGQPENNYKTTPP VLDSDGSFFLVSKLTVDKSRWQQGNVFSCSVMHEALHNH YTQKSLSLSPGK |
| 14 | Anti-PD-1 heavy chain | QVQLVQSGAEVKKPGSSVKVSCKASGGTFSSYAISWVRQ APGQGLEWMGLIIPMFDTAGYAQKFQGRVAITVDESTSTA YMELSSLRSEDTAVYYCARAEHSSTGTFDYWGQGTLVTV SSASTKGPSVFPLAPSSKSTSGGTAALGCLVKDYFPEPVTV SWNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVCTLPPSRD ELTKNQVSLSCAVKGFYPSDIAVEWESNGQPENNYKTTPP VLDSDGSFFLVSKLTVDKSRWQQGNVFSCSVMHEALHNH YTQKSLSLSPGK |
| 15 | Anti-PD-1 light chain variable region | DIQMTQSPSSVSASVGDRVTITCRASQGISSWLAWYQQKP GKAPKLLISAASSLQSGVPSRFSGSGSGTDFTLTISSLQPED FATYYCQQANHLPFTFGGGTKVEIK |
| 16 | Anti-PD-1 light chain variable region CDR1 | RASQGISSWLA |
| 17 | Anti-PD-1 light chain variable region CDR2 | AASSLQS |
| 18 | Anti-PD-1 light chain variable region CDR3 | QQANHLPFT |
| 19 | Anti-PD-1 light chain constant region | |
| 20 | Anti-PD-1 light chain | DIQMTQSPSSVSASVGDRVTITCRASQGISSWLAWYQQKP GKAPKLLISAASSLQSGVPSRFSGSGSGTDFTLTISSLQPED |
| | | FATYYCQQANHLPFTFGGGTKVEIKRTVAAPSVFIFPPSDE QLKSGTASVVCLLNNFYPREAKVQWKVDNALQSGNSQES VTEQDSKDSTYSLSSTLTLSKADYEKHKVYACEVTHQGLS SPVTKSFNRGEC |
| 21 | Heavy chain constant region (without Knob-Hole) | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRD ELTKNQVSLTCLVKGFYPSDIAVEWESNGQPENNYKTTPP VLDSDGSFFLYSKLTVDKSRWQQGNVFSCSVMHEALHNH YTQKSLSLSPGK (with LALA mutation (L234A & L235A), reducing the ADCC effect of IgG1 Fc) |
| 22 | Heavy chain (without Hole mutation) | QVQLVQSGAEVKKPGSSVKVSCKASGGTFSSYAISWVRQ APGQGLEWMGLIIPMFDTAGYAQKFQGRVAITVDESTSTA YMELSSLRSEDTAVYYCARAEHSSTGTFDYWGQGTLVTV SSASTKGPSVFPLAPSSKSTSGGTAALGCLVKDYFPEPVTV SWNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRD ELTKNQVSLTCLVKGFYPSDIAVEWESNGQPENNYKTTPP VLDSDGSFFLYSKLTVDKSRWQQGNVFSCSVMHEALHNH YTQKSLSLSPGK (with LALA mutation (L234A & L235A), reducing the ADCC effect of IgG1 Fc) |
| 23 | Fc (without Knob-Hole mutation, with LALA mutation) | DKTHTCPPCPAPEAAGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPGK |
| 24 | Fc (without Knob-Hole mutation, without LALA mutation) | DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPGK |
| 25 | IL15 B'C' loop region | AGDASIH |
| 26 | Truncated IL-2 B'C' loop region | AQSKNFH |

## Claims

1. Use of a PD-1/IL-2R bispecific antibody fusion protein for treating a solid tumor or a hematologic tumor, wherein the PD-1/IL-2R bispecific antibody fusion protein comprises: (a) a first monomer comprising an IL-2 mutein fused with an Fc fragment, optionally via or not via a linker, wherein the IL-2 mutein has T3A+N88R+S130R mutations and IL15 B'C' loop region AGDASIH relative to wild-type IL-2 (for example an amino acid sequence as set forth in SEQ ID NO: 3); and (b) a second monomer comprising a heavy chain and a light chain of an antibody that specifically binds PD-1.

2. The use according to claim 1, wherein the IL-2 mutein comprises or consists of the following amino acid sequence:
an amino acid sequence as set forth in SEQ ID NO: 4; or
an amino acid sequence having at least 90%, 91%, 92%, 93%, 94%, 95%, 96%, 97%, 98%, or 99% identity to the amino acid sequence as set forth in SEQ ID NO: 4 and having T3A+N88R+S130R mutations and IL15B'C' loop region AGDASIH relative to wild-type IL-2 (for example, an amino acid sequence as set forth in SEQ ID NO: 3).

3. The use according to claim 1, wherein the linker comprises (GGGGS)n, wherein n is 1, 2, 3, or 4, for example SEQ ID NO: 5.

4. The use according to any one of claims 1 to 3, wherein the heavy chain of the antibody that specifically binds PD-1 comprises an Fc fragment, and the Fc fragment as well as the Fc fragment fused to the IL-2 mutein is an Fc fragment of IgG1, IgG2, IgG3, or IgG4, for example human IgG1 Fc.

5. The use according to claim 4, wherein the Fc fragment has mutations reducing binding to Fcγ receptor, for example L234A/L235A mutations or L234A/L235E/G237A.

6. The use according to claim 4 or 5, wherein the Fc fragment of the first monomer comprises a Knob mutation, and the antibody heavy-chain Fc fragment of the second monomer contains a Hole mutation, or the Fc fragment of the first monomer comprises a Hole mutation, and the antibody heavy-chain Fc fragment of the second monomer comprises a Knob mutation; for example, the hole mutations are Y349C, T366S, L368A, and Y407V mutations, and/or the Knob mutations are T366W and S354C mutations.

7. The use according to claim 6, wherein
the Fc fragment of the first monomer comprises or consists of the following amino acid sequence: an amino acid sequence as set forth in SEQ ID NO: 6, or an amino acid sequence having at least 90%, 91%, 92%, 93%, 94%, 95%, 96%, 97%, 98%, or 99% identity to the amino acid sequence as set forth in SEQ ID NO: 6;
or
the antibody heavy-chain Fc fragment of the second monomer comprises or consists of the following amino acid sequence: an amino acid sequence as set forth in SEQ ID NO: 12, or an amino acid sequence having at least 90%, 91%, 92%, 93%, 94%, 95%, 96%, 97%, 98%, or 99% identity to the amino acid sequence as set forth in SEQ ID NO: 12.

8. The use according to any one of claims 1 to 6, wherein the first monomer comprises:
an amino acid sequence as set forth in SEQ ID NO: 7; or
an amino acid sequence having at least 90%, 91%, 92%, 93%, 94%, 95%, 96%, 97%, 98% or 99% identity to the amino acid sequence as set forth in SEQ ID NO: 7 and T3A+N88R+S130R mutations and IL15B'C' loop region AGDASIH relative to wild-type IL-2 (for example, an amino acid sequence as set forth in SEQ ID NO: 3) in the IL-2 mutein, and/or S354C/T366W and optionally L234A/L235A mutations in the Fc fragment.

9. The use according to any one of claims 1 to 7, wherein the heavy chain of the antibody that specifically binds PD-1 comprises HCDR1, HCDR2, and HCDR3, and the light chain of the antibody that specifically binds PD-1 comprises LCDR1, LCDR2, and LCDR3, wherein the amino acid sequences of HCDR1, HCDR2, and HCDR3 are respectively: SEQ ID NO: 9, 10, and 11, and the amino acid sequences of LCDR1, LCDR2, and LCDR3 are respectively: SEQ ID NO: 16, 17, and 18.

10. The use according to claim 9, wherein the heavy chain of the antibody that specifically binds PD-1 comprises a heavy-chain variable region VH, and the light chain of the antibody that specifically binds PD-1 comprises a light-chain variable region VL, wherein the VH comprises or consists of an amino acid sequence as set forth in SEQ ID NO: 8 or an amino acid sequence having at least 90%, 91%, 92%, 93%, 94%, 95%, 96%, 97%, 98%, or 99% identity thereto, and the VL comprises or consists of an amino acid sequence as set forth in SEQ ID NO: 15 or an amino acid sequence having at least 90%, 91%, 92%, 93%, 94%, 95%, 96%, 97%, 98%, or 99% identity thereto.

11. The use according to claim 9 or 10, wherein the heavy chain comprises or consists of the following amino acid sequence: an amino acid sequence as set forth in SEQ ID NO: 14, or an amino acid sequence comprising a Hole mutation and having at least 85%, 90%, 91%, 92%, 93%, 94%, 95%, 96%, 97%, 98%, or 99% identity to the amino acid sequence as set forth in SEQ ID NO: 14; the light chain comprises or consists of the following amino acid sequence: an amino acid sequence as set forth in SEQ ID NO: 20, or an amino acid sequence with at least 85%, 90%, 91%, 92%, 93%, 94%, 95%, 96%, 97%, 98%, or 99% identity to the amino acid sequence as set forth in SEQ ID NO: 20.

12. The use according to claim 1, wherein
the first monomer comprises or consists of the amino acid sequence as set forth in SEQ ID NO: 7;
the heavy chain of the second monomer comprises or consists of the amino acid sequence as set forth in SEQ ID NO: 14;
the light chain of the second monomer comprises or consists of the amino acid sequence as set forth in SEQ ID NO: 20.

13. The use according to any of the preceding claims, wherein the fusion protein is administered by injection, preferably intravenous injection, according to the following dosage regimen: a dose level of 0.2 µg/kg to 3 mg/kg body weight, for example, 0.2 µg/kg, 2 µg/kg, 10 µg/kg, 30 µg/kg, 0.1 mg/kg, 0.3 mg/kg, 0.6 mg/kg, 1 mg/kg, 1.5 mg/kg, 2 mg/kg, 2.5 mg/kg, 3 mg/kg body weight or a range composed of any two thereof.

14. The use according to any one of the preceding claims, wherein the fusion protein is administered by injection, preferably intravenous injection, according to the following dosage regimen: a dose of 0.2 µg/kg to 3 mg/kg body weight, for example 0.2 µg/kg, 2 µg/kg, 10 µg/kg, 30 µg/kg, 0.1 mg/kg, 0.3 mg/kg, 0.6 mg/kg, 1 mg/kg, 1.5 mg/kg, 2 mg/kg, 2.5 mg/kg, 3 mg/kg body weight or a range composed of any two thereof, for example a dose of 30 µg/kg-3 mg/kg, 0.1-3 mg/kg, 0.1-2 mg/kg, 0.6-3 mg/kg, 0.6-2 mg/kg, 1-3 mg/kg, or 1-2 mg/kg body weight administered once weekly (QW), once every two weeks (Q2W), or once every three weeks (Q3W); wherein the fusion protein is administered for one or more treatment cycles, and the treatment cycle is three weeks for once weekly or once every three weeks administration, and four weeks for once every two weeks administration.

15. The use according to any one of the preceding claims, wherein the fusion protein is administered by injection, preferably intravenous injection, according to the following dosage regimen:
1-3 mg/kg or 1-2 mg/kg body weight administered once weekly (QW), once every two weeks (Q2W), or once every three weeks (Q3W), wherein the fusion protein is administered for one or more treatment cycles, and the treatment cycle is three weeks for once weekly or once every three weeks administration, and four weeks for once every two weeks administration;
preferably, 0.3, 0.6, 1, or 2 mg/kg body weight administered once every two weeks (Q2W), wherein the fusion protein is administered for one or more treatment cycles, and the treatment cycle is four weeks; or
0.6, 1, 1.5, 2, or 3 mg/kg body weight administered once every three weeks (Q3W), wherein the fusion protein is administered for one or more treatment cycles, and the treatment cycle is three weeks.

16. The use according to any one of the preceding claims, wherein when a single dose is ≥0.6 mg/kg body weight, a dose of the fusion protein of ≤200 µg/kg body weight is administered once by injection, preferably intravenous injection, 7±1 days before a first single dose as a priming dose.

17. The use according to claim 16, wherein the fusion protein is administered by injection, preferably intravenous injection, according to the following dosage regimen: a priming dose of 0.2-200 µg/kg body weight administered on day 1, and from day 8±1, a normal dose of 0.6-3 mg/kg body weight administered once weekly (QW), once every two weeks (Q2W), or once every three weeks (Q3W).

18. The use according to any one of claims 16 to 17, wherein the priming dose is 2-200 µg/kg body weight, preferably 30-200 µg/kg body weight, more preferably 30-150 µg/kg body weight, most preferably 30-100 µg/kg body weight.

19. The use according to claim 17 or 18, wherein the normal dose is 0.6-3 mg/kg, or 0.6-2.5 mg/kg, or 0.6-2 mg/kg, or 1-3 mg/kg, or 1-2.5 mg/kg, or 1-2 mg/kg body weight administered once weekly (QW), once every two weeks (Q2W), or once every three weeks (Q3W), and wherein the normal dose of the fusion protein is administered for one or more treatment cycles, and the treatment cycle is three weeks for once weekly or once every three weeks administration, and four weeks for once every two weeks administration.

20. The use according to any one of claims 17 to 19, wherein the fusion protein is administered by injection, preferably intravenous injection, according to the following dosage regimen: a priming dose of 30-100 µg/kg body weight administered on day 1, and from day 8±1, a normal dose of 0.6-3 mg/kg, for example 0.6-2 mg/kg, for example 1-3 mg/kg, for example 1-2 mg/kg body weight administered once weekly (QW), once every two weeks (Q2W), or once every three weeks (Q3W), wherein the normal dose of the fusion protein is administered for one or more treatment cycles, and the treatment cycle is three weeks for once weekly or once every three weeks administration, and four weeks for once every two weeks administration.

21. The use according to any one of claims 17 to 20, wherein the fusion protein is administered by injection, preferably intravenous injection, according to the following dosage regimen:
a priming dose of 100 µg/kg body weight administered on day 1, and from day 8±1, a normal dose of 0.6, 1, or 2 mg/kg body weight administered once every two weeks (Q2W), wherein the normal dose of the fusion protein is administered for one or more treatment cycles, and the treatment cycle is four weeks; or
a priming dose of 100 µg/kg body weight administered on day 1, and from day 8±1, a normal dose of 0.6, 1, 1.5, 2, or 3 mg/kg body weight administered once every three weeks (Q3W), wherein the normal dose of the fusion protein is administered for one or more treatment cycles, and the treatment cycle is three weeks.

22. The use according to any one of claims 14 to 21, wherein when there is more than one treatment cycle, the treatment cycles may be consecutive or separated.

23. The use according to any one of the preceding claims, wherein the solid tumor or hematologic tumor is selected from intestinal cancer (comprising rectal cancer, colon cancer, colorectal cancer), melanoma, lung cancer (for example non-small cell lung cancer, comprising squamous and non-squamous non-small cell lung cancer), breast cancer (for example triple-negative breast cancer), pancreatic cancer, cholangiocarcinoma, ovarian cancer, cervical cancer, uterine cancer, head and neck cancer (for example head and neck squamous cell carcinoma), liver cancer (for example hepatocellular carcinoma), renal cancer (for example renal cell carcinoma), stomach cancer, lymphoma, leukemia, or multiple myeloma;
preferably, the solid tumor or hematologic tumor is selected from colorectal cancer, melanoma, non-small cell lung cancer (comprising squamous and non-squamous non-small cell lung cancer), breast cancer, pancreatic cancer, cholangiocarcinoma, ovarian cancer, cervical cancer, head and neck cancer (for example head and neck squamous cell carcinoma), hepatocellular carcinoma, renal cell carcinoma, and lymphoma;
more preferably, the solid tumor or hematologic tumor is selected from colorectal cancer, melanoma, and non-small cell lung cancer (comprising squamous and non-squamous non-small cell lung cancer).

24. The use according to any one of the preceding claims, wherein the solid tumor or hematologic tumor is standard therapy-failed or standard therapy-intolerant, advanced, recurrent, metastatic, or unresectable.

25. The use according to any one of the preceding claims, wherein the solid tumor or hematologic tumor is selected from:
melanoma, for example cutaneous melanoma, acral melanoma, mucosal melanoma, or melanoma of unknown primary origin, particularly those that are failed or intolerant to standard therapy, advanced, recurrent, metastatic, or unresectable; for example, unresectable locally advanced or metastatic melanoma that has progressed or recurred after prior immune checkpoint inhibitor treatment, or unresectable locally advanced or metastatic melanoma untreated by systemic immune checkpoint inhibitor therapy;
colorectal cancer, preferably colorectal cancer that is failed or intolerant to standard therapy, advanced, recurrent, metastatic, or unresectable, particularly colorectal cancer with proficient mismatch repair (pMMR) and/or microsatellite stable (MSS) status; and
non-small cell lung cancer, comprising squamous and non-squamous non-small cell lung cancer, particularly non-small cell lung cancer without known driver gene mutation, failed or intolerant to standard therapy, advanced, recurrent, metastatic, or unresectable.

26. A method of treating a solid tumor or a hematologic tumor in an individual, wherein the method comprises administering to an individual in need thereof an effective amount of the PD-1/IL-2R bispecific antibody fusion protein as defined in any one of claims 1 to 12.

27. The method according to claim 26, wherein the PD-1/IL-2R bispecific antibody fusion protein is administered by injection, preferably intravenous injection, according to the dosage regimen as defined in any one of claims 13 to 22.

28. The method according to claim 26, wherein the fusion protein is administered by injection, preferably intravenous injection, according to the following dosage regimen:
a priming dose of 100 µg/kg body weight administered on day 1, and from day 8±1, a normal dose of 0.6, 1, or 2 mg/kg body weight administered once every two weeks (Q2W), wherein the normal dose of the fusion protein is administered for one or more treatment cycles, and the treatment cycle is four weeks; or
a priming dose of 100 µg/kg body weight administered on day 1, and from day 8±1, a normal dose of 0.6, 1, 1.5, 2, or 3 mg/kg body weight administered once every three weeks (Q3W), wherein the normal dose of the fusion protein is administered for one or more treatment cycles, and the treatment cycle is three weeks.

29. The method according to any one of claims 26 to 28, wherein the solid tumor and hematologic tumor are as defined in any one of claims 23 to 25.

30. A pharmaceutical composition for treating a solid tumor or a hematologic tumor, comprising the PD-1/IL-2R bispecific antibody fusion protein as defined in any one of claims 1 to 12, wherein the pharmaceutical composition is administered by injection, preferably intravenous injection, according to the dosage regimen as defined in any one of claims 13 to 22.

31. A pharmaceutical combination for treating a solid tumor or a hematologic tumor, wherein the pharmaceutical combination comprises:
(i) a first active ingredient, wherein the first active ingredient is a PD-1/IL-2R bispecific antibody fusion protein, and the PD-1/IL-2R bispecific antibody fusion protein comprises: (a) a first monomer comprising an IL-2 mutein fused with an Fc fragment, optionally via or not via a linker, wherein the IL-2 mutein has T3A+N88R+S130R mutations and IL15 B'C' loop region AGDASIH relative to wild-type IL-2 (for example an amino acid sequence as set forth in SEQ ID NO: 3); and (b) a second monomer comprising a heavy chain and a light chain of an antibody that specifically binds PD-1; and
(ii) a second active ingredient, wherein the second active ingredient is bevacizumab.

32. The pharmaceutical combination according to claim 31, wherein the IL-2 mutein comprises or consists of the following amino acid sequence:
an amino acid sequence as set forth in SEQ ID NO: 4; or
an amino acid sequence having at least 90%, 91%, 92%, 93%, 94%, 95%, 96%, 97%, 98%, or 99% identity to the amino acid sequence as set forth in SEQ ID NO: 4 and having T3A+N88R+S130R mutations and IL15B'C' loop region AGDASIH relative to wild-type IL-2 (for example, an amino acid sequence as set forth in SEQ ID NO: 3).

33. The pharmaceutical combination according to claim 31, wherein the linker comprises (GGGGS)n, wherein n is an integer of at least 1, for example 1, 2, 3, or 4, for example SEQ ID NO: 5.

34. The pharmaceutical combination according to any one of claims 31 to 33, wherein the heavy chain of the antibody that specifically binds PD-1 comprises an Fc fragment, and the Fc fragment as well as the Fc fragment fused to the IL-2 mutein is an Fc fragment of IgG1, IgG2, IgG3, or IgG4, for example human IgG1 Fc.

35. The pharmaceutical combination according to claim 34, wherein the Fc fragment has mutations that bind to an Fcγ receptor, for example L234A/L235A mutations or L234A/L235E/G237A.

36. The pharmaceutical combination according to claim 34 or 35, wherein the Fc fragment of the first monomer comprises a Knob mutation, and the antibody heavy-chain Fc fragment of the second monomer contains a Hole mutation, or the Fc fragment of the first monomer comprises a Hole mutation, and the antibody heavy-chain Fc fragment of the second monomer comprises a Knob mutation; for example, the hole mutations are Y349C, T366S, L368A, and Y407V mutations, and/or the Knob mutations are T366W and S354C mutations.

37. The pharmaceutical combination according to claim 36, wherein
the Fc fragment of the first monomer comprises or consists of the following amino acid sequence:
an amino acid sequence as set forth in SEQ ID NO: 6, or
an amino acid sequence having at least 90%, 91%, 92%, 93%, 94%, 95%, 96%, 97%, 98%, or 99% identity to the amino acid sequence as set forth in SEQ ID NO: 6;
or
the antibody heavy-chain Fc fragment of the second monomer comprises or consists of the following amino acid sequence:
an amino acid sequence as set forth in SEQ ID NO: 12, or
an amino acid sequence having at least 90%, 91%, 92%, 93%, 94%, 95%, 96%, 97%, 98%, or 99% identity to the amino acid sequence as set forth in SEQ ID NO: 12.

38. The pharmaceutical combination according to any one of claims 31 to 36, wherein the first monomer comprises:
an amino acid sequence as set forth in SEQ ID NO: 7; or
an amino acid sequence having at least 90%, 91%, 92%, 93%, 94%, 95%, 96%, 97%, 98% or 99% identity to the amino acid sequence as set forth in SEQ ID NO: 7 and T3A+N88R+S130R mutations and IL15B'C' loop region AGDASIH relative to wild-type IL-2 (for example, an amino acid sequence as set forth in SEQ ID NO: 3) in the IL-2 mutein, and/or S354C/T366W and optionally L234A/L235A mutations in the Fc fragment.

39. The pharmaceutical combination according to any one of claims 31 to 37, wherein the heavy chain of the antibody that specifically binds PD-1 comprises HCDR1, HCDR2, and HCDR3, and the light chain of the antibody that specifically binds PD-1 comprises LCDR1, LCDR2, and LCDR3, wherein the amino acid sequences of HCDR1, HCDR2, and HCDR3 are respectively: SEQ ID NO: 9, 10, and 11, and the amino acid sequences of LCDR1, LCDR2, and LCDR3 are respectively: SEQ ID NO: 16, 17, and 18.

40. The pharmaceutical combination according to claim 39, wherein the heavy chain of the antibody that specifically binds PD-1 comprises a heavy-chain variable region VH, and the light chain of the antibody that specifically binds PD-1 comprises a light-chain variable region VL,
wherein the VH comprises or consists of the following amino acid sequence: an amino acid sequence as set forth in SEQ ID NO: 8, or an amino acid sequence having at least 90%, 91%, 92%, 93%, 94%, 95%, 96%, 97%, 98%, or 99% identity to the amino acid sequence as set forth in SEQ ID NO: 8, and the VL comprises or consists of the following amino acid sequence: an amino acid sequence as set forth in SEQ ID NO: 15, or an amino acid sequence having at least 90%, 91%, 92%, 93%, 94%, 95%, 96%, 97%, 98%, or 99% identity to the amino acid sequence as set forth in SEQ ID NO: 15.

41. The pharmaceutical combination according to claim 39 or 40, wherein the heavy chain comprises or consists of the following amino acid sequence: an amino acid sequence as set forth in SEQ ID NO: 14, or an amino acid sequence comprising a Hole mutation and having at least 85%, 90%, 91%, 92%, 93%, 94%, 95%, 96%, 97%, 98%, or 99% identity to the amino acid sequence as set forth in SEQ ID NO: 14; the light chain comprises or consists of the following amino acid sequence: an amino acid sequence as set forth in SEQ ID NO: 20, or an amino acid sequence having 85%, 90%, 91%, 92%, 93%, 94%, 95%, 96%, 97%, 98%, or 99% identity to the amino acid sequence as set forth in SEQ ID NO: 20.

42. The pharmaceutical combination according to claim 31, wherein
the first monomer comprises or consists of the amino acid sequence as set forth in SEQ ID NO: 7;
the heavy chain of the second monomer comprises or consists of the amino acid sequence as set forth in SEQ ID NO: 14; and
the light chain of the second monomer comprises or consists of the amino acid sequence as set forth in SEQ ID NO: 20.

43. The pharmaceutical combination according to any one of claims 31 to 42, wherein the first active ingredient is for injection administration, for example intravenous infusion; the second active ingredient is for injection administration, for example intravenous infusion.

44. The pharmaceutical combination according to any one of claims 31 to 42, wherein the first active ingredient is administered by injection, preferably intravenous injection, according to the dosage regimen defined in any one of claims 13 to 22; and the second active ingredient is for intravenous infusion administration at a dose of 5 mg/kg to 15 mg/kg body weight administered once every two weeks with each four weeks constituting a treatment cycle, or administered once every three weeks with each three weeks constituting a treatment cycle, for one or more treatment cycles.

45. The pharmaceutical combination according to claim 44, wherein
the first active ingredient is administered for injection, preferably intravenous injection as follows: a dose of 0.6-3 mg/kg, for example 0.6-2 mg/kg, for example 1-3 mg/kg, or for example 1-2 mg/kg body weight administered once weekly (QW), once every two weeks (Q2W), or once every three weeks (Q3W), for one or more treatment cycles with each treatment cycle being three weeks; or a priming dose of 30-100 µg/kg body weight administered on day 1, and from day 8±1, a normal dose of 0.6-3 mg/kg, for example 0.6-2 mg/kg, for example 1-3 mg/kg, or for example 1-2 mg/kg body weight administered once weekly (QW), once every two weeks (Q2W), or once every three weeks (Q3W), wherein the normal dose is administered for one or more treatment cycles, and each treatment cycle is three weeks; and
the second active ingredient is for intravenous infusion administration at a dose of 5-15 mg/kg body weight (for example 5, 7.5, 10, 12.5, or 15 mg/kg body weight or a range composed of any two thereof) administered once every two weeks with each four weeks constituting a treatment cycle, or once every three weeks with each three weeks constituting a treatment cycle, for one or more treatment cycles.

46. The pharmaceutical combination according to any one of claims 31 to 42, wherein
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle;
or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg to 15 mg/kg body weight administered once every two weeks with each four weeks constituting a treatment cycle, or once every three weeks with each three weeks constituting a treatment cycle.

47. The pharmaceutical combination according to any one of claims 31 to 46, wherein the solid tumor or the hematologic tumor is selected from intestinal cancer (comprising rectal cancer, colon cancer, colorectal cancer), melanoma, non-small cell lung cancer (comprising squamous and non-squamous non-small cell lung cancer), hepatocellular carcinoma, renal cell carcinoma, lymphoma, or other advanced solid tumors.

48. The pharmaceutical combination according to claim 47, wherein the solid tumor is colorectal cancer, preferably advanced colorectal cancer, more preferably advanced colorectal cancer failed or intolerant to standard therapy;
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg to 10 mg/kg body weight (for example 5 mg/kg or 7.5 mg/kg) administered once every two weeks with each four weeks constituting a treatment cycle, or once every three weeks with each three weeks constituting a treatment cycle; especially 7.5 mg/kg administered once every three weeks (Q3W).

49. The pharmaceutical combination according to claim 47, wherein the solid tumor is melanoma, preferably advanced melanoma, more preferably advanced melanoma untreated with immunotherapy or advanced melanoma with prior immunotherapy failure;
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg body weight, 7.5 mg/kg, 10 mg/kg, 12.5 mg/kg body weight, or 15 mg/kg body weight administered once every two weeks with each four weeks constituting a treatment cycle, or once every three weeks with each three weeks constituting a treatment cycle.

50. The pharmaceutical combination according to claim 47, wherein the solid tumor is non-small cell lung cancer, for example squamous or non-squamous non-small cell lung cancer (preferably advanced non-squamous non-small cell lung cancer, more preferably advanced non-squamous non-small cell lung cancer failed or intolerant to standard therapy), hepatocellular carcinoma (preferably advanced hepatocellular carcinoma, more preferably advanced hepatocellular carcinoma untreated with systemic therapy), or renal cell carcinoma (preferably advanced renal cell carcinoma, more preferably advanced renal cell carcinoma failed or intolerant to standard therapy);
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 10 mg/kg body weight to 15 mg/kg body weight (for example 10 mg/kg body weight, 12.5 mg/kg body weight, or 15 mg/kg body weight) administered once every two weeks with each four weeks constituting a treatment cycle, or once every three weeks with each three weeks constituting a treatment cycle.

51. The pharmaceutical combination according to claim 47, wherein the solid tumor is lymphoma and other solid tumors, preferably lymphoma or other advanced solid tumors failed or intolerant to standard therapy;
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for intravenous infusion administration at a dose of 5 mg/kg body weight to 15 mg/kg body weight administered once every two weeks with each four weeks constituting a treatment cycle, or once every three weeks with each three weeks constituting a treatment cycle; preferably, the second active ingredient is for intravenous infusion administration at a dose of 5 mg/kg body weight, 7.5 mg/kg, 10 mg/kg, 12.5 mg/kg body weight, or 15 mg/kg body weight administered once every two weeks with each four weeks constituting a treatment cycle, or once every three weeks with each three weeks constituting a treatment cycle.

52. A pharmaceutical combination for treating melanoma, wherein the pharmaceutical combination comprises:
(i) a first active ingredient, wherein the first active ingredient is the PD-1/IL-2R bispecific antibody fusion protein as defined in any one of claims 1 to 12 or claims 31 to 42; and
(ii) a second active ingredient, wherein the second active ingredient is dacarbazine.

53. The pharmaceutical combination according to claim 52, wherein the first active ingredient is for injection administration, for example intravenous infusion, and is administered by injection, preferably intravenous injection, according to the dosage regimen defined in any one of claims 13 to 22; the second active ingredient is administered by injection, for example intravenous infusion, intravenous bolus, intraarterial infusion, or intraarterial bolus.

54. The pharmaceutical combination according to claim 53, wherein
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient dacarbazine is administered by intravenous or intraarterial infusion, and when administered by intravenous or intraarterial infusion, the dacarbazine is administered at a dose of 2.5-6 mg/kg body weight or 200-400 mg/m² body surface area once daily for 5-10 consecutive days, followed by a drug-free interval, with each 3-6 weeks constituting a treatment cycle; or a dose of 650-1450 mg/m² body surface area once every 4-6 weeks, correspondingly with each 4-6 weeks constituting a treatment cycle; when administered by intravenous or intraarterial bolus, the dacarbazine is administered at a dose of 200-400 mg/m² once daily for 5 consecutive days, followed by a drug-free interval, with each 3-4 weeks constituting a treatment cycle.

55. The pharmaceutical combination according to any one of claims 52 to 54, wherein the melanoma is advanced melanoma untreated with systemic therapy; the second active ingredient dacarbazine is for intravenous infusion administration at a dose of 850 mg/m² body surface area administered once every four weeks with each four weeks constituting a treatment cycle; or for intravenous infusion administration at a dose of 650-1050 mg/m² body surface area (for example, 850 mg/m² body surface area) administered once every three weeks with each three weeks constituting a treatment cycle; or for intravenous bolus administration at a dose of 200-300 mg/m² body surface area (for example, 250 mg/m² body surface area) administered once daily for 5 consecutive days, followed by a drug-free interval, with each four weeks constituting a treatment cycle; or for intravenous bolus administration at a dose of 200-300 mg/m² body surface area (for example, 250 mg/m² body surface area) administered once daily for 5 consecutive days, followed by a drug-free interval, with each three weeks constituting a treatment cycle.

56. A pharmaceutical combination for treating non-small cell lung cancer, wherein the pharmaceutical combination comprises:
(i) a first active ingredient, wherein the first active ingredient is the PD-1/IL-2R bispecific antibody fusion protein as defined in any one of claims 1 to 12 or claims 31 to 42; and
(ii) a second active ingredient, wherein the second active ingredient is a compound of formula (II) or a pharmaceutically acceptable salt thereof, 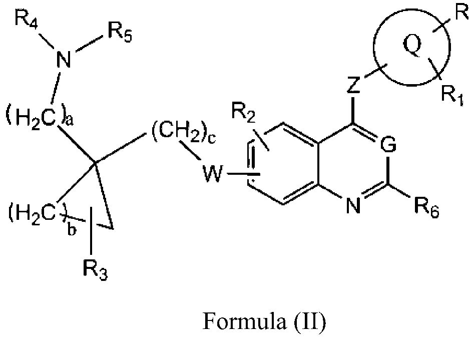 wherein
W is O; Z is O; G is CR; a is 0; c is 1; b is 1; R is H or C₁₋₆ alkyl; 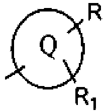 is 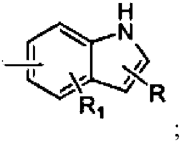
R₁, R₂, R₃ are each independently selected from H, halogen, and C₁₋₆ alkoxy;
R₄ and R₅ are each independently selected from H or C₁₋₆ alkyl;
R₆ is H.

57. The pharmaceutical combination according to claim 56, wherein the second active ingredient is anlotinib or a pharmaceutically acceptable salt thereof, for example anlotinib dihydrochloride.

58. The pharmaceutical combination according to claim 55 or 57, wherein the first active ingredient is for injection administration, for example intravenous infusion, and is administered by injection, preferably intravenous injection, according to the dosage regimen defined in any one of claims 13 to 22; the second active ingredient is for oral administration.

59. The pharmaceutical combination according to claim 58, wherein
the first active ingredient (for example molecule 2149) is for intravenous infusion administration at a dose of 0.2 µg/kg body weight to 1 mg/kg body weight administered once weekly with each three weeks constituting a treatment cycle; or at a dose of 1 mg/kg body weight to 3 mg/kg body weight administered once weekly with each three weeks constituting a treatment cycle; or at a dose of 1 mg/kg body weight to 3 mg/kg body weight administered once every two weeks with each four weeks constituting a treatment cycle; or at a dose of 1 mg/kg body weight to 3 mg/kg body weight once every three weeks with each three weeks constituting a treatment cycle; or the first active ingredient is for intravenous infusion administration at a priming dose of 0.2 µg/kg body weight to 1 mg/kg body weight (for example 100 µg/kg body weight or 1 mg/kg body weight) administered on day 1, and a maintenance dose of 2 mg/kg body weight administered on day 8±1, and the maintenance dose is administered once every two weeks; or a priming dose of 0.2 µg/kg body weight to 1 mg/kg body weight (for example 100 µg/kg body weight or 1 mg/kg body weight) administered on day 1, and a maintenance dose of 2 mg/kg body weight or 3 mg/kg administered on day 8±1, and the maintenance dose is administered once every three weeks;
the second active ingredient is for oral administration at a dose of 8 mg to 12 mg administered once daily for 2 consecutive weeks, followed by a drug-free interval of 1 week, with each three weeks constituting a treatment cycle.

60. The pharmaceutical combination according to any one of claims 56 to 59, wherein the non-small cell lung cancer is advanced non-small cell lung cancer, preferably advanced non-small cell lung cancer failed or intolerant to standard therapy; the second active ingredient (for example anlotinib dihydrochloride) is for oral administration at a dose of 12 mg (when the second active ingredient is a pharmaceutically acceptable salt of anlotinib for example dihydrochloride of anlotinib, calculated as anlotinib) administered once daily for 2 consecutive weeks, followed by a drug-free interval of 1 week, with each three weeks constituting a treatment cycle.

61. The pharmaceutical combination according to any one of claims 31 to 60, wherein the pharmaceutical combination comprises the first active ingredient and the second active ingredient as the only active ingredients.

62. The pharmaceutical combination according to any one of claims 31 to 61, wherein the pharmaceutical combination is in the form of a kit or pack, and the kit or pack comprises: a pharmaceutical composition comprising the first active ingredient; a pharmaceutical composition comprising the second active ingredient; and an instruction for use, wherein the instruction describes administration methods of the two pharmaceutical compositions.

63. Use of the pharmaceutical combination according to any one of claims 31 to 61 in the preparation of a medicament.

64. A method for treating cancer, wherein the method comprises administering the pharmaceutical combination according to any one of claims 31 to 61, wherein the first active ingredient and the second active ingredient are administered simultaneously in a single pharmaceutical composition, or administered simultaneously or separately in separate pharmaceutical compositions.

65. A single-dose administration unit comprising a single therapeutically effective amount of the fusion protein as defined in any one of claims 1 to 12, for example 10 µg to 300 mg of the fusion protein as defined in any one of claims 1 to 12; preferably, the single-dose administration unit is for injection administration.

66. A kit comprising one or more single-dose administration units according to claim 65, and an instruction for use indicating administration of the one or more single-dose administration units according to the dosage regimen as defined in any one of claims 13 to 22.
